# EUROPEAN PATENT SPECIFICATION

(11) **EP 2 612 924 B1**
(45) Date of publication and mention of the grant of the patent: **20.12.2017**
(21) Application number: 12192837.8
(22) Date of filing: 21.05.2008
(51) Int. Cl.: C12Q 1/68

(54) **METHODS AND COMPOSITIONS FOR IDENTIFYING AND TREATING LUPUS**
VERFAHREN UND ZUSAMMENSETZUNGEN ZUM IDENTIFIZIEREN UND BEHANDELN VON LUPUS
PROCÉDÉS ET COMPOSITIONS POUR L'IDENTIFICATION ET LE TRAITEMENT DU LUPUS

(30) Priority: 21.05.2007 US 939156 P; 12.12.2007 US 13283 P
(43) Date of publication of application: 10.07.2013
(62) Divisional of application: 08769569.8
(73) Proprietor: Genentech, Inc., South San Francisco, CA 94080-4990 (US)
(72) Inventor: Behrens, Timothy W., Burlingame, CA 94010 (US); Hom, Geoffrey, Daly City, CA 94015 (US); Ortmann, Ward A., Walnut Creek, CA 94598 (US); Graham, Robert Royal, San Francisco, CA 94118 (US)
(74) Representative: Woolley, Lindsey Claire

(56) References cited:
- HARLEY JOHN B ET AL: "Genome-wide association scan in women with systemic lupus erythematosus identifies susceptibility variants in ITGAM, PXK, KIAA1542 and other loci", NATURE GENETICS, vol. 40, no. 2, 1 February 2008 (2008-02-01), pages 204-210, XP002525263, NATURE PUBLISHING GROUP, NEW YORK, US ISSN: 1061-4036, DOI: 10.1038/NG.81 [retrieved on 2008-01-20]
- CHAIM O. JACOB ET AL: "Identification of novel susceptibility genes in childhood-onset systemic lupus erythematosus using a uniquely designed candidate gene pathway platform", ARTHRITIS & RHEUMATISM, vol. 56, no. 12, 1 December 2007 (2007-12-01), pages 4164-4173, XP055062511, ISSN: 0004-3591, DOI: 10.1002/art.23060
- DATABASE dbSNP [Online] 5 September 2001 (2001-09-05), XP002696818, retrieved from NCBI Database accession no. ss3203424

## Description

### CROSS-REFERENCES TO RELATED APPLICATIONS

### FIELD OF THE INVENTION

The present invention generally relates to a unique set of genetic polymorphisms associated with lupus, and compositions and methods for assessing risk of developing lupus as well as for diagnosing and treating lupus.

### BACKGROUND

Lupus is an autoimmune disease involving antibodies that attack connective tissue. The disease is estimated to affect nearly 1 million Americans, primarily women between the ages of 20-40. The principal form of lupus is a systemic one (systemic lupus erythematosus; SLE). Systemic Lupus Erythematosus (SLE) is a chronic autoimmune disease with strong genetic as well as environmental components (See, *e.g.*, Hochberg MC, Dubois' Lupus Erythematosus. 5th ed., Wallace DJ, Hahn BH, eds. Baltimore: Williams and Wilkins (1997); Wakeland EK, et al., Immunity 2001;15(3):397-408; Nath SK, et al., Curr. Opin. Immunol. 2004;16(6):794-800). Autoantibodies play an important role in the pathogenesis of SLE, and the diverse clinical manifestations of the disease are due to the deposition of antibody-containing immune complexes in blood vessels leading to inflammation in the kidney, brain and skin, together with direct pathogenic effects of autoantibodies contributing to hemolytic anemia and thrombocytopenia. SLE is generally characterized as an autoimmune connective-tissue disorder with a wide range of clinical features, which predominantly affects women, especially from certain ethnic groups. D'Cruz et al., Lancet (2007), 369:587-596. SLE is associated with the production of antinuclear antibodies, circulating immune complexes, and activation of the complement system. SLE has an incidence of about 1 in 700 women between the ages of 20 and 60. SLE can affect any organ system and can cause severe tissue damage. Numerous autoantibodies of differing specificity are present in SLE. SLE patients often produce autoantibodies having anti-DNA, anti-Ro, and anti-platelet specificity and that are capable of initiating clinical features of the disease, such as glomerulonephritis, arthritis, serositis, complete heart block in newborns, and hematologic abnormalities. These autoantibodies are also possibly related to central nervous system disturbances. Arbuckle *et al.* describes the development of autoantibodies before the clinical onset of SLE (Arbuckle et al. N. Engl. J. Med. 349(16): 1526-1533 (2003)). Definitive diagnosis of lupus, including SLE, is not easy, resulting in clinicians resorting to a multi-factorial signs and symptoms-based classification approach. Gill et al., American Family Physician (2003), 68(11): 2179-2186.

Untreated lupus can be fatal as it progresses from attack of skin and joints to internal organs, including lung, heart, and kidneys (with renal disease being the primary concern), thus making early and accurate diagnosis of and/or assessment of risk of developing lupus particularly critical. Lupus mainly appears as a series of flare-ups, with intervening periods of little or no disease manifestation. Kidney damage, measured by the amount of proteinuria in the urine, is one of the most acute areas of damage associated with pathogenicity in SLE, and accounts for at least 50% of the mortality and morbidity of the disease.

One of the most difficult challenges in clinical management of complex autoimmune diseases such as lupus is the accurate and early identification of the disease in a patient. Over the years, many linkage and candidate gene studies have been performed to identify genetic factors contributing to SLE susceptibility. Haplotypes carrying the HLA Class II alleles *DRB1*0301* and *DRB1*1501* are clearly associated with disease as well as the presence of antibodies to nuclear autoantigens. See, *.e.g*., Goldberg MA, et al., Arthritis Rheum 1976; 19(2):129-32; Graham RR, et al., Am J Hum Genet 2002; 71(3):543-53; and Graham RR, et al., Eur J Hum Genet 2007; 15(8):823-30). More recently, variants of Interferon Regulatory Factor 5 (*IRF5*) and Signal Transducer and Activator of Transcription 4 (*STAT4*) were discovered to be significant risk factors for SLE. See, *e.g.,* Sigurdsson S, et al., Am J Hum Genet 2005; 76(3):528-37; Graham RR, et al., Nat Genet 2006; 38(5):550-55; Graham RR, et al., Proc Natl Acad Sci U S A 2007; 104(16):6758-63; and Remmers EF, et al., N Engl J Med 2007; 357(10):977-86. The identification of *IRF5* and *STAT4* as SLE risk genes provides support for the concept that the Type-I interferon pathway is central to disease pathogenesis. See, *e.g*., Ronnblom L, et al., J Exp Med 2001; 194(12):F59-63; Baechler EC, et al., Curr Opin Immunol 2004; 16(6):801-07; Banchereau J, et al., Immunity 2006; 25(3):383-92; Miyagi T, et al., J Exp Med 2007; Epublication; Sept 10.

To this end, it would be highly advantageous to have molecular-based diagnostic methods that can be used to objectively identify the presence of and/or classify the disease in a patient. Genetic variations, or polymorphisms, are genetic variations that are present in an organism's genome. Polymorphisms include single nucleotide polymorphisms (SNPs). See, *e.g.,* Carlson et al., Nature 2004; 429:446-452; Bell, Nature 2004; 429:453-463; Evans & Relling, Nature 2004; 429:464-468. SNPs have been strongly correlated with risk and/or presence of serious diseases such as diabetes (Sladek et al., Nature 2007; 445: 881-828; Zeggini et al., Science 2007; Apr 26; Scott et al., Science 2007; Apr 26; and Saxena et al., Science 2007; Apr 26); Crohn disease (e.g., Hampe et al., Nat. Genet. 2007; Feb;39(2):207-11); rheumatoid arthritis (e.g., US Pat. Pub. No. 2007/0031848); and other inflammatory autoimmune disease (e.g., U.S. Pat. 6,900,016; U.S. Pat. 7,205,106).

Until recently, it has not been possible to comprehensively examine the genome for variants that modify risk to complex diseases such as lupus. However, the generation of an extensive catalog of common human variation (see, *e.g*., Nature 2005; 437(7063):1299-320) coupled with technological advances that permit cost-effective and accurate genotyping of hundreds of thousands of variants, has fueled a revolution in human genetics. For the first time, it is possible to conduct well-powered genome-wide association scans to more fully test the hypothesis that common variants influence risk. In the past two years, this technology has been highly validated. See, *e.g*., Dewan A, et al., Science 2006; 314(5801):989-92; Nature 2007; 447(7145):661-78, Matarin M, et al., Lancet neurology 2007; 6(5):414-20; Moffatt MF, et al., Nature 2007; 448(7152):470-73; Plenge RM, et al., N Engl J Med 2007; Saxena R, et al., Science 2007; 316(5829):1331-36; Scott LJ, et al., Science 2007; 316(5829):1341-45; Scuteri A, et al., PLoS Genet 2007;3(7):e115. The identified risk loci are providing new insights into the molecular pathways dysregulated in human disease.

However, there continues to be a significant lack of credible information on SNP associations with complex diseases such as lupus, thus it is clear that a continuing need exists to identify polymorphisms associated with such diseases. Such associations would greatly benefit the identification of the presence of lupus in patients or the determination of susceptibility to develop the disease. In addition, statistically and biologically significant and reproducible information regarding association of a SNP with a complex disease such as lupus could be utilized as an integral component in efforts to identify specific subsets of patients who would be expected to significantly benefit from treatment with a particular therapeutic agent, for example where the therapeutic agent is or has been shown in clinical studies to be of therapeutic benefit in such specific lupus patient subpopulation.

The invention described herein meets the above-described needs and provides other benefits.

### BRIEF SUMMARY OF THE INVENTION

The invention provides accurate, simple, and rapid methods and compositions for identifying lupus, and for assessing risk of developing lupus, based at least in part on the identification of one or more genetic variations, e.g., SNPs, that are correlated with high statistical and biological significance with the presence, subtypes, and/or patient subpopulations of lupus, as defined in the claims. More specifically, the invention relates to the identification of a unique set of SNPs, unique combinations of such SNPs, and linkage disequilibrium regions that are associated with lupus and its subtypes, and patient subpopulations suffering from same.

In particular, the unique set and/or combinations of SNPs can be used as a genetic profile or signature indicative of a subject at risk of developing lupus, or indicative of the disease or symptom or condition thereof. The polymorphisms disclosed herein are useful as biomarkers for assessing risk of developing lupus, as well as for targets for the design of diagnostic reagents. In some aspects, the SNP is not associated with a gene. In other aspects, the SNP is associated with a gene, and can be located either in an intergenic or intragenic region, and more particularly, can be located in a coding or noncoding region. The genes associated with a SNP of the present invention may be associated with an unknown gene, or may be associated with a known gene e.g., ITGAM or BLK.

The SNPs identified herein provide targets for development of therapeutic agents for use in the diagnosis and treatment of genetically identified lupus patients, including diagnosis and targeted treatment of lupus patient subpopulations exhibiting a distinct genetic signature comprising one or more of the SNPs of the present invention. For example, in one aspect, the genes containing the genetic variations identified herein, and the nucleic acid (e.g., DNA or RNA) associated with these genes, and proteins encoded by these genes, can be used as targets for the development of therapeutic agents (e.g., small molecule compounds, antibodies, antisense/RNAi agents, etc.) or used directly as therapeutic agents (e.g., therapeutic proteins, etc.) for the treatment of lupus.

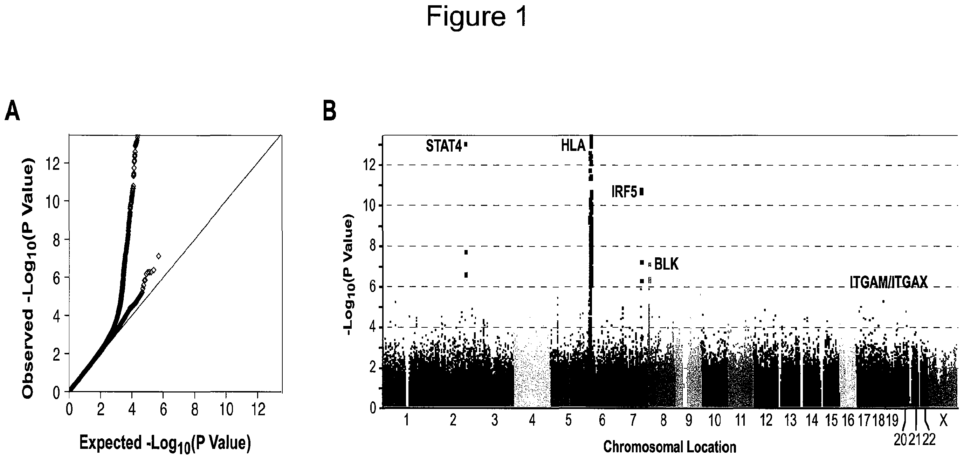

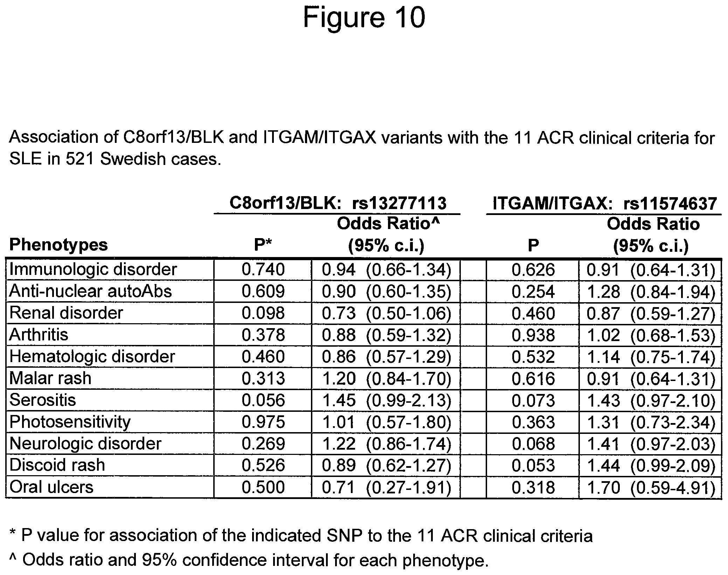

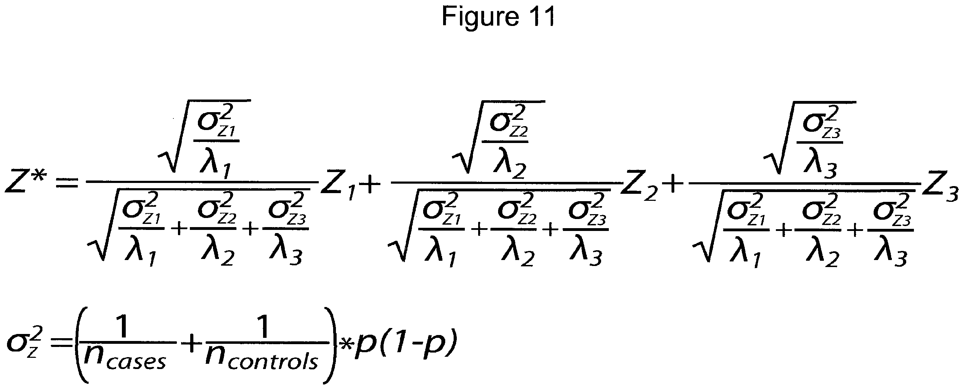

Accordingly, in one aspect, the disclosure provides a set of one or more SNPs that form a unique genetic signature for assessing the risk of developing lupus. In one aspect, the unique genetic signature comprises about 1-10, 10-20, 20-30, 30-40, or 40-50 SNPs selected from any of the SNPs set forth in Figures 1-17 and Tables 1-10.

In one aspect, the unique genetic signature comprises one or more SNPs, 2 or more SNPs, 3 or more SNPs, 4 or more SNPs, 5 or more SNPs, 6 or more SNPs, 7 or more SNPs, 8 or more SNPs, 9 or more SNPs, 10 or more SNPs, 11 or more SNPs, 12 or more SNPs, 13 or more SNPs, 14 or more SNPs, 15 or more SNPs, 16 or more SNPs, 17 or more SNPs, 18 or more SNPs, 19 or more SNPs, or 20 or more SNPs selected from any of the SNPs set forth in Figures 1-17 and Tables 1-10. In one aspect, the SNPs of the genetic signature are selected from Table 6. In another aspect, the SNPs are selected from the group consisting of rs9888739, rs13277113, rs7574865, rs2269368, rs6889239, rs2391592 and rs21177770. In another aspect, the SNPs are selected from the group consisting of rs2187668, rs10488631, rs7574865, rs9888739, rs13277113, rs2431697, rs6568431, rs10489265, rs2476601, rs2269368, rs1801274, rs4963128, rs5754217, rs6445975, rs3129860, rs10516487, rs6889239, rs2391592, and rs2177770.

In another aspect, the disclosure provides for methods of assessing whether a subject is at risk of developing lupus by detecting in a biological sample obtained from said subject, the presence of a genetic signature indicative of risk of developing lupus, wherein said genetic signature comprises a set of one or more SNPs selected from any of the SNPs set forth in Figures 1-17 and Tables 1-10. In one aspect, the set of SNPs comprises about 1-10, 10-20, 20-30, 30-40, or 40-50 SNPs selected from any of the SNPs set forth in Figures 1-17 and Tables 1-10. In another aspect, the set of SNPs comprises 2 or more SNPs, 3 or more SNPs, 4 or more SNPs, 5 or more SNPs, 6 or more SNPs, 7 or more SNPs, 8 or more SNPs, 9 or more SNPs, 10 or more SNPs, 11 or more SNPs, 12 or more SNPs, 13 or more SNPs, 14 or more SNPs, 15 or more SNPs, 16 or more SNPs, 17 or more SNPs, 18 or more SNPs, 19 or more SNPs, or 20 or more SNPs selected from any of the SNPs set forth in Figures 1-17 and Tables 1-10. In another aspect, the set of SNPs comprises 1-19 SNPs selected from Table 6. In another aspect, the set of SNPs comprises a BLK SNP selected from any of the BLK SNPs set forth in Tables 7-10. In another aspect, the set of SNPs comprises an ITGAM SNP selected from any of the ITGAM SNPs set forth in Tables 7-10. In another aspect, the set of SNPs further comprises a BLK SNP selected from any of the BLK SNPs set forth in Tables 7-10. In another aspect, the set of SNPs comprises one or more SNPs selected from the following group of SNPs: rs2187668, rs10488631, rs7574865, rs9888739, rs13277113, rs2431697, rs6568431, rs10489265, rs2476601, rs2269368, rs1801274, rs4963128, rs5754217, rs6445975, rs3129860, rs10516487, rs6889239, rs2391592, and rs2177770.

In another aspect, the disclosure provides for methods of diagnosing lupus in a subject by detecting in a biological sample obtained from said subject, the presence of a genetic signature indicative of lupus, wherein said genetic signature comprises a set of one or more SNPs selected from any of the SNPs set forth in Figures 1-17 and Tables 1-10.

In another aspect, the disclosure provides for an isolated polynucleotide or fragment thereof that is at least about 10 nucleotides in length, wherein the polynucleotide or fragment thereof comprises: a) a genetic variation at a nucleotide position corresponding to the position of a single nucleotide polymorphism (SNP) selected from any of the SNPs set forth in Figures 1-17 and Tables 1-10, or (b) the complement of (a). In one aspect, the isolated polynucleotide is a genomic DNA comprising a single nucleotide polymorphism (SNP) selected from any of the SNPs set forth in Figures 1-17 and Tables 1-10. In another aspect, the isolated polynucleotide is an RNA comprising a single nucleotide polymorphism (SNP) selected from any of the SNPs set forth in Figures 1-17 and Tables 1-10.

In one aspect, the disclosure provides for an isolated PRO-associated polynucleotide or fragment thereof that is at least about 10 nucleotides in length, wherein the PRO-associated polynucleotide or fragment thereof comprises: a) a genetic variation at a nucleotide position corresponding to the position of a single nucleotide polymorphism (SNP) selected from any of the SNPs set forth in Figures 1-17 and Tables 1-10, or (b) the complement of (a). In one aspect, the isolated polynucleotide is a genomic DNA that encodes a gene (and/or regulatory region of the gene) comprising a single nucleotide polymorphism (SNP) selected from any of the SNPs set forth in Figures 1-17 and Tables 1-10. In another aspect, the SNP is in a region of a chromosome that does not encode a gene. In another aspect, the SNP is in an intergenic region of a chromosome. In another aspect, the isolated polynucleotide is a primer. In another aspect, the isolated polynucleotide is an oligonucleotide.

In another aspect, the disclosure provides for an oligonucleotide that is (a) an allele-specific oligonucleotide that hybridizes to a region of a polynucleotide comprising a genetic variation at a nucleotide position corresponding to the position of a single nucleotide polymorphism (SNP) set forth in Figures 1-17 and Tables 1-10, or (b) the complement of (a). In one aspect, the SNP is in a PRO-associated polynucleotide that encodes a gene (or its regulatory region) comprising a single nucleotide polymorphism (SNP) selected from any of the SNPs set forth in Figures 1-17 and Tables 1-10. In another aspect, the SNP is in a genomic DNA that encodes a gene (or its regulatory region) comprising a single nucleotide polymorphism (SNP) selected from any of the SNPs set forth in Figures 1-17 and Tables 1-10. In another aspect, the SNP is in a non-coding region of the gene. In another aspect, the SNP is in a coding region of the gene. In another aspect, the allele-specific oligonucleotide is an allele-specific primer.

In another aspect, the disclosure provides for a kit comprising any one of the oligonucleotide above and, optionally, at least one enzyme. In one aspect, the at least one enzyme is a polymerase. In another aspect, the at least one enzyme is a ligase.

In another aspect, the invention provides for a microarray comprising any of the oligonucleotides above.

In another aspect, the disclosure provides for a method of detecting the absence or presence of a variation in a polynucleotide at a nucleotide position corresponding to the position of a single nucleotide polymorphism (SNP) as set forth in Figures 1-17 and Tables 1-10, the method comprising (a) contacting nucleic acid suspected of comprising the variation with an allele-specific oligonucleotide that is specific for the variation under conditions suitable for hybridization of the allele-specific oligonucleotide to the nucleic acid; and (b) detecting the absence or presence of allele-specific hybridization. In one aspect, the variation comprises a SNP as set forth in Figures 1-17 and Tables 1-10. In one aspect, the polynucleotide is a PRO-associated polynucleotide.

In another aspect, the disclosure provides for a method of amplifying a nucleic acid comprising a variation in a polynucleotide at a nucleotide position corresponding to the position of a single nucleotide polymorphism (SNP) selected from any of the SNPs as set forth in Figures 1-17 and Tables 1-10, the method comprising (a) contacting the nucleic acid with a primer that hybridizes to the nucleic acid at a sequence 3' of the variation, and (b) extending the primer to generate an amplification product comprising the variation. In one aspect, the polynucleotide is a PRO-associated polynucleotide.

In another aspect, the disclosure provides for a method of determining the genotype of a biological sample from a mammal, the method comprising detecting, in nucleic acid material derived from the biological sample, the absence or presence of a variation in a polynucleotide at a nucleotide position corresponding to the position of a single nucleotide polymorphism (SNP) selected from any of the SNPs as set forth in Figures 1-17 and Tables 1-10. In one aspect, the polynucleotide is a PRO-associated polynucleotide.

In another aspect, the biological sample is known to or suspected of comprising a polynucleotide of the present invention, wherein the polynucleotide comprises a variation at a nucleotide position corresponding to the position of a single nucleotide polymorphism (SNP) selected from any of the SNPs as set forth in Figures 1-17 and Tables 1-10. In another aspect, the biological sample is a disease tissue. In another aspect, the detecting comprises carrying out a process selected from a primer extension assay; an allele-specific primer extension assay; an allele-specific nucleotide incorporation assay; an allele-specific oligonucleotide hybridization assay; a 5' nuclease assay; an assay employing molecular beacons; and an oligonucleotide ligation assay.

In another aspect, the disclosure provides for a method of sub-classifying lupus in a mammal, the method comprising detecting the presence of one or more of the SNPs set forth in Figures 1-17 and Tables 1-10, in a biological sample derived from the mammal, wherein the biological sample is known to or suspected of comprising at least one polynucleotide comprising a SNP selected from any of the SNPs set forth in Figures 1-17 and Tables 1-10. In one aspect, the polynucleotide is a PRO-associated polynucleotide.

In another aspect, the detecting comprises carrying out a process selected from a primer extension assay; an allele-specific primer extension assay; an allele-specific nucleotide incorporation assay; an allele-specific oligonucleotide hybridization assay; a 5' nuclease assay; an assay employing molecular beacons; and an oligonucleotide ligation assay.

In another aspect, the disclosure provides for a method for predicting whether a subject with lupus will respond to a lupus therapeutic agent, the method comprising determining whether the subject comprises a variation in a polynucleotide at a nucleotide position corresponding to the position of a single nucleotide polymorphism (SNP) selected from any of the SNPs as set forth in Figures 1-17 and Tables 1-10, wherein the presence of a variation indicates that the subject will respond to the therapeutic agent. In one aspect, the polynucleotide is a PRO-associated polynucleotide.

In another aspect, the disclosure provides a method of diagnosing or prognosing lupus in a subject, the method comprising detecting the presence of a variation in a polynucleotide derived from a biological sample obtained from the subject, wherein: (a) the biological sample is known to comprise, or is suspected of comprising, a polynucleotide comprising the variation; (b) the variation comprises, or is located at a nucleotide position corresponding to, a SNP selected from any of the SNPs set forth in Figures 1-17 and Tables 1-10; and (c) the presence of the variation is a diagnosis or prognosis of lupus in the subject.

In another aspect, the disclosure provides a method of diagnosing or prognosing lupus in a subject, the method comprising detecting the presence of a variation in a PRO or PRO-associated polynucleotide derived from a biological sample obtained from the subject, wherein: (a) the biological sample is known to comprise, or is suspected of comprising, a PRO or PRO-associated polynucleotide comprising the variation; (b) the variation comprises, or is located at a nucleotide position corresponding to, a SNP set forth in Figures 1-17 and Tables 1-10; and (c) the presence of the variation is a diagnosis or prognosis of lupus in the subject.

In another aspect, the disclosure provides a method of aiding in the diagnosis or prognosis of lupus in a subject, the method comprising detecting the presence of a variation in a polynucleotide derived from a biological sample obtained from the subject, wherein: (a) the biological sample is known to comprise, or suspected of comprising, a polynucleotide comprising the variation; (b) the variation comprises, or is located at a nucleotide position corresponding to, a SNP selected from any of the SNPs set forth in Figures 1-17 and Tables 1-10; and (c) the presence of the variation is a diagnosis or prognosis of a condition or symptom of lupus in the subject.

In another aspect, the disclosure provides a method of aiding in the diagnosis or prognosis of lupus in a subject, the method comprising detecting the presence of a variation in a PRO or PRO-associated polynucleotide derived from a biological sample obtained from the subject, wherein: (a) the biological sample is known to comprise, or suspected of comprising, a PRO or PRO-associated polynucleotide comprising the variation; (b) the variation comprises, or is located at a nucleotide position corresponding to, a SNP selected from any of the SNPs set forth in Figures 1-17 and Tables 1-10; and (c) the presence of the variation is a diagnosis or prognosis of a condition or symptom of lupus in the subject.

In another aspect, the polynucleotide comprises a sequence within a linkage disequilibrium region (e.g., as set forth in Figures 1-17 and Tables 1-10). In one aspect, the variation is in genomic DNA comprising a SNP selected from any of the SNPs set forth in Figures 1-17 and Tables 1-10. In one aspect, the SNP is in a chromosomal region that does not encode a gene. In another aspect, the SNP is in an intergenic region.

In another aspect, the PRO-associated polynucleotide encodes a PRO that is encoded by a sequence within a linkage disequilibrium region (e.g., as set forth in Figures 1-17 and Tables 1-10). In one aspect, the variation is in genomic DNA that encodes a gene (or its regulatory region), wherein the gene (or its regulatory region) comprises a SNP selected from any of the SNPs set forth in Figures 1-17 and Tables 1-10. In one aspect, the SNP is in a non-coding region of the gene. In another aspect, the SNP is in a coding region of the gene.

In another aspect, the disclosure provides for a method of identifying a therapeutic agent effective to treat lupus in a patient subpopulation, the method comprising correlating efficacy of the agent with the presence in the patient of one or more of the SNPs selected from any of the SNPs set forth in Figures 1-17 and Tables 1-10, thereby identifying the agent as effective to treat lupus in said patient subpopulation.

In another aspect, the disclosure provides for a method of identifying a therapeutic agent effective to treat lupus in a patient subpopulation, the method comprising correlating efficacy of the agent with the presence of a combination of the SNPs selected from any of the SNPs set forth in Figures 1-17 and Tables 1-10, thereby identifying the agent as effective to treat lupus in said patient subpopulation.

In another aspect, the disclosure provides for a method of treating a lupus condition in a subject in whom a genetic variation is known to be present at a nucleotide position corresponding to a single nucleotide polymorphism (SNP) selected from any of the SNPs set forth in Figures 1-17 and Tables 1-10, the method comprising administering to the subject a therapeutic agent effective to treat the condition.

In another aspect, the disclosure provides for a method of treating a subject having a lupus condition, the method comprising administering to the subject a therapeutic agent effective to treat the condition in a subject who has a genetic variation at a nucleotide position corresponding to a single nucleotide polymorphism (SNP) selected from any of the SNPs set forth in Figures 1-17 and Tables 1-10.

In another aspect, the disclosure provides for a method of treating a subject having a lupus condition, the method comprising administering to the subject a therapeutic agent shown to be effective to treat said condition in at least one clinical study wherein the agent was administered to at least five human subjects who each had a genetic variation at a nucleotide position corresponding to a single nucleotide polymorphism (SNP) selected from any of the SNPs set forth in Figures 1-17 and Tables 1-10. In one aspect, the at least five subjects had two or more different SNPs in total for the group of at least five subjects. In another aspect, the at least five subjects had the same SNP for the entire group of at least five subjects.

In another aspect, the disclosure provides for a method of treating a lupus subject of a specific lupus patient subpopulation, wherein the subpopulation is characterized at least in part by association with genetic variation at a nucleotide position corresponding to a SNP selected from any of the SNPs set forth in Figures 1-17 and Tables 1-10, and wherein the method comprises administering to the subject an effective amount of a therapeutic agent that is approved as a therapeutic agent for said subpopulation. In one aspect, the subpopulation has lupus nephritis. In another aspect, the subpopulation is female. In another aspect, the subpopulation is of European ancestry.

In another aspect, the disclosure provides for a method comprising manufacturing a lupus therapeutic agent, and packaging the agent with instruction to administer the agent to a subject who has or is believed to have lupus and who has a genetic variation at a position corresponding to a single nucleotide polymorphism (SNP) selected from any of the SNPs set forth in Figures 1-17 and Tables 1-10.

In another aspect, the disclosure provides for a method of specifying a therapeutic agent for use in a lupus patient subpopulation, the method comprising providing instruction to administer the therapeutic agent to a patient subpopulation characterized by a genetic variation at a position corresponding to a single nucleotide polymorphism (SNP) selected from any of the SNPs set forth in Figures 1-17 and Tables 1-10.

In another aspect, the disclosure provides for a method for marketing a therapeutic agent for use in a lupus patient subpopulation, the method comprising informing a target audience about the use of the therapeutic agent for treating the patient subpopulation as characterized by the presence, in patients of such subpopulation, of a genetic variation at a position corresponding to a single nucleotide polymorphism (SNP) selected from any of the SNPs set forth in Figures 1-17 and Tables 1-10.

In another aspect, the disclosure provides for a method for modulating signaling through the B cell receptor in a subject in whom a genetic variation is known to be present at a nucleotide position corresponding to a single nucleotide polymorphism (SNP) selected from any of the SNPs set forth in Figures 1-17 and Tables 1-10, the method comprising administering to the subject a therapeutic agent effective to modulate signaling through the B cell receptor.

In another aspect, the disclosure provides for a method for modulating the differentiation of Th17 cells in a subject in whom a genetic variation is known to be present at a nucleotide position corresponding to a single nucleotide polymorphism (SNP) selected from any of the SNPs set forth in Figures 1-17 and Tables 1-10, the method comprising administering to the subject a therapeutic agent effective to modulate the differentiation of Th17 cells.

In another aspect, the disclosure provides for a set of SNPs comprising a genetic signature indicative of the risk of developing lupus, wherein said set of SNPs comprises one or more SNPs selected from any of the SNPs set forth in Figures 1-17 and Tables 1-10. In one aspect, the set of SNPs comprises about 1-10, 10-20, 20-30, 30-40, or 40-50 SNPs selected from any of the SNPs set forth in Figures 1-17 and Tables 1-10. In another aspect, the set of SNPs comprises one or more SNPs selected from the group consisting of rs9888739, rs13277113, rs7574865, rs2269368, rs6889239, rs2391592 and rs21177770. In another aspect, the set of SNPs comprises 2 or more SNPs, 3 or more SNPs, 4 or more SNPs, 5 or more SNPs, 6 or more SNPs, 7 or more SNPs, 8 or more SNPs, 9 or more SNPs, 10 or more SNPs, 11 or more SNPs, 12 or more SNPs, 13 or more SNPs, 14 or more SNPs, 15 or more SNPs, 16 or more SNPs, 17 or more SNPs, 18 or more SNPs, 19 or more SNPs, or 20 or more SNPs selected from any of the SNPs set forth in Figures 1-17 and Tables 1-10. In another aspect, the set of SNPs comprises 1-19 SNPs selected from Table 6. In another aspect, the set of SNPs comprises a BLK SNP selected from any of the BLK SNPs set forth in Tables 7-10. In another aspect, the set of SNPs comprises an ITGAM SNP selected from any of the ITGAM SNPs set forth in Tables 7-10. In another aspect, the set of SNPs further comprises a BLK SNP selected from any of the BLK SNPs set forth in Tables 7-10. In another aspect, the set of SNPs comprises one or more SNPs selected from the following group of SNPs: rs2187668, rs10488631, rs7574865, rs9888739, rs13277113, rs2431697, rs6568431, rs10489265, rs2476601, rs2269368, rs1801274, rs4963128, rs5754217, rs6445975, rs3129860, rs10516487, rs6889239, rs2391592, and rs2177770.

In another aspect, the disclosure provides for a set of SNPs comprising a genetic signature indicative of lupus, wherein said set of SNPs comprises one or more SNPs selected from any of the SNPs set forth in Figures 1-17 and Tables 1-10.

### BRIEF DESCRIPTION OF THE DRAWINGS

Figure 1 depicts the results from a genome-wide association scan in SLE identifies 5 major genes. Data represent 502,033 SNP variants typed in 3 sample series, for a total of 1311 SLE cases and 3340 controls. Panel A shows a quantile-quantile plot of the observed P value distribution vs the expected null P value distribution. The diamonds represent all P values, and the circles represent P values after exclusion of the *HLA*, *IRF5* and *STAT4* region variants. Panel B is a graphical representation of the -log₁₀ P values from the combined analysis organized by chromosome. Additional HLA region variants (N=34) with P < 1 x 10⁻¹³ are not shown in Panel B.

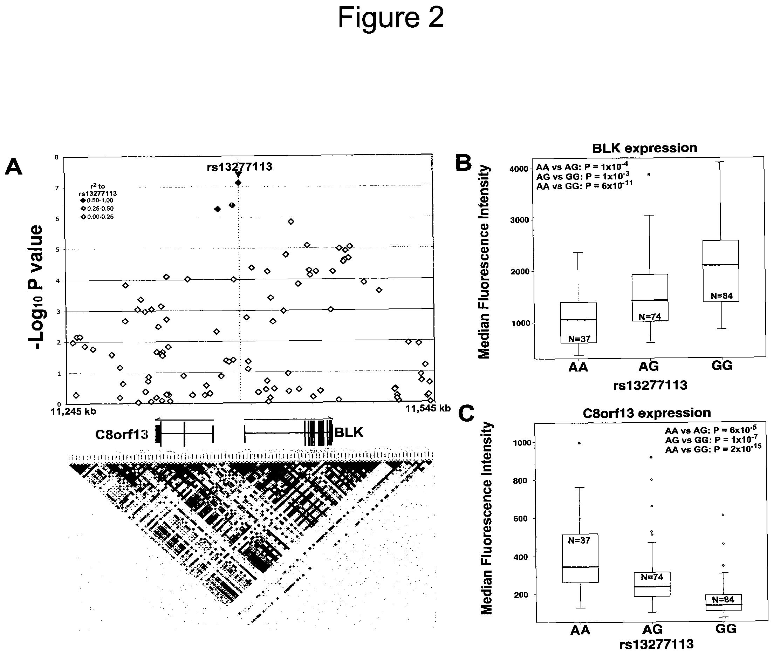

Figure 2 shows that associated variants from the *BLK*/*C8orf13* region correlate with expression levels in transformed B cells. A) The -log₁₀ P values from the *BLK*/*C8orf13* region are displayed. The color of the diamonds represents the r² correlations with rs13277113. All RefSeq genes in the region are displayed above a plot showing the LD in the region as determined by analysis of control chromosomes. Of note, this associated region on chromosome 8 lies within a common polymorphic 4.2 Mb intra-chromosomal inversion (See, for example, Giglio et al. Am J Hum Genet 2001;68(4):874-83 and Sugawara et al. Genomics 2003;82(2):238-44), and is associated with unusually low levels of extended LD across the region, as shown. However, the association of *BLK*/*C8orf13* to SLE is independent of the inversion. The expression of *BLK* (B) and *C8orf13* (C) in transformed B cell lines from 210 unrelated healthy CEU HapMap founders is shown stratified by genotype at rs13277113. Significance of the differential expression was determined using unpaired Student's T-tests.
Figure 3 shows that variants within the *ITGAM*/*ITGAX* locus are associated with SLE. Panel A shows the -log₁₀ P values from the *ITGAM*/*ITGAX* region. The color of the diamonds represent the r² correlations with rs11574637. All RefSeq genes in the region are displayed above a plot showing the LD in the region as determined by the control chromosomes studied. Panel B depicts the genomic structure of *ITGAM,* the conserved major protein domains, and the relationship between rs11574637 and two nonsynonymous alleles of *ITGAM.*
Figure 4 depicts the frequency of clinical characteristics in SLE Series 1-3 and the Swedish cases.
Figure 5 depicts the top 50 loci associated with SLE in a whole genome scan in 1311 cases and 3340 controls.
Figure 6 depicts the expression levels of BLK, *C8orf1* and control genes in 210 transformed B cell lines from HapMap individuals.
Figure 7 depicts the expression of BLK in transformed B cells from the HapMap populations.
Figure 8 depicts the association of C8orf13/BLK and ITGAM/ITGAX region variants with SLE by case/control series.
Figure 9 depicts the association of C8orf13/BLK and ITGAM/ITGAX variants with the 11 ACR clinical criteria for SLE Series 1-3.
Figure 10 depicts the association of C8orf13/BLK and ITGAM/ITGAX variants with the 11 ACR clinical criteria for 521 Swedish SLE cases. In the Swedish samples, 521 cases were examined for an association to the ACR criteria. Statistical significance was assessed by 2x2 contingency tables and a chi square test. The calculated P-values were not adjusted for multiple testing, since the ACR criteria are known to be correlated and a simple Bonferroni correction of X=0.05/11=0.0045 would likely be overly conservative.
Figure 11 depicts the formula used to combine corrected Z scores weighted for series size and adjusted for residual genomic control inflation factor (λgc). The variance (σ2) of each series was calculated where *p* = the allele frequency in cases and controls. The combined Z score for the 3 SLE series (Z*) was calculated where Z1, Z2, and Z3 equals the Z score based on the EIGENSTRAT corrected chi square for the association of a variant to SLE from each series, and where λ1, λ2, and λ3 is the residual genomic control inflation factor (λgc) after EIGENSTRAT correction for each series.
The following key applies to the headings in Figures 12-17:

| | |
|---|---|
| ***SNP*** # | Arbitrary numbering of SNP in a specific patient subset/study group |
| ***Region*** # | Arbitrary numbering of linkage disequilibrium regions in the specific patient subsets |
| ***SNP_ID*** | SNP rsID number |
| ***EIG P*** | P value of chi-square statistic from EIGENSTRAT. |
| ***P in Main*** | P value for this SNP in the Main Group |
| ***P in Females*** | P value for this SNP in the Female Subset |
| ***Coordinate*** | SNP's base pair on its chromosome |
| ***cM*** | centiMorgans from start of chromosome |
| ***MAF CEU*** | SNP's Minor Allele Frequency from HapMap CEU samples |
| ***SNP before region*** | The SNP immediately before the linkage disequilibrium (LD) region containing the SNP indicated under SNP_ID. |
| ***SNP after region*** | The SNP immediately after the LD region |
| ***Why genes (region) chosen*** | Rationale for this region being chosen as a relevant region |
| | |
| ***1st gene in region*** | The first of the genes in the indicated region, listed in order by coordinate (base pair). |
| ***Description (Descr.)*** | Gene description from HUGO Gene Nomenclature Committee |
| ***IRIS*** | Ratio of highest immune mean / highest non-immune mean; from IRIS study |
| ***LD Region SNP*** | Any SNP located in a linkage disequilibrium region delineated by either (i) coordinate A and coordinate B, or (ii) SNP A and SNP B, inclusive. |

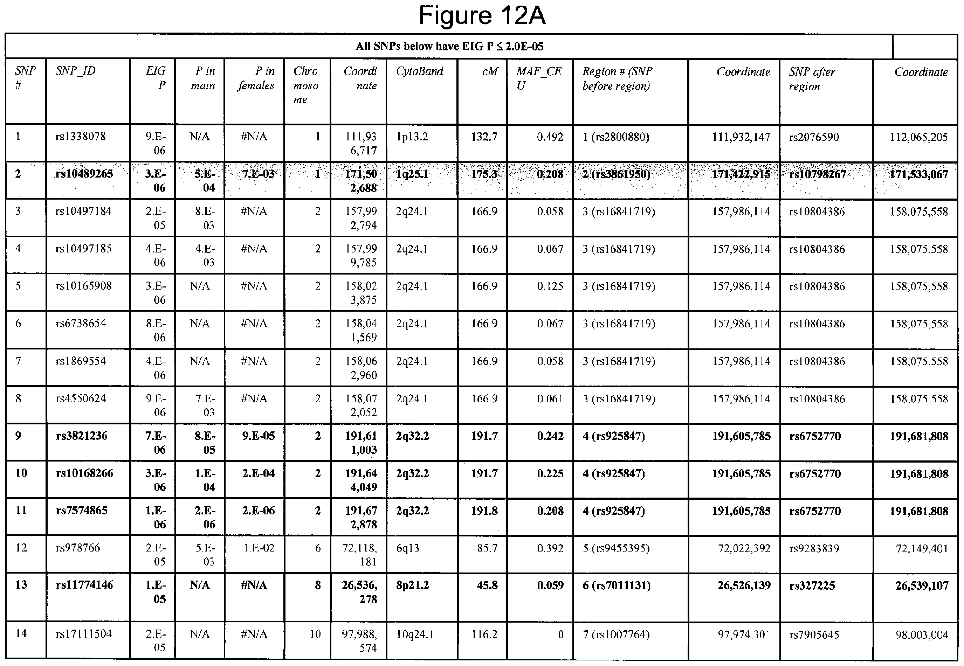

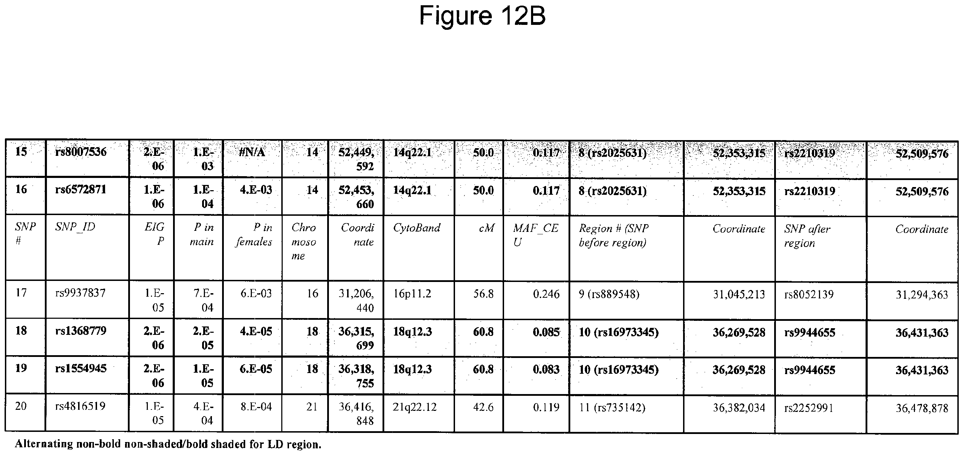

Figure 12 (A) and (B), together, depict analysis of a lupus nephritis subset, showing 11 regions containing 20 candidate SNPs deemed likely to contain at least one risk allele for lupus nephritis. (C) and (D), together, provide further characterization of linkage disequilibrium regions, identity of certain genes within these regions, and criteria for identifying such genes.

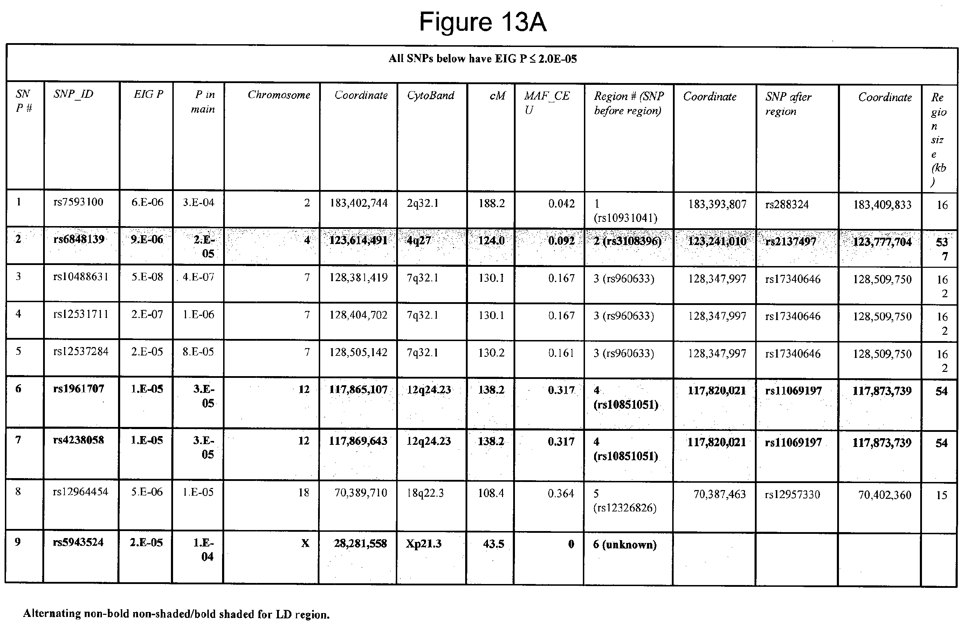

Figure 13 (A) depicts analysis of a female subset, showing 6 additional regions containing 9 candidate SNPs deemed likely to contain at least one risk allele. (B) provides further characterization of linkage disequilibrium regions, identity of certain genes within these regions, and criteria for identifying such genes.

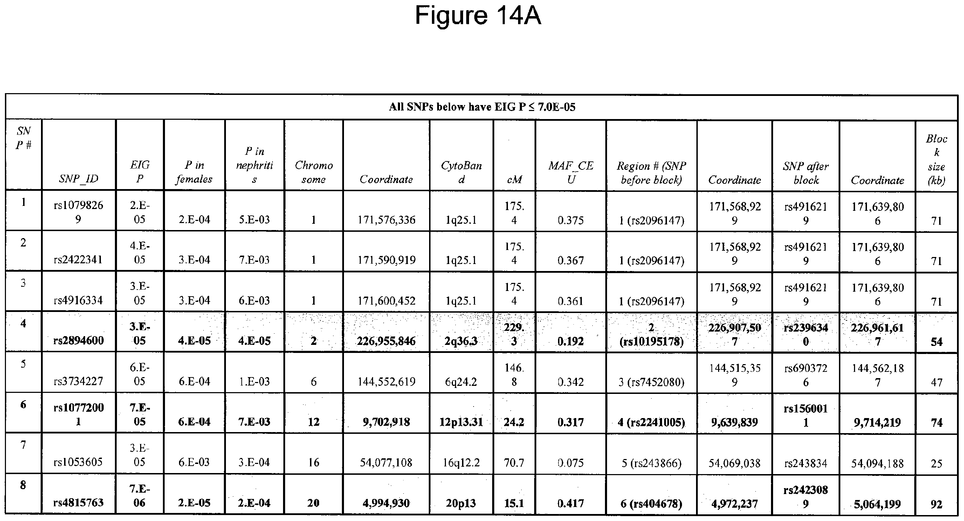

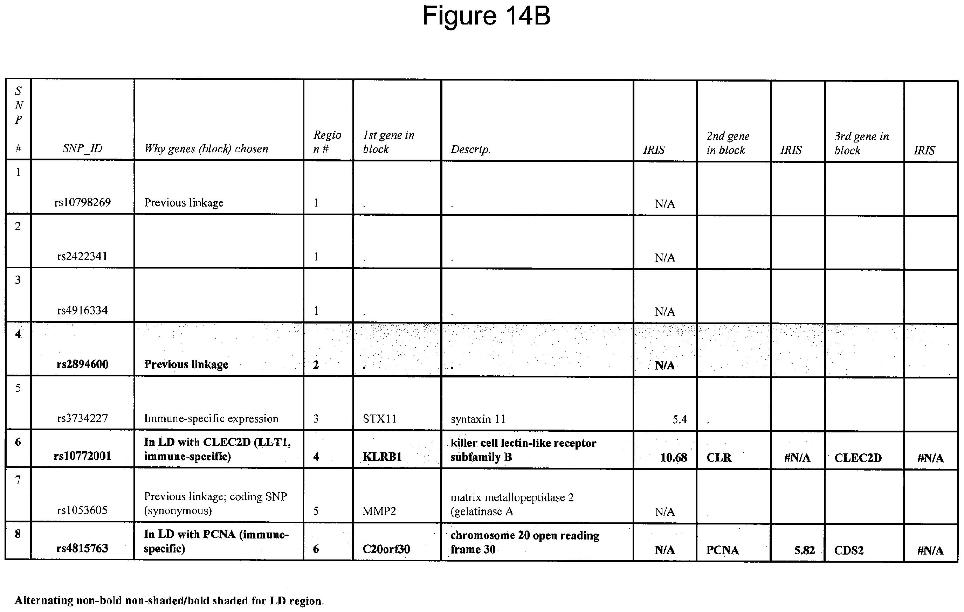

Figure 14 (A) depicts analysis of the Main Group, showing 6 additional regions containing 8 candidate SNPs deemed likely to contain at least one risk allele. Figure 14 (B) provides further characterization of linkage disequilibrium regions, certain genes within these regions, and criteria for identifying such genes.

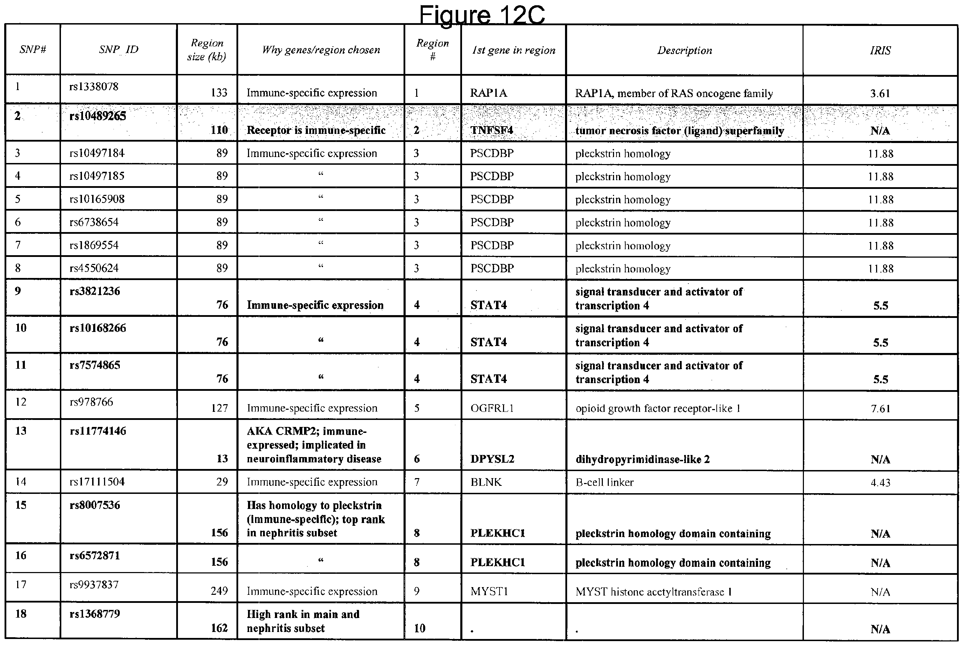

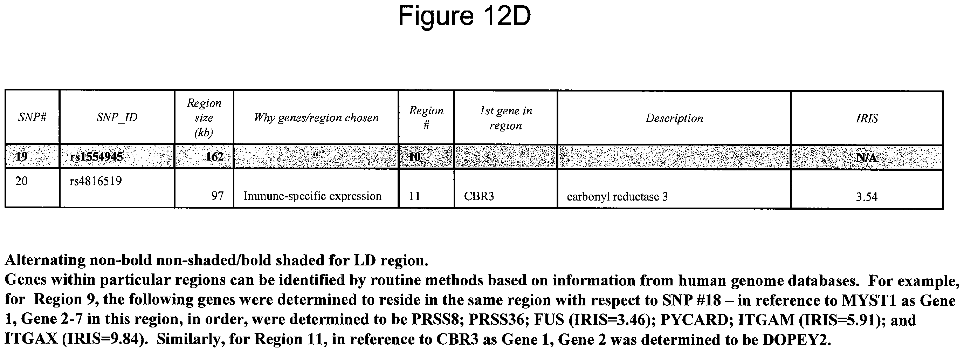

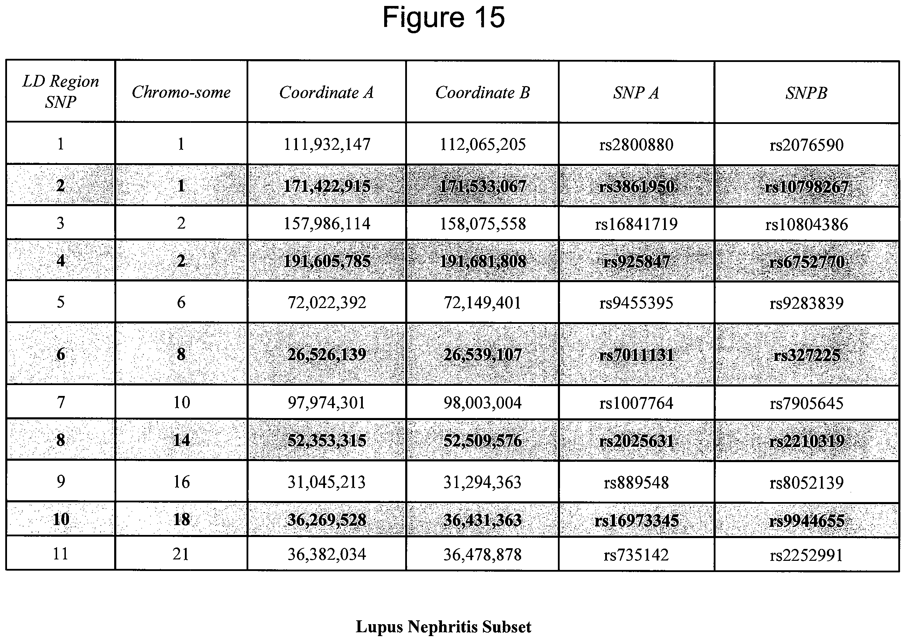

Figure 15 depicts delineation of linkage disequilibrium regions, and SNPs contained therein, based on certain data from Figure 12.

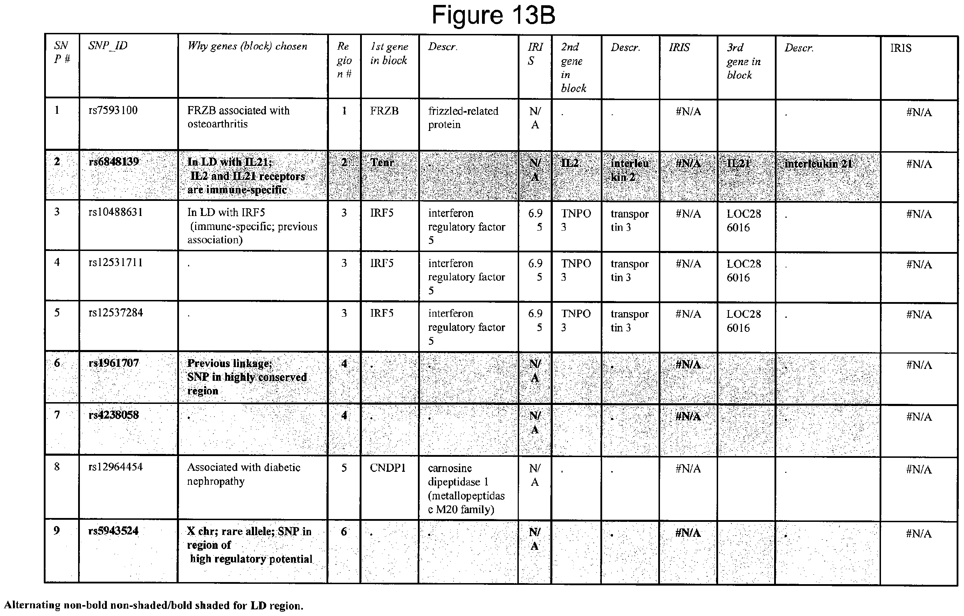

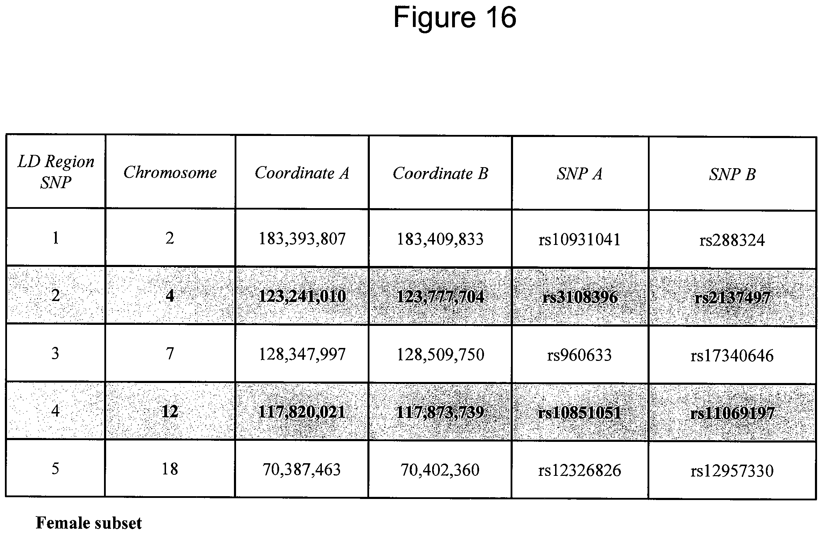

Figure 16 depicts delineation of linkage disequilibrium regions, and SNPs contained therein, based on certain data from Figure 13.

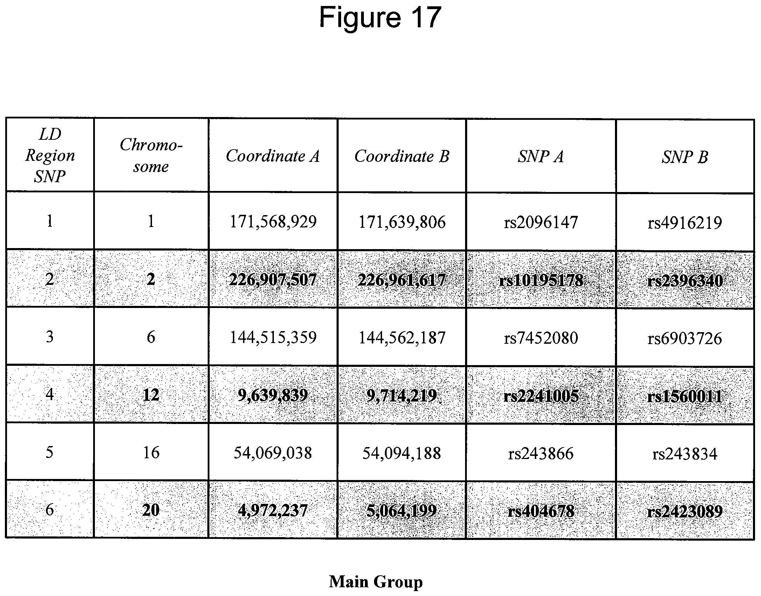

Figure 17 depicts delineation of linkage disequilibrium regions, and SNPs contained therein, based on certain data from Figure 14.

### DETAILED DESCRIPTION OF THE INVENTION

The invention provides accurate, simple, and rapid methods and compositions for identifying lupus, and for assessing risk of developing lupus, based at least in part on the identification of one or more genetic variations, e.g., SNPs, that are correlated with high statistical and biological significance with the presence, subtypes, and/or patient subpopulations of lupus. More specifically, the invention relates to the identification of a unique set of SNPs, unique combinations of such SNPs, and linkage disequilibrium regions that are associated with lupus and its subtypes, and patient subpopulations suffering from same.

In particular, the unique set and/or combinations of SNPs can be used as a genetic profile or signature indicative of a subject at risk of developing lupus, or indicative of the disease or symptom or condition thereof. The polymorphisms disclosed herein are useful as biomarkers for assessing risk of developing lupus, as well as for targets for the design of diagnostic reagents. In some embodiments, the SNP is not associated with a gene. In other embodiments, the SNP is associated with a gene, and can be located either in an intergenic or intragenic region, and more particularly, can be located in a coding or noncoding region. The genes associated with a SNP of the present invention may be associated with an unknown gene, or may be associated with a known gene e.g., ITGAM or BLK.

The SNPs identified herein provide targets for development of therapeutic agents for use in the diagnosis and treatment of genetically identified lupus patients, including diagnosis and targeted treatment of lupus patient subpopulations exhibiting a distinct genetic signature comprising one or more of the SNPs of the present invention. For example, in one embodiment, the genes containing the genetic variations identified herein, and the nucleic acid (e.g., DNA or RNA) associated with these genes, and proteins encoded by these genes, can be used as targets for the development of therapeutic agents (e.g., small molecule compounds, antibodies, antisense/RNAi agents, etc.) or used directly as therapeutic agents (e.g., therapeutic proteins, etc.) for the treatment of lupus.

### General Techniques

The practice of the present invention will employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Current Protocols in Molecular Biology" (F. M. Ausubel et al., eds., 1987, and periodic updates); "PCR: The Polymerase Chain Reaction", (Mullis et al., eds., 1994).

Primers, oligonucleotides and polynucleotides employed in the present invention can be generated using standard techniques known in the art.

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application.

### I. DEFINITIONS

For purposes of interpreting this specification, the following definitions will apply and whenever appropriate, terms used in the singular will also include the plural and vice versa. In the event that any definition set forth below conflicts with any document incorporated herein by reference, the definition set forth below shall control.

"Lupus" or "lupus condition", as used herein is an autoimmune disease or disorder that in general involves antibodies that attack connective tissue. The principal form of lupus is a systemic one, systemic lupus erythematosus (SLE), including cutaneous SLE and subacute cutaneous SLE, as well as other types of lupus (including nephritis, extrarenal, cerebritis, pediatric, non-renal, discoid, and alopecia). See, generally, D'Cruz et al., *supra*.

The term "polynucleotide" or "nucleic acid," as used interchangeably herein, refers to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after polymerization, such as by conjugation with a labeling component. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, cabamates, etc.) and with charged linkages (e.g., phosphorothioates, phosphorodithioates, etc.), those containing pendant moieties, such as, for example, proteins (e.g., nucleases, toxins, antibodies, signal peptides, poly-L-lysine, etc. ), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid supports. The 5' and 3' terminal OH can be phosphorylated or substituted with amines or organic capping groups moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-2'-O- allyl, 2'-fluoro- or 2'-azido-ribose, carbocyclic sugar analogs, α- anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR 2 ("amidate"), P(O)R, P(O)OR', CO or CH 2 ("formacetal"), in which each R or R' is independently H or substituted or unsubstituted alkyl (1-20 C) optionally containing an ether (--O--) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, refers to short, single stranded polynucleotides that are at least about seven nucleotides in length and less than about 250 nucleotides in length. Oligonucleotides may be synthetic. The terms "oligonucleotide" and "polynucleotide" are not mutually exclusive. The description above for polynucleotides is equally and fully applicable to oligonucleotides.

The term "primer" refers to a single stranded polynucleotide that is capable of hybridizing to a nucleic acid and allowing the polymerization of a complementary nucleic acid, generally by providing a free 3'-OH group.

The term "PRO" refers to a polypeptide encoded by any gene encoded by a nucleic acid sequence located within a linkage disequilibrium region (LD region), where the LD region is determined in accordance with information set forth in Figures 1-17 and Tables 1-10. In one embodiment, a PRO of the invention does not include a polypeptide known in the art to cause lupus. In one embodiment, a PRO of the invention does not include a polypeptide known in the art to be associated with lupus, e.g., IRF5, or any polypeptide encoded by a gene indicated in Tables 5-9 of WO2007/019219. The term "PRO-associated polynucleotide" or "nucleic acid associated with PRO" refers to a nucleic acid molecule that comprises a contiguous sequence, wherein the contiguous sequence comprises a position identified herein as exhibiting genetic variation. In one embodiment, the position exhibiting genetic variation is located at the 5' or 3' end of the contiguous sequence. In one embodiment, the position exhibiting genetic variation in the contiguous sequence is flanked, at either or both its 5' and/or 3' regions, by one or more nucleotides that constitute the position's naturally-occurring flanking sequence. In one embodiment, a position exhibiting genetic variation is a position corresponding to a SNP indicated in any of Figures 1-17 and Tables 1-10.

The term "genetic variation" or "nucleotide variation" refers to a change in a nucleotide sequence (e.g., an insertion, deletion, inversion, or substitution of one or more nucleotides, such as a single nucleotide polymorphism (SNP)) relative to a reference sequence (e.g., a commonly-found and/or wild-type sequence, and/or the sequence of a major allele). The term also encompasses the corresponding change in the complement of the nucleotide sequence, unless otherwise indicated. In one embodiment, a genetic variation is a somatic polymorphism. In one embodiment, a genetic variation is a germline polymorphism.

A "single nucleotide polymorphism", or "SNP", refers to a single base position in an RNA or DNA molecule (e.g., a polynucleotide), at which different alleles, or alternative nucleotides, exist in a population. The SNP position (interchangeably referred to herein as SNP, SNP site, SNP locus) is usually preceded by and followed by highly conserved sequences of the allele (e.g., sequences that vary in less than 1/100 or 1/1000 members of the populations). An individual may be homozygous or heterozygous for an allele at each SNP position.

The term "amino acid variation" refers to a change in an amino acid sequence (e.g., an insertion, substitution, or deletion of one or more amino acids, such as an internal deletion or an N- or C-terminal truncation) relative to a reference sequence.

The term "variation" refers to either a nucleotide variation or an amino acid variation.

The term "a genetic variation at a nucleotide position corresponding to a SNP," "a nucleotide variation at a nucleotide position corresponding to a SNP," and grammatical variants thereof refer to a nucleotide variation in a polynucleotide sequence at the relative corresponding nucleotide position occupied by the SNP in the genome. The term also encompasses the corresponding variation in the complement of the nucleotide sequence, unless otherwise indicated. In some embodiments, the nucleotide variation is in a PRO-associated polynucleotide sequence at the relative corresponding nucleotide position occupied by the SNP in the genome.

The term "linkage disequilibrium region SNP," or "LD region SNP" refers to a SNP present in a specific region of DNA, such region delineated by appropriate nucleic acid/genomic markers, e.g., coordinates or SNPs. In one embodiment, a LD region is delineated by a first coordinate (e.g., coordinate A) and a second coordinate (e.g., coordinate B), both coordinates referring to the same chromosome. In one embodiment, a LD region is delineated by a first SNP (e.g., SNP A) and a second SNP (e.g., SNP B). Thus, in one embodiment, a LD region SNP refers to a SNP located in a nucleic acid region (e.g., genomic region) ranging from a first coordinate to a second coordinate, or a first SNP to a second SNP. Examples of such LD regions and LD region SNPs are shown in Figures 1-17 and Tables 1-10.

The term "array" or "microarray" refers to an ordered arrangement of hybridizable array elements, preferably polynucleotide probes (e.g., oligonucleotides), on a substrate. The substrate can be a solid substrate, such as a glass slide, or a semi-solid substrate, such as nitrocellulose membrane.

The term "amplification" refers to the process of producing one or more copies of a reference nucleic acid sequence or its complement. Amplification may be linear or exponential (e.g., PCR). A "copy" does not necessarily mean perfect sequence complementarity or identity relative to the template sequence. For example, copies can include nucleotide analogs such as deoxyinosine, intentional sequence alterations (such as sequence alterations introduced through a primer comprising a sequence that is hybridizable, but not fully complementary, to the template), and/or sequence errors that occur during amplification.

The term "allele-specific oligonucleotide" refers to an oligonucleotide that hybridizes to a region of a target nucleic acid that comprises a nucleotide variation (generally a substitution). "Allele-specific hybridization" means that, when an allele-specific oligonucleotide is hybridized to its target nucleic acid, a nucleotide in the allele-specific oligonucleotide specifically base pairs with the nucleotide variation. An allele-specific oligonucleotide capable of allele-specific hybridization with respect to a particular nucleotide variation is said to be "specific for" that variation.

The term "allele-specific primer" refers to an allele-specific oligonucleotide that is a primer.

The term "primer extension assay" refers to an assay in which nucleotides are added to a nucleic acid, resulting in a longer nucleic acid, or "extension product," that is detected directly or indirectly. The nucleotides can be added to extend the 5' or 3' end of the nucleic acid.

The term "allele-specific nucleotide incorporation assay" refers to a primer extension assay in which a primer is (a) hybridized to target nucleic acid at a region that is 3' or 5' of a nucleotide variation and (b) extended by a polymerase, thereby incorporating into the extension product a nucleotide that is complementary to the nucleotide variation.

The term "allele-specific primer extension assay" refers to a primer extension assay in which an allele-specific primer is hybridized to a target nucleic acid and extended.

The term "allele-specific oligonucleotide hybridization assay" refers to an assay in which (a) an allele-specific oligonucleotide is hybridized to a target nucleic acid and (b) hybridization is detected directly or indirectly.

The term "5' nuclease assay" refers to an assay in which hybridization of an allele-specific oligonucleotide to a target nucleic acid allows for nucleolytic cleavage of the hybridized probe, resulting in a detectable signal.

The term "assay employing molecular beacons" refers to an assay in which hybridization of an allele-specific oligonucleotide to a target nucleic acid results in a level of detectable signal that is higher than the level of detectable signal emitted by the free oligonucleotide.

The term "oligonucleotide ligation assay" refers to an assay in which an allele-specific oligonucleotide and a second oligonucleotide are hybridized adjacent to one another on a target nucleic acid and ligated together (either directly or indirectly through intervening nucleotides), and the ligation product is detected directly or indirectly.

The term "target sequence," "target nucleic acid," or "target nucleic acid sequence" refers generally to a polynucleotide sequence of interest in which a nucleotide variation is suspected or known to reside, including copies of such target nucleic acid generated by amplification.

As used herein, a subject "at risk" of developing lupus may or may not have detectable disease or symptoms of disease, and may or may not have displayed detectable disease or symptoms of disease prior to the treatment methods described herein. "At risk" denotes that a subject has one or more risk factors, which are measurable parameters that correlate with development of lupus, as described herein and known in the art. A subject having one or more of these risk factors has a higher probability of developing lupus than a subject without one or more of these risk factor(s). For example, in some embodiments, a subject "at risk" of developing lupus has a genetic signature comprising one or more of the SNPs set forth in Figures 1-17 and Tables 1-10. In another embodiment, a subject "at risk" of developing lupus has a genetic signature comprising one or more of the SNPs set forth in Table 6.

The term "detection" includes any means of detecting, including direct and indirect detection.

The term "diagnosis" is used herein to refer to the identification or classification of a molecular or pathological state, disease or condition. For example, "diagnosis" may refer to identification of a particular type of lupus condition, e.g., SLE. "Diagnosis" may also refer to the classification of a particular sub-type of lupus, e.g., by tissue/organ involvement (e.g., lupus nephritis), by molecular features (e.g., a patient subpopulation characterized by genetic variation(s) in a particular gene or nucleic acid region.)

The term "aiding diagnosis" is used herein to refer to methods that assist in making a clinical determination regarding the presence, degree or other nature, of a particular type of symptom or condition of lupus. For example, a method of aiding diagnosis of lupus can comprise measuring the amount or detecting the presence orabsence of one or more SNPs in a biological sample from an individual. In another example, a method of aiding diagnosis of lupus can comprise measuring the amount or detecting the presence of one or more SNPsin a biological sample from an individual.

The term "prognosis" is used herein to refer to the prediction of the likelihood of autoimmune disorder-attributable disease symptoms, including, for example, recurrence, flaring, and drug resistance, of an autoimmune disease such as lupus. The term "prediction" is used herein to refer to the likelihood that a patient will respond either favorably or unfavorably to a drug or set of drugs. In one embodiment, the prediction relates to the extent of those responses. In one embodiment, the prediction relates to whether and/or the probability that a patient will survive or improve following treatment, for example treatment with a particular therapeutic agent, and for a certain period of time without disease recurrence. The predictive methods of the invention can be used clinically to make treatment decisions by choosing the most appropriate treatment modalities for any particular patient. The predictive methods of the present invention are valuable tools in predicting if a patient is likely to respond favorably to a treatment regimen, such as a given therapeutic regimen, including for example, administration of a given therapeutic agent or combination, surgical intervention, steroid treatment, etc., or whether long-term survival of the patient, following a therapeutic regimen is likely. Diagnosis of SLE may be according to current American College of Rheumatology (ACR) criteria. Active disease may be defined by one British Isles Lupus Activity Group's (BILAG) "A" criteria or two BILAG "B" criteria. Some signs, symptoms, or other indicators used to diagnose SLE adapted from: Tan et al. "The Revised Criteria for the Classification of SLE" Arth Rheum 25 (1982) may be malar rash such as rash over the cheeks, discoid rash, or red raised patches, photosensitivity such as reaction to sunlight, resulting in the development of or increase in skin rash, oral ulcers such as ulcers in the nose or mouth, usually painless, arthritis, such as non-erosive arthritis involving two or more peripheral joints (arthritis in which the bones around the joints do not become destroyed), serositis, pleuritis or pericarditis, renal disorder such as excessive protein in the urine (greater than 0.5 gm/day or 3+ on test sticks) and/or cellular casts (abnormal elements derived from the urine and/or white cells and/or kidney tubule cells), neurologic signs, symptoms, or other indicators, seizures (convulsions), and/or psychosis in the absence of drugs or metabolic disturbances that are known to cause such effects, and hematologic signs, symptoms, or other indicators such as hemolytic anemia or leukopenia (white bloodcount below 4,000 cells per cubic millimeter) or lymphopenia (less than 1,500 lymphocytes per cubic millimeter) or thrombocytopenia (less than 100,000 platelets per cubic millimeter). The leukopenia and lymphopenia generally must be detected on two or more occasions. The thrombocytopenia generally must be detected in the absence of drugs known to induce it. The invention is not limited to these signs, symptoms, or other indicators of lupus.

As used herein, "treatment" refers to clinical intervention in an attempt to alter the natural course of the individual or cell being treated, and can be performed before or during the course of clinical pathology. Desirable effects of treatment include preventing the occurrence or recurrence of a disease or a condition or symptom thereof, alleviating a condition or symptom of the disease, diminishing any direct or indirect pathological consequences of the disease, decreasing the rate of disease progression, ameliorating or palliating the disease state, and achieving remission or improved prognosis. In some embodiments, methods and compositions of the invention are useful in attempts to delay development of a disease or disorder.

An "effective amount" refers to an amount effective, at dosages and for periods of time necessary, to achieve the desired therapeutic or prophylactic result. A "therapeutically effective amount" of a therapeutic agent may vary according to factors such as the disease state, age, sex, and weight of the individual, and the ability of the antibody to elicit a desired response in the individual. A therapeutically effective amount is also one in which any toxic or detrimental effects of the therapeutic agent are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

An "individual," "subject" or "patient" is a vertebrate. In certain embodiments, the vertebrate is a mammal. Mammals include, but are not limited to, primates (including human and non-human primates) and rodents (e.g., mice and rats). In certain embodiments, a mammal is a human.

A "patient subpopulation," and grammatical variations thereof, as used herein, refers to a patient subset characterized as having one or more distinctive measurable and/or identifiable characteristics that distinguishes the patient subset from others in the broader disease category to which it belongs. Such characteristics include disease subcategories (e.g., SLE, lupus nephritis), gender, lifestyle, health history, organs/tissues involved, treatment history, etc. In one embodiment, a patient subpopulation is characterized by genetic signatures, including genetic variations in particular nucleotide positions and/or regions (such as SNPs).

The term "sample", as used herein, refers to a composition that is obtained or derived from a subject of interest that contains a cellular and/or other molecular entity that is to be characterized and/or identified, for example based on physical, biochemical, chemical and/or physiological characteristics. For example, the phrase "disease sample" and variations thereof refers to any sample obtained from a subject of interest that would be expected or is known to contain the cellular and/or molecular entity that is to be characterized.

By "tissue or cell sample" is meant a collection of similar cells obtained from a tissue of a subject or patient. The source of the tissue or cell sample may be solid tissue as from a fresh, frozen and/or preserved organ or tissue sample or biopsy or aspirate; blood or any blood constituents; bodily fluids such as cerebral spinal fluid, amniotic fluid, peritoneal fluid, or interstitial fluid; cells from any time in gestation or development of the subject. The tissue sample may also be primary or cultured cells or cell lines. Optionally, the tissue or cell sample is obtained from a disease tissue/organ. The tissue sample may contain compounds which are not naturally intermixed with the tissue in nature such as preservatives, anticoagulants, buffers, fixatives, nutrients, antibiotics, or the like. A "reference sample", "reference cell", "reference tissue", "control sample", "control cell", or "control tissue", as used herein, refers to a sample, cell or tissue obtained from a source known, or believed, not to be afflicted with the disease or condition for which a method or composition of the invention is being used to identify. In one embodiment, a reference sample, reference cell, reference tissue, control sample, control cell, or control tissue is obtained from a healthy part of the body of the same subject or patient in whom a disease or condition is being identified using a composition or method of the invention. In one embodiment, a reference sample, reference cell, reference tissue, control sample, control cell, or control tissue is obtained from a healthy part of the body of an individual who is not the subject or patient in whom a disease or condition is being identified using a composition or method of the invention.

For the purposes herein a "section" of a tissue sample is meant a single part or piece of a tissue sample, *e.g.* a thin slice of tissue or cells cut from a tissue sample. It is understood that multiple sections of tissue samples may be taken and subjected to analysis according to the present invention, provided that it is understood that the present invention comprises a method whereby the same section of tissue sample is analyzed at both morphological and molecular levels, or is analyzed with respect to both protein and nucleic acid.

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to the embodiment of gene expression analysis or protocol, one may use the results of the gene expression analysis or protocol to determine whether a specific therapeutic regimen should be performed.

The word "label" when used herein refers to a compound or composition which is conjugated or fused directly or indirectly to a reagent such as a nucleic acid probe or an antibody and facilitates detection of the reagent to which it is conjugated or fused. The label may itself be detectable (*e.g*., radioisotope labels or fluorescent labels) or, in the case of an enzymatic label, may catalyze chemical alteration of a substrate compound or composition which is detectable.

A "medicament" is an active drug to treat a disease, disorder, and/or condition. In one embodiment, the disease, disorder, and/or condition is lupus or its symptoms or side effects.

The term "increased resistance" to a particular therapeutic agent or treatment option, when used in accordance with the invention, means decreased response to a standard dose of the drug or to a standard treatment protocol.

The term "decreased sensitivity" to a particular therapeutic agent or treatment option, when used in accordance with the invention, means decreased response to a standard dose of the agent or to a standard treatment protocol, where decreased response can be compensated for (at least partially) by increasing the dose of agent, or the intensity of treatment.

"Patient response" can be assessed using any endpoint indicating a benefit to the patient, including, without limitation, (1) inhibition, to some extent, of disease progression, including slowing down and complete arrest; (2) reduction in the number of disease episodes and/or symptoms; (3) reduction in lesional size; (4) inhibition (i.e., reduction, slowing down or complete stopping) of disease cell infiltration into adjacent peripheral organs and/or tissues; (5) inhibition (i.e. reduction, slowing down or complete stopping) of disease spread; (6) decrease of auto-immune response, which may, but does not have to, result in the regression or ablation of the disease lesion; (7) relief, to some extent, of one or more symptoms associated with the disorder; (8) increase in the length of disease-free presentation following treatment; and/or (9) decreased mortality at a given point of time following treatment.

The term "antagonist" is used in the broadest sense, and includes any molecule that partially or fully inhibits or neutralizes a biological activity of a polypeptide, such as PRO, or that partially or fully inhibits the transcription or translation of a nucleic acid encoding the polypeptide. Exemplary antagonist molecules include, but are not limited to, antagonist antibodies, polypeptide fragments, oligopeptides, organic molecules (including small molecules), and anti-sense nucleic acids.

The term "agonist" is used in the broadest sense, and includes any molecule that partially or fully mimics a biological activity of a polypeptide, such as PRO, or that increases the transcription or translation of a nucleic acid encoding the polypeptide. Exemplary agonist molecules include, but are not limited to, agonist antibodies, polypeptide fragments, oligopeptides, organic molecules (including small molecules), PRO-associated polynucleotides, PRO polypeptides, and PRO-Fc fusions.

A "therapeutic agent that targets a PRO or a PRO-associated polynucleotide" means any agent that affects the expression and/or activity of PRO or a PRO-associated polynucleotide including, but not limited to, any of the PRO agonists or antagonists described herein, including such therapeutic agents that are already known in the art as well as those that are later developed.

A "lupus therapeutic agent", a "therapeutic agent effective to treat lupus", and grammatical variations thereof, as used herein, refer to an agent that when provided in an effective amount is known, clinically shown, or expected by clinicians to provide a therapeutic benefit in a subject who has lupus. In one embodiment, the phrase includes any agent that is marketed by a manufacturer, or otherwise used by licensed clinicians, as a clinically-accepted agent that when provided in an effective amount would be expected to provide a therapeutic effect in a subject who has lupus. In one embodiment, a lupus therapeutic agent comprises a non-steroidal anti-inflammatory drug (NSAID), which includes acetylsalicylic acid (e.g., aspirin), ibuprofen (Motrin), naproxen (Naprosyn), indomethacin (Indocin), nabumetone (Relafen), tolmetin (Tolectin), and any other embodiments that comprise a therapeutically equivalent active ingredient(s) and formulation thereof. In one embodiment, a lupus therapeutic agent comprises acetaminophen (e.g., Tylenol), corticosteroids, or anti-malarials (e.g., chloroquine, hydroxychloroquine). In one embodiment, a lupus therapeutic agent comprises an immunomodulating drug (e.g., azathioprine, cyclophosphamide, methotrexate, cyclosporine). In one embodiment, a lupus therapeutic agent is an anti-B cell agent (e.g., anti-CD20 (e.g., rituximab), anti-CD22), an anti-cytokine agent (e.g., anti-tumor necrosis factor α, anti-interleukin-1-receptor (e.g., anakinra), anti-interleukin 10, anti-interleukin 6 receptor, anti-interferon alpha, anti-B-lymphocyte stimulator), an inhibitor of costimulation (e.g., anti-CD154, CTLA4-Ig (e.g., abatacept)), a modulator of B-cell anergy (e.g., LJP 394 (e.g., abetimus)). In one embodiment, a lupus therapeutic agent comprises hormonal treatment (*e.g*., DHEA), and anti-hormonal therapy (*e.g*., the anti-prolactin agent bromocriptine). In one embodiment, a lupus therapeutic agent is an agent that provides immunoadsorption, is an anti-complement factor (e.g., anti-C5a), T cell vaccination, cell transfection with T-cell receptor zeta chain, or peptide therapies (e.g., edratide targeting anti-DNA idiotypes).

A therapeutic agent that has "marketing approval", or that has been "approved as a therapeutic agent", or grammatical variations thereof of these phrases, as used herein, refer to an agent (e.g., in the form of a drug formulation, medicament) that is approved, licensed, registered or authorized by a relevant governmental entity (e.g., federal, state or local regulatory agency, department, bureau) to be sold by and/or through and/or on behalf of a commercial entity (e.g., a for-profit entity) for the treatment of a particular disorder (e.g., lupus) or a patient subpopulation (e.g., patients with lupus nephritis, patients of a particular ethnicity, gender, lifestyle, disease risk profile, etc.). A relevant governmental entity includes, for example, the Food and Drug Administration (FDA), European Medicines Evaluation Agency (EMEA), and equivalents thereof.

"Antibodies" (Abs) and "immunoglobulins" (Igs) refer to glycoproteins having similar structural characteristics. While antibodies exhibit binding specificity to a specific antigen, immunoglobulins include both antibodies and other antibody-like molecules which generally lack antigen specificity. Polypeptides of the latter kind are, for example, produced at low levels by the lymph system and at increased levels by myelomas.

The terms "antibody" and "immunoglobulin" are used interchangeably in the broadest sense and include monoclonal antibodies (*e.g*., full length or intact monoclonal antibodies), polyclonal antibodies, monovalent antibodies, multivalent antibodies, multispecific antibodies (*e.g.*, bispecific antibodies so long as they exhibit the desired biological activity) and may also include certain antibody fragments (as described in greater detail herein). An antibody can be chimeric, human, humanized and/or affinity matured.

The term "anti-PRO antibody" or "an antibody that binds to PRO" refers to an antibody that is capable of binding PRO with sufficient affinity such that the antibody is useful as a diagnostic and/or therapeutic agent in targeting PRO. Preferably, the extent of binding of an anti-PRO antibody to an unrelated, non-PRO protein is less than about 10% of the binding of the antibody to PRO as measured, e.g., by a radioimmunoassay (RIA). In certain embodiments, an antibody that binds to PRO has a dissociation constant (Kd) of ≤ 1µM, ≤ 100 nM, ≤ 10 nM, ≤ 1 nM, or ≤ 0.1 nM. In certain embodiments, an anti-PRO antibody binds to an epitope of PRO that is conserved among PRO from different species.

The terms "full length antibody," "intact antibody" and "whole antibody" are used herein interchangeably to refer to an antibody in its substantially intact form, not antibody fragments as defined below. The terms particularly refer to an antibody with heavy chains that contain the Fc region.

"Antibody fragments" comprise a portion of an intact antibody, preferably comprising the antigen binding region thereof. Examples of antibody fragments include Fab, Fab', F(ab')₂, and Fv fragments; diabodies; linear antibodies; single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-combining sites and is still capable of cross-linking antigen.

"Fv" is a minimum antibody fragment which contains a complete antigen-binding site. In one embodiment, a two-chain Fv species consists of a dimer of one heavy- and one light-chain variable domain in tight, non-covalent association. Collectively, the six CDRs of an Fv confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment contains the heavy- and light-chain variable domains and also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv see Pluckthun, in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 404,097; WO93/1161; Hudson et al. (2003) Nat. Med. 9:129-134; and Hollinger et al., Proc. Natl. Acad. Sci. USA 90: 6444-6448 (1993). Triabodies and tetrabodies are also described in Hudson et al. (2003) Nat. Med. 9:129-134.

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity *in vivo,* to create a multispecific antibody, etc., and that an antibody comprising the altered target binding sequence is also a monoclonal antibody of this invention. In contrast to polyclonal antibody preparations which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g., Kohler et al., Nature, 256: 495 (1975); Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, e.g., U.S. Patent No. 4,816,567), phage display technologies (see, e.g., Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, Proc. Natl. Acad Sci. USA 101(34): 12467-12472 (2004); and Lee et al., J. Immunol. Methods 284(1-2): 119-132(2004), and technologies for producing human or human-like antibodies in animals that have parts or all of the human immunoglobulin loci or genes encoding human immunoglobulin sequences (see, e.g., WO98/24893; WO96/34096; WO96/33735; WO91/10741; Jakobovits et al., Proc. Natl. Acad Sci. USA 90: 2551 (1993); Jakobovits et al., Nature 362: 255-258 (1993); Bruggemann et al., Year in Immunol. 7:33 (1993); U.S. Patent Nos. 5,545,807; 5,545,806; 5,569,825; 5,625,126; 5,633,425; 5,661,016; Marks et al., Bio.Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996) and Lonberg and Huszar, Intern. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6855-9855 (1984)).

"Humanized" forms of non-human (*e.g*., murine) antibodies are chimeric antibodies that contain minimal sequence derived from non-human immunoglobulin. In one embodiment, a humanized antibody is a human immunoglobulin (recipient antibody) in which residues from a hypervariable region of the recipient are replaced by residues from a hypervariable region of a non-human species (donor antibody) such as mouse, rat, rabbit, or nonhuman primate having the desired specificity, affinity, and/or capacity. In some instances, framework region (FR) residues of the human immunoglobulin are replaced by corresponding non-human residues. Furthermore, humanized antibodies may comprise residues that are not found in the recipient antibody or in the donor antibody. These modifications may be made to further refine antibody performance. In general, a humanized antibody will comprise substantially all of at least one, and typically two, variable domains, in which all or substantially all of the hypervariable loops correspond to those of a non-human immunoglobulin, and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994).

A "human antibody" is one which comprises an amino acid sequence corresponding to that of an antibody produced by a human and/or has been made using any of the techniques for making human antibodies as disclosed herein. Such techniques include screening human-derived combinatorial libraries, such as phage display libraries (see, e.g., Marks et al., J. Mol. Biol., 222: 581-597 (1991) and Hoogenboom et al., Nucl. Acids Res., 19: 4133-4137 (1991)); using human myeloma and mouse-human heteromyeloma cell lines for the production of human monoclonal antibodies (see, e.g., Kozbor J. Immunol., 133: 3001 (1984); Brodeur et al., Monoclonal Antibody Production Techniques and Applications, pp. 55-93 (Marcel Dekker, Inc., New York, 1987); and Boerner et al., J. Immunol., 147: 86 (1991)); and generating monoclonal antibodies in transgenic animals (e.g., mice) that are capable of producing a full repertoire of human antibodies in the absence of endogenous immunoglobulin production (see, e.g., Jakobovits et al., Proc. Natl. Acad. Sci USA, 90: 2551 (1993); Jakobovits et al., Nature, 362: 255 (1993); Bruggermann et al., Year in Immunol., 7: 33 (1993)). This definition of a human antibody specifically excludes a humanized antibody comprising antigen-binding residues from a non-human animal.

An "affinity matured" antibody is one with one or more alterations in one or more CDRs thereof which result in an improvement in the affinity of the antibody for antigen, compared to a parent antibody which does not possess those alteration(s). In one embodiment, an affinity matured antibody has nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of HVR and/or framework residues is described by: Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

A "blocking antibody" or an "antagonist antibody" is one which inhibits or reduces a biological activity of the antigen it binds. Certain blocking antibodies or antagonist antibodies partially or completely inhibit the biological activity of the antigen.

A "small molecule" or "small organic molecule" is defined herein as an organic molecule having a molecular weight below about 500 Daltons.

A "PRO-binding oligopeptide" or an "oligopeptide that binds PRO" is an oligopeptide that is capable of binding PRO with sufficient affinity such that the oligopeptide is useful as a diagnostic and/or therapeutic agent in targeting PRO. In certain embodiments, the extent of binding of a PRO-binding oligopeptide to an unrelated, non-PRO protein is less than about 10% of the binding of the PRO-binding oligopeptide to PRO as measured, e.g., by a surface plasmon resonance assay. In certain embodiments, a PRO-binding oligopeptide has a dissociation constant (Kd) of ≤ 1µM, ≤ 100 nM, ≤ 10 nM, ≤ 1 nM, or ≤ 0.1 nM.

A "PRO-binding organic molecule" or "an organic molecule that binds PRO" is an organic molecule other than an oligopeptide or antibody as defined herein that is capable of binding PRO with sufficient affinity such that the organic molecule is useful as a diagnostic and/or therapeutic agent in targeting PRO. In certain embodiments, the extent of binding of a PRO-binding organic molecule to an unrelated, non-PRO protein is less than about 10% of the binding of the PRO-binding organic molecule to PRO as measured, e.g., by a surface plasmon resonance assay. In certain embodiments, a PRO-binding organic molecule has a dissociation constant (Kd) of ≤ 1µM, ≤ 100 nM, ≤ 10 nM, ≤ 1 nM, or ≤ 0.1 nM.

The dissociation constant (Kd) of any molecule that binds a target polypeptide may conveniently be measured using a surface plasmon resonance assay. Such assays may employ a BIAcore™-2000 or a BIAcore™-3000 (BIAcore, Inc., Piscataway, NJ) at 25°C with immobilized target polypeptide CM5 chips at ∼10 response units (RU). Briefly, carboxymethylated dextran biosensor chips (CM5, BIAcore Inc.) are activated with *N*-ethyl-*N'-*(3-dimethylaminopropyl)-carbodiimide hydrochloride (EDC) and *N*-hydroxysuccinimide (NHS) according to the supplier's instructions. Target polypeptide is diluted with 10 mM sodium acetate, pH 4.8, to 5 µg/ml (∼0.2 µM) before injection at a flow rate of 5 µl/minute to achieve approximately 10 response units (RU) of coupled protein. Following the injection of target polypeptide, 1 M ethanolamine is injected to block unreacted groups. For kinetics measurements, two-fold serial dilutions of the binding molecule (0.78 nM to 500 nM) are injected in PBS with 0.05% Tween 20 (PBST) at 25°C at a flow rate of approximately 25 µl/min. Association rates (kₒₙ) and dissociation rates (k_{off}) are calculated using a simple one-to-one Langmuir binding model (BIAcore Evaluation Software version 3.2) by simultaneously fitting the association and dissociation sensorgrams. The equilibrium dissociation constant (Kd) is calculated as the ratio k_{off}/kₒₙ. See, e.g., Chen, Y., et al., (1999) J. Mol. Biol. 293:865-881. If the on-rate of an antibody exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM antibody (Fab form) in PBS, pH 7.2, in the presence of increasing concentrations of antigen as measured in a spectrometer, such as a stop-flow equipped spectrophometer (Aviv Instruments) or a 8000-series SLM-Aminco spectrophotometer (ThermoSpectronic) with a stirred cuvette.

A "liposome" is a small vesicle composed of various types of lipids, phospholipids and/or surfactant which is useful for delivery of an agent, e.g., a drug, to a mammal. The components of the liposome are commonly arranged in a bilayer formation, similar to the lipid arrangement of biological membranes.

The word "label" when used herein refers to a detectable compound or composition. The label may be detectable by itself (*e.g*., radioisotope labels or fluorescent labels) or, in the case of an enzymatic label, may catalyze chemical alteration of a substrate compound or composition which results in a detectable product. Radionuclides that can serve as detectable labels include, for example, I-131, I-123, I-125, Y-90, Re-188, Re-186, At-211, Cu-67, Bi-212, and Pd-109.

An "isolated" biological molecule, such as a nucleic acid, polypeptide, or antibody, is one which has been identified and separated and/or recovered from at least one component of its natural environment.

Reference to "about" a value or parameter herein includes (and describes) embodiments that are directed to that value or parameter per se. For example, description referring to "about X" includes description of "X."

It is understood that aspect and embodiments of the invention described herein include "consisting" and/or "consisting essentially of" aspects and embodiments.

### II. GENERAL TECHNIQUES FOR CARRYING OUT COMPOSITIONS AND METHODS OF THE INVENTION

Nucleotide variations associated with lupus are provided herein. These variations provide biomarkers for lupus, and/or predispose or contribute to development, persistence and/or progression of lupus. Accordingly, the invention disclosed herein is useful in a variety of settings, e.g., in methods and compositions related to lupus diagnosis and therapy.

### Detection of Genetic Variations

Nucleic acid, according to any of the above methods, may be genomic DNA; RNA transcribed from genomic DNA; or cDNA generated from RNA. Nucleic acid may be derived from a vertebrate, e.g., a mammal. A nucleic acid is said to be "derived from" a particular source if it is obtained directly from that source or if it is a copy of a nucleic acid found in that source.

Nucleic acid includes copies of the nucleic acid, e.g., copies that result from amplification. Amplification may be desirable in certain instances, e.g., in order to obtain a desired amount of material for detecting variations. For example, a PRO-associated polynucleotide or portion thereof may be amplified from nucleic acid material. The amplicons may then be subjected to a variation detection method, such as those described below, to determine whether a variation is present in the amplicon.

Variations may be detected by certain methods known to those skilled in the art. Such methods include, but are not limited to, DNA sequencing; primer extension assays, including allele-specific nucleotide incorporation assays and allele-specific primer extension assays (e.g., allele-specific PCR, allele-specific ligation chain reaction (LCR), and gap-LCR); allele-specific oligonucleotide hybridization assays (e.g., oligonucleotide ligation assays); cleavage protection assays in which protection from cleavage agents is used to detect mismatched bases in nucleic acid duplexes; analysis of MutS protein binding; electrophoretic analysis comparing the mobility of variant and wild type nucleic acid molecules; denaturing-gradient gel electrophoresis (DGGE, as in, e.g., Myers et al. (1985) Nature 313:495); analysis of RNase cleavage at mismatched base pairs; analysis of chemical or enzymatic cleavage of heteroduplex DNA; mass spectrometry (e.g., MALDI-TOF); genetic bit analysis (GBA); 5' nuclease assays (e.g., TaqMan®); and assays employing molecular beacons. Certain of these methods are discussed in further detail below.

Detection of variations in target nucleic acids may be accomplished by molecular cloning and sequencing of the target nucleic acids using techniques well known in the art. Alternatively, amplification techniques such as the polymerase chain reaction (PCR) can be used to amplify target nucleic acid sequences directly from a genomic DNA preparation from tumor tissue. The nucleic acid sequence of the amplified sequences can then be determined and variations identified therefrom. Amplification techniques are well known in the art, e.g., polymerase chain reaction is described in Saiki et al., Science 239:487, 1988; U.S. Pat. Nos. 4,683,203 and 4,683,195.

The ligase chain reaction, which is known in the art, can also be used to amplify target nucleic acid sequences. See, e.g., Wu et al., Genomics 4:560-569 (1989). In addition, a technique known as allele-specific PCR can also be used to detect variations (e.g., substitutions). See, e.g., Ruano and Kidd (1989) Nucleic Acids Research 17:8392; McClay et al. (2002) Analytical Biochem. 301:200-206. In certain embodiments of this technique, an allele-specific primer is used wherein the 3' terminal nucleotide of the primer is complementary to (i.e., capable of specifically base-pairing with) a particular variation in the target nucleic acid. If the particular variation is not present, an amplification product is not observed. Amplification Refractory Mutation System (ARMS) can also be used to detect variations (e.g., substitutions). ARMS is described, e.g., in European Patent Application Publication No. 0332435, and in Newton et al., Nucleic Acids Research, 17:7, 1989.

Other methods useful for detecting variations (e.g., substitutions) include, but are not limited to, (1) allele-specific nucleotide incorporation assays, such as single base extension assays (see, e.g., Chen et al. (2000) Genome Res. 10:549-557; Fan et al. (2000) Genome Res. 10:853-860; Pastinen et al. (1997) Genome Res. 7:606-614; and Ye et al. (2001) Hum. Mut. 17:305-316); (2) allele-specific primer extension assays (see, e.g., Ye et al. (2001) Hum. Mut. 17:305-316; and Shen et al. Genetic Engineering News, vol. 23, Mar. 15, 2003), including allele-specific PCR; (3) 5'nuclease assays (see, e.g., De La Vega et al. (2002) BioTechniques 32:S48-S54 (describing the TaqMan® assay); Ranade et al. (2001) Genome Res. 11:1262-1268; and Shi (2001) Clin. Chem. 47:164-172); (4) assays employing molecular beacons (see, e.g., Tyagi et al. (1998) Nature Biotech. 16:49-53; and Mhlanga et al. (2001) Methods 25:463-71); and (5) oligonucleotide ligation assays (see, e.g., Grossman et al. (1994) Nuc. Acids Res. 22:4527-4534; patent application Publication No. US 2003/0119004 A1; PCT International Publication No. WO 01/92579 A2; and U.S. Pat. No. 6,027,889).

Variations may also be detected by mismatch detection methods. Mismatches are hybridized nucleic acid duplexes which are not 100% complementary. The lack of total complementarity may be due to deletions, insertions, inversions, or substitutions. One example of a mismatch detection method is the Mismatch Repair Detection (MRD) assay described, e.g., in Faham et al., Proc. Natl Acad. Sci. USA 102:14717-14722 (2005) and Faham et al., Hum. Mol. Genet. 10:1657-1664 (2001). Another example of a mismatch cleavage technique is the RNase protection method, which is described in detail in Winter et al., Proc. Natl. Acad. Sci. USA, 82:7575, 1985, and Myers et al., Science 230:1242, 1985. For example, a method of the invention may involve the use of a labeled riboprobe which is complementary to the human wild-type target nucleic acid. The riboprobe and target nucleic acid derived from the tissue sample are annealed (hybridized) together and subsequently digested with the enzyme RNase A which is able to detect some mismatches in a duplex RNA structure. If a mismatch is detected by RNase A, it cleaves at the site of the mismatch. Thus, when the annealed RNA preparation is separated on an electrophoretic gel matrix, if a mismatch has been detected and cleaved by RNase A, an RNA product will be seen which is smaller than the full-length duplex RNA for the riboprobe and the mRNA or DNA. The riboprobe need not be the full length of the target nucleic acid, but can a portion of the target nucleic acid, provided it encompasses the position suspected of having a variation.

In a similar manner, DNA probes can be used to detect mismatches, for example through enzymatic or chemical cleavage. See, e.g., Cotton et al., Proc. Natl. Acad. Sci. USA, 85:4397, 1988; and Shenk et al., Proc. Natl. Acad. Sci. USA, 72:989, 1975. Alternatively, mismatches can be detected by shifts in the electrophoretic mobility of mismatched duplexes relative to matched duplexes. See, e.g., Cariello, Human Genetics, 42:726, 1988. With either riboprobes or DNA probes, the target nucleic acid suspected of comprising a variation may be amplified before hybridization. Changes in target nucleic acid can also be detected using Southern hybridization, especially if the changes are gross rearrangements, such as deletions and insertions.

Restriction fragment length polymorphism (RFLP) probes for the target nucleic acid or surrounding marker genes can be used to detect variations, e.g., insertions or deletions. Insertions and deletions can also be detected by cloning, sequencing and amplification of a target nucleic acid. Single stranded conformation polymorphism (SSCP) analysis can also be used to detect base change variants of an allele. See, e.g. Orita et al., Proc. Natl. Acad. Sci. USA 86:2766-2770, 1989, and Genomics, 5:874-879, 1989.

### Compositions of the Invention

The invention provides for compositions of isolated polynucleotides that comprise a polynucleotide or fragment thereof comprising a SNP. In one embodiment, the polynucleotide is a PRO-associated polynucleotide.

In particular, the invention provides for compositions that comprise unique sets and/or combinations of SNPs that can be used as a genetic profile or signature indicative of a subject at risk of developing lupus, or indicative of the disease or symptom or condition thereof. The polymorphisms disclosed herein are useful as biomarkers for assessing risk of developing lupus, as well as for targets for the design of diagnostic reagents. In some embodiments, the SNP is not associated with a gene. In other embodiments, the SNP is associated with a gene, and can be located either in an intergenic or intragenic region, and more particularly, can be located in a coding or noncoding region. The genes associated with a SNP of the present invention may be associated with an unknown gene, or may be associated with a known gene e.g., ITGAM or BLK.

The SNPs identified herein provide targets for development of therapeutic agents for use in the diagnosis and treatment of genetically identified lupus patients, including diagnosis and targeted treatment of lupus patient subpopulations exhibiting a distinct genetic signature comprising one or more of the SNPs of the present invention. For example, in one embodiment, the genes containing the genetic variations identified herein, and the nucleic acid (e.g., DNA or RNA) associated with these genes, and proteins encoded by these genes, can be used as targets for the development of therapeutic agents (e.g., small molecule compounds, antibodies, antisense/RNAi agents, etc.) or used directly as therapeutic agents (e.g., therapeutic proteins, etc.) for the treatment of lupus.

Accordingly, in one aspect, the disclosure provides a set of one or more SNPs that form a unique genetic signature for assessing the risk of developing lupus. In one aspect, the unique genetic signature comprises about 1-10, 10-20, 20-30, 30-40, or 40-50 SNPs selected from any of the SNPs set forth in Figures 1-17 and Tables 1-10.

In one aspect, the unique genetic signature comprises 1 or more SNPs, 3 or more SNPs, 3 or more SNPs, 4 or more SNPs, 5 or more SNPs, 6 or more SNPs, 7 or more SNPs, 8 or more SNPs, 9 or more SNPs, 10 or more SNPs, 11 or more SNPs, 12 or more SNPs, 13 or more SNPs, 14 or more SNPs, 15 or more SNPs, 16 or more SNPs, 17 or more SNPs, 18 or more SNPs, 19 or more SNPs, or 20 or more SNPs selected from any of the SNPs set forth in Figures 1-17 and Tables 1-10. In one aspect, the SNPs of the genetic signature are selected from Table 6. In another aspect, the SNPs are selected from the group consisting of rs9888739, rs13277113, rs7574865, rs2269368, rs6889239, rs2391592 and rs21177770. In another aspect, the SNPs are selected from the group consisting of rs2187668, rs10488631, rs7574865, rs9888739, rs13277113, rs2431697, rs6568431, rs10489265, rs2476601, rs2269368, rs1801274, rs4963128, rs5754217, rs6445975, rs3129860, rs10516487, rs6889239, rs2391592, and rs2177770.

In another embodiment, the disclosure provides for an isolated polynucleotide (e.g., DNA or RNA) or fragment thereof that is at least about 10 nucleotides in length, wherein the polynucleotide or fragment thereof comprises: a) a genetic variation at a nucleotide position corresponding to the position of a single nucleotide polymorphism (SNP) selected from any of those SNPs set forth in Figures 1-17 and Tables 1-10, or (b) the complement of (a). In one embodiment, the isolated polynucleotide is a genomic DNA comprising a single nucleotide polymorphism (SNP) selected from any of those SNPs set forth in any of Figures 1-17 and Tables 1-10. In another embodiment, the isolated polynucleotide is an RNA comprising an of a single nucleotide polymorphism (SNP) selected from any of those set forth in Figures 1-17 and Tables 1-10.

In one embodiment of the disclosure genetic variation in the region upstream of the transcription initiation site of B Lymphoid tyrosine Kinase (BLK) and C8orf13 (chromosome 8p23.1) is associated with disease risk in both the U.S. and Swedish case/control series (rs13277113, OR = 1.39, meta P = 1 x 10⁻¹⁰), and also with altered mRNA levels in B cell lines. In another embodiment, variants in the Integrin Alpha M (ITGAM) and Integrin Alpha X (ITGAX) region (chromosome 16p11.2) are associated with SLE in the combined sample (rs11574637, OR=1.33, meta P = 3 x 10⁻¹¹). In a comprehensive genome-wide association scan in SLE, the present inventors have identified and then confirmed through replication two new genetic loci: a) a promoter region allele that correlates with reduced expression of BLK and increased expression of C8orf13 and b) SNPs (or variants) within the ITGAM/ITGAX region that are in strong linkage disequilibrium with two common nonsynonymous alleles of ITGAM.

In one embodiment, the polynucleotide or fragment thereof is at least about 10 nucleotides in length, alternatively at least about 15 nucleotides in length, alternatively at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length, alternatively at least about 1000 nucleotides in length, and alternatively about the length of the full-length coding sequence. In any of these embodiments, the fragment or full-length polynucleotide may also include part or all of a naturally-occurring flanking region of a SNP. In this context the term "about" means the referenced nucleotide sequence length plus or minus 10% of that referenced length.

In another embodiment, the sequence of the polynucleotide comprises a genetic variation within a linkage disequilibrium region e.g., as set forth in any of Figures 1-17 and Tables 1-10. In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene. In another embodiment, the complement of any of the above polynucleotides is provided. In another embodiment, a PRO encoded by the any of the above polynucleotides is provided.

In one embodiment, an isolated polynucleotide provided herein is detectably labeled, e.g., with a radioisotope, a fluorescent agent, or a chromogenic agent. In another embodiment, an isolated polynucleotide is a primer. In another embodiment, an isolated polynucleotide is an oligonucleotide, e.g., an allele-specific oligonucleotide. In another embodiment, an oligonucleotide may be, for example, from 7-60 nucleotides in length, 9-45 nucleotides in length, 15-30 nucleotides in length, or 18-25 nucleotides in length. In another embodiment, an oligonucleotide may be, e.g., PNA, morpholino-phosphoramidates, LNA, or 2'-alkoxyalkoxy. Oligonucleotides as provided herein are useful, e.g., as hybridization probes for the detection of genetic variations.

In one embodiment, the disclosure provides a composition comprising a plurality of polynucleotides capable of specifically hybridizing to at least 1, 2, 3, 4, or 5 PRO-associated polynucleotides, each PRO-associated polynucleotide comprising a genetic variation at a nucleotide position corresponding to the position of a SNP set forth in any of Figures 1-17 and Tables 1-10, or complements of such PRO-associated polynucleotides. In one embodiment, the polynucleotides are provided as an array, gene chip, or gene set (e.g., a set of genes or fragments thereof, provided separately or as a mixture). In another embodiment, an allele-specific oligonucleotide is provided that hybridizes to a region of a PRO-associated polynucleotide comprising a genetic variation (e.g., a substitution). In one embodiment, the genetic variation is at a nucleotide position corresponding to the position of a SNP set forth in any of Figures 1-17 and Tables 1-10. In one such embodiment, the genetic variation comprises a SNP set forth in any of Figures 1-17 and Tables 1-10. The allele-specific oligonucleotide, when hybridized to the region of the PRO-associated polynucleotide, comprises a nucleotide that base pairs with the genetic variation. In another embodiment, the complement of an allele-specific oligonucleotide is provided. In another embodiment, a microarray comprising an allele-specific oligonucleotide or its complement is provided. In another embodiment, an allele-specific oligonucleotide or its complement is an allele-specific primer. In one embodiment, the allele-specific oligonucleotide comprises a genetic variation in a PRO-associated polynucleotide sequence, wherein the PRO-associated polynucleotide encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene. In another embodiment, the complement of any of the above polynucleotides is provided.

An allele-specific oligonucleotide can be used in conjunction with a control oligonucleotide that is identical to the allele-specific oligonucleotide, except that the nucleotide that specifically base pairs with the genetic variation is replaced with a nucleotide that specifically base pairs with the corresponding nucleotide present in the wild type PRO-associated polynucleotide. Such oligonucleotides may be used in competitive binding assays under hybridization conditions that allow the oligonucleotides to distinguish between a PRO-associated polynucleotide comprising a genetic variation and a PRO-associated polynucleotide comprising the corresponding wild type nucleotide.

Using routine methods based on, e.g., the length and base composition of the oligonucleotides, one skilled in the art can arrive at suitable hybridization conditions under which (a) an allele-specific oligonucleotide will preferentially bind to a PRO-associated polynucleotide comprising a genetic variation relative to a wild type PRO-associated polynucleotide, and (b) the control oligonucleotide will preferentially bind to a wild type PRO-associated polynucleotide relative to a PRO-associated polynucleotide comprising a genetic variation. Exemplary conditions include conditions of high stringency, e.g., hybridization conditions of 5x standard saline phosphate EDTA (SSPE) and 0.5% NaDodSO₄ (SDS) at 55°C, followed by washing with 2X SSPE and 0.1% SDS at 55°C or room temperature. In another embodiment, a binding agent is provided that preferentially binds to a PRO comprising an amino acid variation, relative to a wild-type PRO. In one embodiment, the amino acid variation is any resulting from a genetic variation in a nucleotide position corresponding to a SNP set forth in any of Figures 1-17 and Tables 1-10 (including, e.g., any specific SNP in any of these Figures or Tables). In another embodiment, the binding agent is an antibody.

### Methods of Use

The invention also provides a variety of compositions suitable for use in performing methods of the invention. In one embodiment, the disclosure comprises at least one nucleic acid molecule useful for detecting one or more genetic variations as disclosed in Figures 1-17 and Tables 1-10. Such a nucleic acid molecule can be used in the methods of the present invention, e.g., for the detection of, assay for, and treatment of lupus. In some embodiments, the nucleic acid molecule is attached to a solid substrate as described herein.

In another embodiment, the disclosure provides arrays that can be used in the methods of the present invention. In one embodiment, an array of the disclosure comprises individual or collections of nucleic acid molecules useful for detecting one or more genetic variations. For instance, an array of the invention may comprise a series of discretely placed individual allele-specific oligonucleotides or sets of allele-specific oligonucleotides. Several techniques are well-known in the art for attaching nucleic acids to a solid substrate such as a glass slide. One method is to incorporate modified bases or analogs that contain a reactive moiety that is capable of attachment to a solid substrate, such as an amine group, a derivative of an amine group, or another group with a positive charge, into nucleic acid molecules that are synthesized. The synthesized product is then contacted with a solid substrate, such as a glass slide coated with an aldehyde or other reactive group. The aldehyde or other reactive group will form a covalent link with the reactive moiety on the amplified product, which will become covalently attached to the glass slide. Other methods, such as those using amino propryl silican surface chemistry are also known in the art.

A biological sample, according to any of the above methods, may be obtained using certain methods known to those skilled in the art. Biological samples may be obtained from vertebrate animals, and in particular, mammals. Tissue biopsy is often used to obtain a representative piece of tumor tissue. Alternatively, tumor cells can be obtained indirectly in the form of tissues or fluids that are known or thought to contain the tumor cells of interest. For instance, samples of lung cancer lesions may be obtained by resection, bronchoscopy, fine needle aspiration, bronchial brushings, or from sputum, pleural fluid or blood. Variations in target nucleic acids (or encoded polypeptides) may be detected from a tumor sample or from other body samples such as urine, sputum or serum. (Cancer cells are sloughed off from tumors and appear in such body samples.) By screening such body samples, a simple early diagnosis can be achieved for diseases such as cancer. In addition, the progress of therapy can be monitored more easily by testing such body samples for variations in target nucleic acids (or encoded polypeptides). Additionally, methods for enriching a tissue preparation for tumor cells are known in the art. For example, the tissue may be isolated from paraffin or cryostat sections. Cancer cells may also be separated from normal cells by flow cytometry or laser capture microdissection.

Subsequent to the determination that a subject, or the tissue or cell sample comprises a genetic variation disclosed herein, it is contemplated that an effective amount of an appropriate lupus therapeutic agent may be administered to the subject to treat the lupus condition in the subject. Diagnosis in mammals of the various pathological conditions described herein can be made by the skilled practitioner. Diagnostic techniques are available in the art which allow, e.g., for the diagnosis or detection of lupus in a mammal.

A lupus therapeutic agent can be administered in accordance with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Optionally, administration may be performed through mini-pump infusion using various commercially available devices.

Effective dosages and schedules for administering lupus therapeutic agents may be determined empirically, and making such determinations is within the skill in the art. Single or multiple dosages may be employed. For example, an effective dosage or amount of interferon inhibitor used alone may range from about 1 mg/kg to about 100 *mg*/kg of body weight or more per day. Interspecies scaling of dosages can be performed in a manner known in the art, e.g., as disclosed in Mordenti et al., Pharmaceut. Res., 8:1351 (1991).

When *in vivo* administration of a lupus therapeutic agent is employed, normal dosage amounts may vary from about 10 ng/kg to up to 100 mg/kg of mammal body weight or more per day, preferably about 1 µg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

It is contemplated that yet additional therapies may be employed in the methods. The one or more other therapies may include but are not limited to, administration of steroids and other standard of care regimens for the disorder in question. It is contemplated that such other therapies may be employed as an agent separate from, e.g., a targeted lupus therapeutic agent.

The invention also provides for methods of detecting the presence of lupus is provided by detecting a variation in a PRO or PRO-associated polynucleotide derived from a biological sample. In one embodiment, the biological sample is obtained from a mammal suspected of having lupus.

The disclosure also provides for methods of determining the genotype of a biological sample is provided by detecting whether a genetic variation is present in a PRO-associated polynucleotide derived from the biological sample. In one embodiment, the genetic variation is at a nucleotide position corresponding to the position of a SNP set forth in any of Figures 1-17 and Tables 1-10. In one such embodiment, the genetic variation comprises a SNP set forth in any of Figures 1-17 and Tables 1-10. In another embodiment, the PRO-associated polynucleotide encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene. In another embodiment, the biological sample is known to comprise, or suspected of comprising, a PRO or PRO-associated polynucleotide comprising the variation. In another embodiment, the biological sample is a cell line, e.g., a primary or immortalized cell line. In one such embodiment, the genotyping provides a basis for classifying or sub-classifying disease.

The invention also provides for methods identifying cells in a biological sample from a mammal that are known to comprise, or suspected of comprising, a PRO or PRO-associated polynucleotide comprising a variation, by detecting the variation in a PRO or PRO-associated polynucleotide derived from the cells of the biological sample. In one embodiment, the variation is a genetic variation. In one embodiment, the genetic variation is at a nucleotide position corresponding to the position of a SNP set forth in any of Figures 1-17 and Tables 1-10. In one such embodiment, the genetic variation comprises a SNP set forth in any of Figures 1-17 and Tables 1-10. In another embodiment, the PRO-associated polynucleotide encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene.

The disclosure also provides for methods diagnosing lupus in a mammal by detecting the presence of a variation in a PRO or PRO-associated polynucleotide derived from a biological sample obtained from the mammal, wherein the biological sample is known to comprise, or suspected of comprising, a PRO or PRO-associated polynucleotide comprising the variation. The disclosure also provides for methods for aiding in the diagnosing lupus in a mammal by detecting the presence of a variation in a PRO or PRO-associated polynucleotide derived from a biological sample obtained from the mammal, wherein the biological sample is known to comprise, or suspected of comprising, a PRO or PRO-associated polynucleotide comprising the variation. In one embodiment, the variation is a genetic variation. In one embodiment, the genetic variation is at a nucleotide position corresponding to the position of a SNP set forth in any of Figures 1-17 and Tables 1-10. In one such embodiment, the genetic variation comprises a SNP set forth in any of Figures 1-17 and Tables 1-10. In another embodiment, the PRO-associated polynucleotide encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene.

Various algorithms known in the art and described herein can be used for assessing risk of developing lupus and response to therapy. Variants associated with a phenotype can interact in an additive, allelic dose dependent manner. In some embodiments of the invention, an algorithm based on a stratification scheme can be used to assess risk of developing lupus, disease severity, and response to-therapy. Lupus cases can be stratified into groups based on the number of risk alleles carried. In one embodiment, the risk allele is defined as the allele enriched in lupus cases relative to controls from the loci. For example, in one embodiment, where a total of 19 alleles from 18 loci are listed, then the maximum possible number of risk alleles is equal to 38. As described herein, the lupus cases stratified by the number of risk alleles and tertiles of the resulting distribution can be determined. The tertiles of lupus cases can then be examined for differences in disease severity, risk and response to therapy. In another embodiment, a method is provided for predicting whether a subject with lupus will respond to a therapeutic agent that targets a PRO or PRO-associated polynucleotide by determining whether the subject comprises a variation in a PRO or PRO-associated polynucleotide, wherein the presence of a variation in a PRO or PRO-associated polynucleotide indicates that the subject will respond to the therapeutic agent. In one embodiment, the variation is a genetic variation. In one embodiment, the genetic variation is at a nucleotide position corresponding to the position of a SNP set forth in any of Figures 1-17 and Tables 1-10. In one such embodiment, the genetic variation comprises a SNP set forth in any of Figures 1-17 and Tables 1-10. In another embodiment, the PRO-associated polynucleotide encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene.

The disclosure also encompasses methods of detecting the absence or presence in a subject, or sample obtained therefrom, of a genetic variation at a nucleotide position corresponding to the position of a SNP as set forth in any of Figures 1-17 and Tables 1-10 by (a) contacting nucleic acid in the subject or sample with any of the polynucleotides described above under conditions suitable for formation of a hybridization complex between the nucleic acid and the polynucleotide; and (b) detecting whether the polynucleotide specifically base pairs with the nucleic acid at the nucleotide position. In one embodiment, the genetic variation is at a nucleotide position corresponding to the position of a SNP set forth in any of Figures 1-17 and Tables 1-10. In one such embodiment, the genetic variation comprises a SNP set forth in any of Figures 1-17 and Tables 1-10. In one embodiment, the PRO-associated polynucleotide encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene.

The disclosure also provides for methods of detecting the absence or presence of a genetic variation in a nucleic acid associated with a PRO by (a) contacting the nucleic acid with an allele-specific oligonucleotide that is specific for the genetic variation under conditions suitable for hybridization of the allele-specific oligonucleotide to the nucleic acid; and (b) detecting the absence or presence of allele-specific hybridization. In one embodiment, the genetic variation is at a nucleotide position corresponding to the position of a SNP set forth in any of Figures 1-17 and Tables 1-10. In one such embodiment, the genetic variation comprises a SNP set forth in any of Figures 1-17 and Tables 1-10. In one embodiment, the PRO-associated polynucleotide encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene. In another embodiment, an allele-specific oligonucleotide is an allele-specific primer.

The disclosure also provides for methods for assessing predisposition of a subject to develop lupus by detecting presence or absence in the subject of a variation in a PRO or PRO-associated polynucleotide, wherein the presence of a variation in a PRO or PRO-associated polynucleotide indicates that the subject is predisposed to develop lupus. In one embodiment, the variation is a genetic variation. In one embodiment, the genetic variation is at a nucleotide position corresponding to the position of a SNP set forth in any of Figures 1-17 and Tables 1-10. In one such embodiment, the genetic variation comprises a SNP set forth in any of Figures 1-17 and Tables 1-10. In another embodiment, the PRO-associated polynucleotide encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene.

The disclosure also provides for methods of sub-classifying lupus in a mammal, the method comprising detecting the presence of a variation in a PRO-associated polynucleotide at a nucleotide position corresponding to the position of a single nucleotide polymorphism (SNP) as set forth in any of Figures 1-17 and Tables 1-10 in a biological sample derived from the mammal, wherein the biological sample is known to comprise, or suspected of comprising, a PRO or PRO-associated polynucleotide comprising the variation. In one embodiment, the variation is a genetic variation. In one embodiment, the variation comprises a SNP as set forth in any of Figures 1-17 and Tables 1-10. In one embodiment, the PRO-associated polynucleotide encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene. In one embodiment, the subclassification is characterized by tissue/organ involvement (e.g., lupus nephritis), gender, and/or ethnicity.

In one embodiment of the detection methods of the invention, the detecting comprises carrying out a process selected from a primer extension assay; an allele-specific primer extension assay; an allele-specific nucleotide incorporation assay; an allele-specific oligonucleotide hybridization assay; a 5' nuclease assay; an assay employing molecular beacons; and an oligonucleotide ligation assay.

The disclosure also provides methods of identifying a therapeutic agent effective to treat lupus in a patient subpopulation, the method comprising correlating efficacy of the agent with the presence of a genetic variation at a nucleotide position corresponding to a single nucleotide polymorphism (SNP) in the patient subpopulation, wherein the SNP is one of those listed in Figures 1-17 and Tables 1-10, thereby identifying the agent as effective to treat lupus in said patient subpopulation. In one embodiment, the genetic variation is at a nucleotide position corresponding to the position of a SNP set forth in any of Figures 1-17 and Tables 1-10. In one such embodiment, the genetic variation comprises a SNP set forth in any of Figures 1-17 and Tables 1-10. In one embodiment, the PRO-associated polynucleotide encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene.

Methods of the invention provide information useful for determining appropriate clinical intervention steps, if and as appropriate. Therefore, in one embodiment of a method of the disclosure the method further comprises a clinical intervention step based on results of the assessment of the presence or absence of a variation in a PRO or PRO-associated polynucleotide as disclosed herein. For example, appropriate intervention may involve prophylactic and treatment steps, or adjustment(s) of any then-current prophylactic or treatment steps based on genetic information obtained by a method of the invention.

As would be evident to one skilled in the art, in any method of the invention, while detection of presence of a variation would positively indicate a characteristic of a disease (e.g., presence or subtype of a disease), non-detection of a variation would also be informative by providing the reciprocal characterization of the disease.

The disclosure also provides for methods of amplifying a nucleic acid comprising a PRO-associated polynucleotide or fragment thereof is provided, wherein the PRO-associated polynucleotide or fragment thereof comprises a genetic variation. In one embodiment, the method comprises (a) contacting the nucleic acid with a primer that hybridizes to a sequence 5' or 3' of the genetic variation, and (b) extending the primer to generate an amplification product comprising the genetic variation. In one embodiment, the method further comprises contacting the amplification product with a second primer that hybridizes to a sequence 5' or 3' of the genetic variation, and extending the second primer to generate a second amplification product. In one such embodiment, the method further comprises amplifying the amplification product and second amplification product, e.g., by polymerase chain reaction.

In some embodiments, the genetic variation is at a nucleotide position corresponding to the position of a SNP of the present invention. In one such embodiment, the genetic variation comprises a SNP set forth in any of Figures 1-17 and Tables 1-10. In one embodiment, the PRO-associated polynucleotide encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene.

Still further methods of the invention include methods of treating lupus in a mammal, comprising steps of obtaining tissue or a cell sample from the mammal, examining the tissue or cells for presence or absence of a variation as disclosed herein, and upon determining presence or absence of the variation in said tissue or cell sample, administering an effective amount of an appropriate therapeutic agent to said mammal. Optionally, the methods comprise administering an effective amount of a targeted lupus therapeutic agent, and, optionally, a second therapeutic agent (e.g., steroids, etc.) to said mammal.

In one embodiment, a method of treating lupus is provided, the method comprising administering to the subject an effective amount of an antagonist or agonist of PRO. In one embodiment, the subject exhibits variation in a PRO or PRO-associated polynucleotide. In one embodiment, the variation is a genetic variation. In one embodiment, the genetic variation is at a nucleotide position corresponding to the position of a SNP set forth in any of Figures 1-17 and Tables 1-10. In one such embodiment, the genetic variation comprises a SNP set forth in any of Figures 1-17 and Tables 1-10. In one embodiment, the PRO-associated polynucleotide encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene.

The invention also provides for methods of treating a lupus condition in a subject in whom a genetic variation is known to be present at a nucleotide position corresponding to a single nucleotide polymorphism (SNP) listed in Figures 1-17 and Tables 1-10, the method comprising administering to the subject a therapeutic agent effective to treat the condition. In one embodiment, the variation comprises a SNP as set forth in any of Figures 1-17 and Tables 1-10. In one embodiment, the variation is a SNP in a PRO-associated polynucleotide that encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene.

The disclosure also provides for methods of treating a subject having a lupus condition, the method comprising administering to the subject a therapeutic agent known to be effective to treat the condition in a subject who has a genetic variation at a nucleotide position corresponding to a single nucleotide polymorphism (SNP) listed in Figures 1-17 and Tables 1-10. In one embodiment, the variation comprises a SNP as set forth in any of Figures 1-17 and Tables 1-10. In one embodiment, the variation is a SNP in a PRO-associated polynucleotide that encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene.

The disclosure also provides for methods of treating a subject having a lupus condition, the method comprising administering to the subject a therapeutic agent previously shown to be effective to treat said condition in at least one clinical study wherein the agent was administered to at least five human subjects who each had a genetic variation at a nucleotide position corresponding to a single nucleotide polymorphism (SNP) listed in Figures 1-17 and Tables 1-10. In one embodiment, the variation comprises a SNP as set forth in any of Figures 1-17 and Tables 1-10. In one embodiment, the variation is a SNP in a PRO-associated polynucleotide that encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene. In one embodiment, the at least five subjects had two or more different SNPs in total for the group of at least five subjects. In one embodiment, the at least five subjects had the same SNP for the entire group of at least five subjects.

The disclosure also provides for methods of treating a lupus subject who is of a specific lupus patient subpopulation comprising administering to the subject an effective amount of a therapeutic agent that is approved as a therapeutic agent for said subpopulation, wherein the subpopulation is characterized at least in part by association with genetic variation at a nucleotide position corresponding to a SNP listed in Figures 1-17 and Tables 1-10. In one embodiment, the variation comprises a SNP as set forth in any of Figures 1-17 and Tables 1-10. In one embodiment, the variation is a SNP in a PRO-associated polynucleotide that encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene. In one embodiment, the subpopulation is of European ancestry. In one embodiment, the disclosure provides a method comprising manufacturing a lupus therapeutic agent, and packaging the agent with instruction to administer the agent to a subject who has or is believed to have lupus and who has a genetic variation at a position corresponding to a single nucleotide polymorphism (SNP) listed in Figures 1-17 and Tables 1-10. In one embodiment, the variation comprises a SNP as set forth in any of Figures 1-17 and Tables 1-10. In one embodiment, the variation is a SNP in a PRO-associated polynucleotide that encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene.

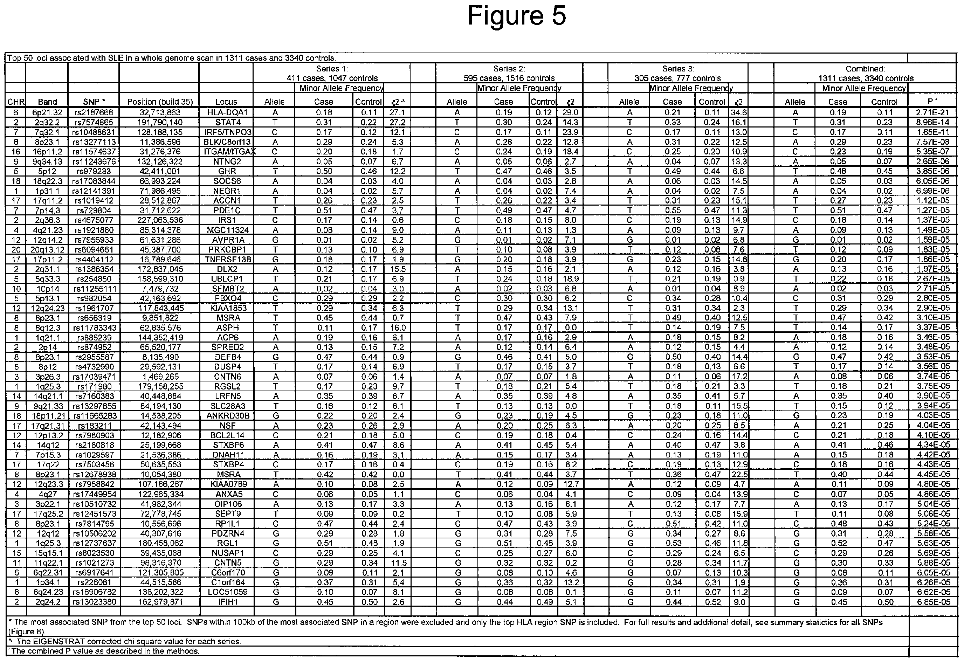

The disclosure also provides for methods of specifying a therapeutic agent for use in a lupus patient subpopulation, the method comprising providing instruction to administer the therapeutic agent to a patient subpopulation characterized by a genetic variation at a position corresponding to a single nucleotide polymorphism (SNP) listed in Figure 5-10. In one embodiment, the variation comprises a SNP as set forth in any of Figures 1-17 and Tables 1-10. In one embodiment, the variation is a SNP in a PRO-associated polynucleotide that encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene. In one embodiment, the subpopulation is of European ancestry.

The disclosure also provides for methods for marketing a therapeutic agent for use in a lupus patient subpopulation, the method comprising informing a target audience about the use of the therapeutic agent for treating the patient subpopulation as characterized by the presence, in patients of such subpopulation, of a genetic variation at a position corresponding to a single nucleotide polymorphism (SNP) listed in Figure 5-10. In one embodiment, the variation comprises a SNP as set forth in any of Figures 1-17 and Tables 1-10. In one embodiment, the variation is a SNP in a PRO-associated polynucleotide that encodes a PRO that is encoded by a sequence within a linkage disequilibrium region (e.g., as set forth in Figures 1-17 and Tables 1-10). In one embodiment, the genetic variation is in genomic DNA that encodes a gene (or its regulatory region), wherein the gene (or its regulatory region) comprises a SNP set forth in any of Figures 1-17 and Tables 1-10. In one embodiment, the SNP is in a non-coding region of the gene. In one embodiment, the SNP is in a coding region of the gene. In an embodiment of any of the above methods that comprise the use of a therapeutic agent, such agent comprises a lupus therapeutic agent as disclosed herein.

The disclosure also provides for methods for modulating signaling through the B cell receptor in a subject in whom a genetic variation is known to be present at a nucleotide position corresponding to a single nucleotide polymorphism (SNP) listed in Figures 1-17 and Tables 1-10, the method comprising administering to the subject a therapeutic agent effective to modulate signaling through the B cell receptor.

The disclosure also provides for methods for modulating the differentiation of Th17 cells in a subject in whom a genetic variation is known to be present at a nucleotide position corresponding to a single nucleotide polymorphism (SNP) listed in Figures 1-17 and Tables 1-10, the method comprising administering to the subject a therapeutic agent effective to modulate the differentiation of Th17 cells.

### Kits

In one embodiment of the disclosure kits are provided. In one embodiment, a kit comprises any of the polynucleotides described herein, optionally with an enzyme. In one embodiment, the enzyme is at least one enzyme selected from a nuclease, a ligase, and a polymerase.

In one embodiment, the disclosure provides a kit comprising a composition of the invention, and instructions for using the composition to detect lupus by determining whether a subject's genome comprises a genetic variation as disclosed herein. In one embodiment, the composition of the disclosure comprises a plurality of polynucleotides capable of specifically hybridizing to at least 1, 2, 3, 4, or 5 PRO-associated polynucleotides, each PRO-associated polynucleotide comprising a genetic variation at a nucleotide position corresponding to the position of a SNP set forth in any of Figures 1-17 and Tables 1-10, or complements of such PRO-associated polynucleotides. In one embodiment, the composition of the disclosure comprises polynucleotides encoding at least a portion of a PRO. In one embodiment, the composition of the invention comprises nucleic acid primers capable of binding to and effecting polymerization (e.g., amplification) of at least a portion of a PRO-associated polynucleotide. In one embodiment, the composition of the disclosure comprises a binding agent (e.g., primer, probe) that specifically detects PRO-associated polynucleotide (or complement thereof) (or corresponding gene product). In one embodiment, the composition of the disclosure comprises a binding agent that specifically binds to at least a portion of a PRO. In one embodiment, the disclosure provides an article of manufacture comprising a therapeutic agent, combined with instructions to use the agent to treat a lupus patient who has a variation in a PRO-associated polynucleotide as disclosed herein.

For use in the applications described or suggested above, kits or articles of manufacture are also provided by the invention. Such kits may comprise a carrier means being compartmentalized to receive in close confinement one or more container means such as vials, tubes, and the like, each of the container means comprising one of the separate elements to be used in the method. For example, one of the container means may comprise a probe that is or can be detectably labeled. Such probe may be a polynucleotide specific for a PRO-associated polynucleotide. Where the kit utilizes nucleic acid hybridization to detect the target nucleic acid, the kit may also have containers containing nucleotide(s) for amplification of the target nucleic acid sequence and/or a container comprising a reporter means, such as a biotin-binding protein, such as avidin or streptavidin, bound to a reporter molecule, such as an enzymatic, florescent, or radioisotope label.

The kit of the disclosure will typically comprise the container described above and one or more other containers comprising materials desirable from a commercial and user standpoint, including buffers, diluents, filters, needles, syringes, and package inserts with instructions for use. A label may be present on the container to indicate that the composition is used for a specific therapy or non-therapeutic application, and may also indicate directions for either *in vivo* or *in vitro* use, such as those described above.

The kits of the disclosure have a number of embodiments. A typical embodiment is a kit comprising a container, a label on said container, and a composition contained within said container; wherein the composition includes detecting agent for a PRO or PRO-associated polynucleotide, the label on said container indicates that the composition can be used to evaluate the presence of the PRO or PRO-associated polynucleotide in at least one type of mammalian cell, and instructions for using the detecting agent for evaluating the presence of the PRO or PRO-associated polynucleotide in at least one type of mammalian cell. The kit can further comprise a set of instructions and materials for preparing a tissue sample and applying antibody and probe to the same section of a tissue sample. For example, a kit may comprise a container, a label on said container, and a composition contained within said container; wherein the composition includes a polynucleotide that hybridizes to a complement of a PRO-associatd polynucleotide under stringent conditions, the label on said container indicates that the composition can be used to evaluate the presence of a PRO-associated polynucleotide in at least one type of mammalian cell, and instructions for using the polynucleotide for evaluating the presence of PRO-associated RNA or DNA in at least one type of mammalian cell.

Other optional components in the kit include one or more buffers (e.g., block buffer, wash buffer, substrate buffer, etc), other reagents such as substrate (e.g., chromogen) which is chemically altered by an enzymatic label, epitope retrieval solution, control samples (positive and/or negative controls), control slide(s) etc.

### Methods of Marketing

The disclosure herein also encompasses a method for marketing a lupus therapeutic agent or a pharmaceutically acceptable composition thereof comprising promoting to, instructing, and/or specifying to a target audience, the use of the agent or pharmaceutical composition thereof for treating a patient or patient population with lupus from which a sample has been obtained showing the presence of a genetic variation as disclosed herein.

Marketing is generally paid communication through a non-personal medium in which the sponsor is identified and the message is controlled. Marketing for purposes herein includes publicity, public relations, product placement, sponsorship, underwriting, and sales promotion. This term also includes sponsored informational public notices appearing in any of the print communications media designed to appeal to a mass audience to persuade, inform, promote, motivate, or otherwise modify behavior toward a favorable pattern of purchasing, supporting, or approving the invention herein.

The marketing of the diagnostic method herein may be accomplished by any means. Examples of marketing media used to deliver these messages include television, radio, movies, magazines, newspapers, the internet, and billboards, including commercials, which are messages appearing in the broadcast media.

The type of marketing used will depend on many factors, for example, on the nature of the target audience to be reached, *e.g.,* hospitals, insurance companies, clinics, doctors, nurses, and patients, as well as cost considerations and the relevant jurisdictional laws and regulations governing marketing of medicaments and diagnostics. The marketing may be individualized or customized based on user characterizations defined by service interaction and/or other data such as user demographics and geographical location.

The following are examples of the methods and compositions of the invention. It is understood that various other embodiments may be practiced, given the general description provided above.

### EXAMPLES

The bibliographic information for the references cited (and denoted by number) in Examples 1-3 are provided at the end of Example 3. The bibliographic information for the references cited (and denoted by number) in Examples 4-6 are provided at the end of Example 6.

### Example 1 Materials and Methods for A Genome-Wide Association Scan in Systemic Lupus Erythematosus

This Example describes materials and methods undertaken to perform a genome-wide scan for SLE in a large sample comprising 1311 SLE cases and 3340 controls. Over 500,000 variants, which captured common variation across an estimated 85% of the human genome, 24 were genotyped and tested for an association to SLE.

### Subjects

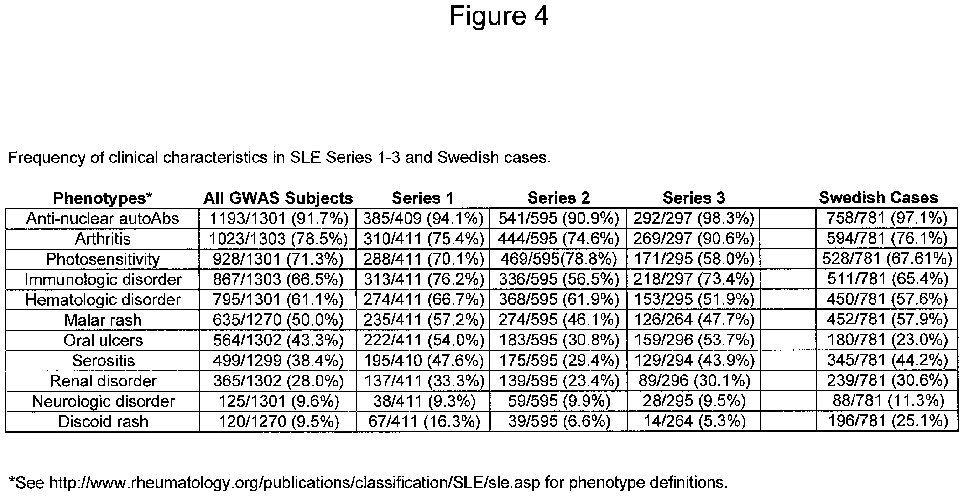

SLE case samples were genotyped from the following collections: a) 338 subjects from the Autoimmune Biomarkers Collaborative Network (ABCoN), an NIH/NIAMS funded repository,²⁵ b) 141 subjects from the Multiple Autoimmune Disease Genetics Consortium (MADGC),²⁶ c) 613 subjects from the University of California San Francisco (UCSF) Lupus Genetics Project^{10,27} and d) 335 subjects from the University of Pittsburgh Medical Center (UPMC)²⁸ plus 8 samples collected at The Feinstein Institute for Medical Research. All SLE cases were self-described Caucasians. The diagnosis of SLE (fulfillment of four or more of the American College of Rheumatology (ACR) defined criteria²⁹) was confirmed in all cases by medical record review (94%) or through written documentation of criteria by treating rheumatologists (6%). Clinical data were reviewed and tabulated at each institution. Figure 4 shows the counts and percentages for each of the eleven ACR classification criteria for SLE.²⁹

A total of 3583 control samples were examined in the association analyses. As part of this project, 1861 control samples were selected and then genotyped from the New York Cancer Project (NYCP) collection³⁰, based on self-described ethnicity, gender and age. In addition, genotype data from 1722 self-described Caucasian control samples were obtained from the publicly available iControlDB database <www.illumina.com/pages.ilmn?ID=231>.

For replication, DNA samples from an independent collection of 793 Swedish SLE patients (all of whom fulfilled four or more of the classification criteria for SLE as defined by the ACR) and 857 healthy Swedish control individuals, were genotyped. The patients were from rheumatology clinics at the Lund, Uppsala, Karolinska (Solna) and Umeå University Hospitals. ⁷ The Institutional Review Boards of all collaborating institutions approved these studies, and all participants gave informed consent.

### Genotyping

Control samples from the NYCP (N=1861) were genotyped on the Illumina HumanHap550 Genotyping BeadChip³¹ at The Feinstein Institute. 1465 samples (464 cases, 1001 controls) were genotyped on the HumanHap550v1 chip and 1875 samples (1015 cases, 860 controls) were genotyped on the HumanHap550v3 chip. Genotype data from 1452 of these control samples were submitted to iControlDB and made publicly available prior to publication. An additional, independent set of 1722 Caucasian samples genotyped using the HumanHap550 BeadChip was obtained from Studies 66 and 67 of the iControlDB <www.illumina.com/pages.ilmn?ID=231>. Case samples were genotyped at The Feinstein Institute in serial phases; Series 1 consisted of the 479 cases from ABCoN and MADGC, Series 2 included the 613 cases from UCSF, and Series 3 was comprised of 387 cases from UPMC and The Feinstein Institute. The 545,080 single nucleotide polymorphisms (SNPs) present on both HumanHap550 versions were advanced into the analysis. Case and control samples with average call rates < 80% across the chip were re-genotyped.

In the Swedish replication collection, the SNPs rs11574637 and rs13277113 were genotyped using homogeneous single base primer extension assays with fluorescence polarization detection at the SNP Technology Platform in Uppsala <www.genotyping.se> and reagents from Perkin-Elmer.³² The genotype call rate in the samples was 96% and the reproducibility was 100% according to duplicate assays of 4.6% of the genotypes. A three generation CEPH pedigree with 20 members was genotyped in parallel with the study samples, and no deviation from Mendelian inheritance was observed for either of the SNPs.

### Data Quality Filters

Samples with an average call rate of ≤ 95% (N=42) or where the reported sex of the individual was discordant with observed sex (N=21) were excluded from the analysis. The identity by state (IBS) across the genome was estimated for each sample, and the samples examined for cryptic relatedness. One sample from each pair estimated to be duplicates or 1st-3rd degree relatives was removed (Pi_hat >0.10 and Z1 ≥ 0.15, N=161). Three of these pairs were comprised of both a case and a control; the control was removed. SNPs with a frequency in cases of <1% (N=21,644) or a HWE P ≤ 1 x 10⁻⁶ in controls (N=2819) were removed from the analysis. SNPs with missingness > 5% (N=6074) were removed. SNPs were tested for the probability of a significant difference in missingness between cases and controls; SNPs with P ≤ 1 x 10⁻⁵ (N=7646) were removed. SNPs were also tested for batch effects: for example, between ABCoN samples and all other cases; SNPs with P < 1 x 10⁻⁹ (N=13) were removed.

Population outliers were detected using EIGENSTRAT³³. Samples more than 6 standard deviations from the mean along any of the top 10 principal components were excluded from the analysis (N=141). Data from the 3340 remaining control samples were randomly assigned to each SLE case series proportionately, resulting in a ∼2.5 control:case ratio (Table 1).

Series 1 consisted of 411 cases and 1047 controls, Series 2 was comprised of 595 cases and 1516 controls, and Series 3 was comprised of 305 cases and 777 controls. Overall, 93% of cases were female, and 62% of controls were female. No significant differences in allele frequencies were noted between males and females.

SNPs with > 2% missing data in at least one series and where the missing data was unequally distributed between cases and controls (differential missingness, P < 1 x 10⁻³) were removed (N= 3323). SNPs in the pseudo-autosomal region of chromosome X (N=13) showed no significant association and were excluded from further analysis. The sample and marker filtering were conducted using analytical modules within the software program PLINK³⁴. For each series, a total of 502,033 SNPs were advanced into downstream analyses.

### Data Analysis

The association of all SNPs to SLE susceptibility was calculated using 2x2 contingency tables. A genomic control inflation factor (λ_{gc}) was then calculated for each sample series.³⁵ The genomic control inflation factor is a metric based on the median chi square that reflects whether the bulk of the distribution conforms to the null hypothesis (λ_{gc}=1.0). A λ_{gc} value > 1 indicates an elevation of the average chi square association statistic due to systemic technical artifacts or the presence of population stratification. After removing low quality data to minimize technical artifacts, evidence of inflation was noted for each series: 1.14, 1.18, and 1.11, respectively, for Series 1, 2 and 3. To correct for the presence of population stratification, principal components for each series were calculated using a subset of SNPs in EIGENSTRAT. SNPs with case MAF < 2% (5011), control HWE P ≤ 1 x 10⁻⁴ (1792), or missing data > 1% (50414) were removed, as were SNPs in regions of abnormal LD patterns due to structural variation on chromosomes 6 (from 24-36 Mb), 8 (8-12 Mb), 11 (42-58 Mb), and 17 (40-43 Mb). The remaining 440,202 SNPs were used to calculate principal components. In each series, the first 4 principal components were used to adjust the association statistic for all 502,033 SNPs. After adjustment for population stratification, the λ_{gc} for each series approached 1.0 (see Table 1). The corrected association statistic for each series was combined by the weighted merging of the Z-score incorporating λ_{gc} (Figure 12). The top 50 loci are shown in Figure 5. Additionally, the statistics for all SNPs passing QC filters from each series and the combined association statistics have been summarized in a table (not shown).

To test for heterogeneity between the three case-control studies for the most associated variants, the Breslow-Day test implemented in PLINK was run for the SNP with the best association in each of these regions: HLA DRB, STAT4, IRF5, BLK, and ITGAM/ITGAX. No significant heterogeneity was detected (each P > 0.2).

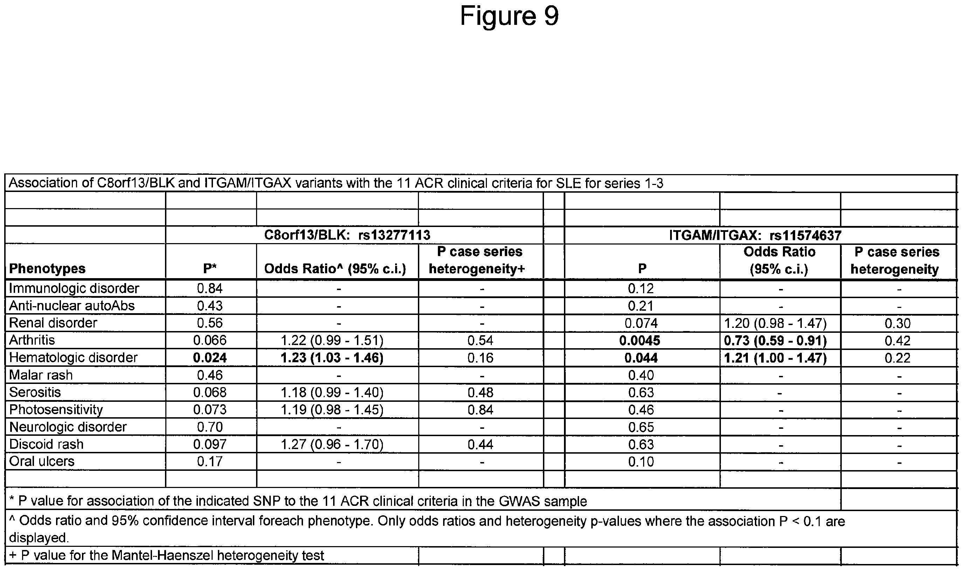

Associations between individual SNPs and subphenotypes were calculated for the combined dataset (Figure 9), using the Mantel-Haenszel heterogeneity test and combined odds ratio implemented in Stata 9.2 (www.stata.com/). The calculated P-values were not adjusted for multiple testing, since the ACR criteria are known to be correlated and a simple Bonferroni correction of α=0.05/11=0.0045 would likely be overly conservative.

### Gene Expression Analysis

Gene expression measurements of Epstein-Barr Virus transformed B cell lines from 210 unrelated, healthy HapMap individuals from a publicly available dataset (GENEVAR project, www.sanger.ac.uk/humgen/genevar/) were examined for a correlation to variants significantly associated with SLE.³⁶ Specifically, the median fluorescence intensity of 4 measurements from probes for *BLK* (GI_33469981-S), *C8orf13* (GI_32698772-S), *ITGAM*(GI_6006013-S), *ITGAX*(GI_34452172-S), *ACTB* (beta-actin, GI_5016088-S), and *GAPDH*(GI_7669491-S) from 60 U.S. residents with northern and western European ancestry (CEU), 60 Yoruba (YRI), 45 Han Chinese individuals from Beijing (CHB) and 45 Japanese individuals from Tokyo (JPT) were examined. The expression data for *BLK, C8orf13, GAPDH* and *ACTB* were stratified by rs13277113 genotype (obtained from the HapMap (www.hapmap.org)), and the significance of the differential expression was measured by a 2-tailed t-test assuming an equal variance. Similarly, the expression data for *ITGAM, ITGAX, GAPDH* and *ACTB* were stratified by genotype at rs11574637 and tested for significance using a t-test. Expression data normalized on a log scale across the HapMap populations as described by the GENEVAR project yielded similar results to the median fluorescence intensity.

The association of BLK and C8orf13 expression to cis-genetic variation in an independent set of 400 EBV-transformed B cells was obtained by the examination and data-mining of a recently published study (www.sph.umich.edu/csg/liang/asthma/).³⁷ Specifically, the association of a proxy for rs13277113 (rs4840568) to the expression levels of BLK (probe 206255_at) and C8orf13 (probe 226614_s_at) was measured as described by Dixon et al.³⁷

### Example 2 Identification of C8orf13/BLK and ITGAM/ITGAX as Novel Susceptibility Loci

### Genomewide Association Analysis

A total of 502,033 polymorphic SNPs on the Illumina chips passed quality control filters and were tested for association to SLE in a staged fashion using 3 case-control series (Table 1). A combined association statistic was calculated by addition of the Z-scores converted from the EIGENSTRAT-corrected chi square test statistic, weighted for series size and adjusted for the residual λ_{gc} of each series (see Methods).

A comparison of the observed meta-analysis P values relative to the P values for a null distribution is shown in Figure 1. Significant deviation from the null distribution was observed at the tail of the distribution (Figure 1A, black diamonds), which may indicate the presence of true positive associations. Strong association to SLE was noted for three established risk loci. In the HLA Class II region, rs2187668 is a near perfect predictor of the DRB1*0301 allele³⁸ and was the variant most strongly associated with SLE in the combined analysis (P = 3 x 10⁻²¹). An additional 157 HLA region SNPs, many of which are correlated to the DRB1*0301 allele, had observed P values less than 5 x 10⁻⁷ (Figure 1B). A strong association was observed with variants linked to the well-validated risk haplotype of Interferon Regulatory Factor 5 (*IRF5*) (e.g. rs10488631, P = 2 x 10⁻¹¹).⁷⁻⁹ In addition, an association with *STAT4* was observed (rs7574865, P = 9 x 10⁻¹⁴). A *STAT4* association with both SLE and rheumatoid arthritis was reported recently.¹⁰ The SLE dataset here overlaps with that of the earlier report¹⁰, and includes an additional 341 cases and 2905 controls that were not included in the previous analysis. In addition, the P values for the top *STAT4* SNPs reported here have been corrected for population stratification.

After removing variants in HLA, *IRF5* and *STAT4* from the chi expected vs. observed analysis, the deviation of P values from the null distribution was not eliminated (Figure 1A, circles), suggesting the presence of additional SLE loci. As shown in Figure 1B, multiple SNPs near the B lymphoid tyrosine Kinase (*BLK*) gene and in a region that contains the Integrin Alpha M (*ITGAM*) and Integrin Alpha X (*ITGAX*) genes were highly associated with SLE in the combined analysis. Neither of these genes or regions has previously been implicated in SLE susceptibility.

### BLK/C8orf13

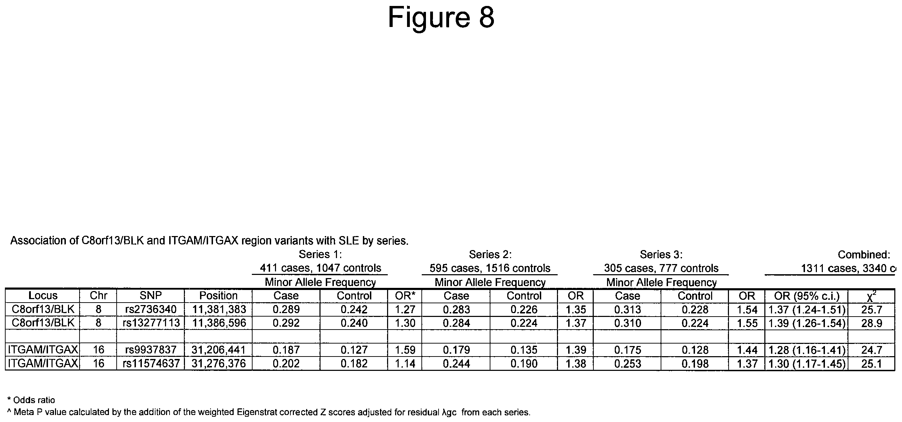

Several variants on the short arm of chromosome 8 (8p23.1) were associated with SLE (Figure 2, Table 2, Figure 8). The "A" allele of rs13277113 was highly enriched in the U.S. SLE cases relative to controls (P = 8 x 10⁻⁸, combined OR = 1.39, 95% C.I. = 1.26-1.54). To confirm this initial observation, an independent collection of 793 SLE cases and 857 matched controls from Sweden was typed for rs13277113, and a convincing association of the minor "A" allele to SLE was also observed (P = 3.6 x 10⁻⁴, OR = 1.33, 95% C.I. = 1.13 - 1.55; Table 2). A meta-analysis of rs13277113 using both the U.S. and Swedish samples showed a P = 1.4 x10⁻¹⁰, which surpasses the rigorous genome-wide significance threshold of association P < 5x10⁻⁸.³⁹

rs13277113 maps to the interval between two genes transcribed in opposite directions: *BLK-* a src family tyrosine kinase that signals downstream of the B cell receptor, and *C8orf13 -* an ubiquitously expressed gene of unknown function (Figure 2). No known coding region variants of *BLK* or *CBorf13* are in linkage disequilibrium (LD) with rs13277113.

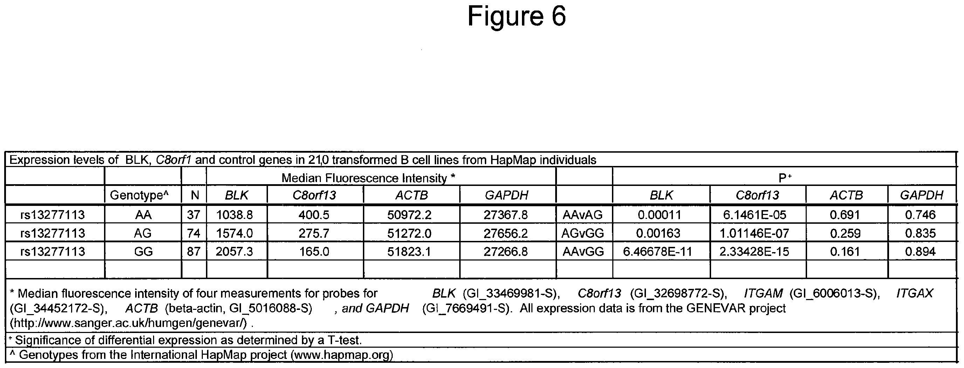

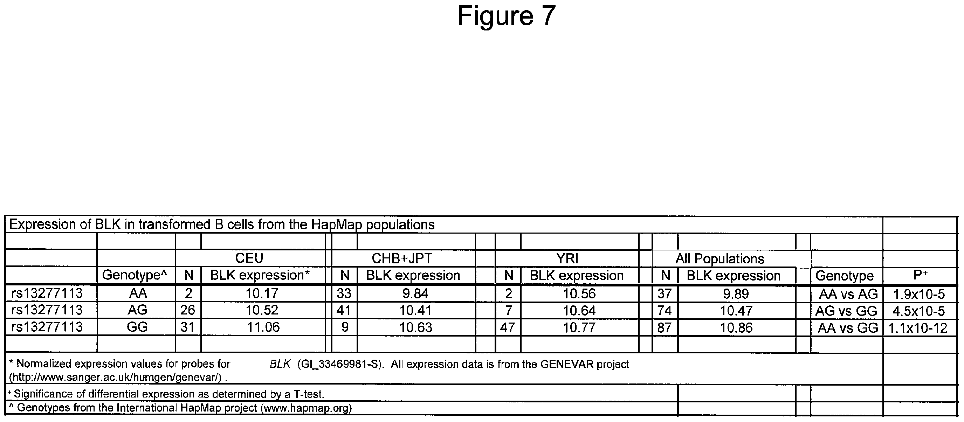

Common genetic variation has been shown to correlate with levels of *cis* gene expression. 8,36,37,40 To determine whether the associated promoter SNPs might influence mRNA expression of *BLK* and/or *C8orf13,* a gene expression dataset generated from Epstein-Barr virus transformed B lymphocyte cell lines from the 210 unrelated HapMap samples was queried.³⁶ Strikingly, the risk "A" allele of rs13277113 was associated with lower levels of mRNA expression of *BLK* (Figure 2B). Homozygotes for the A allele showed ∼50% lower levels of expression than homozygotes for the G allele, and A/G heterozygotes had intermediate levels. Interestingly, the expression of the *C8orf13* gene also correlated with the risk haplotype, but in the opposite direction. The A allele of rs13277113 was associated with higher expression of *C8orf13* in the transformed lines, while the G allele was significantly associated with lower expression (Figure 2C). Again, A/G heterozygotes showed intermediate levels of expression. The expression of a number of control mRNAs (e.g. beta-actin, GAPDH) did not vary in the cell lines based on genotype at rs13277113 (Figure 6), and consistent allelic differences in BLK expression were observed in all HapMap populations (Figure 7). These results were confirmed by analyzing an independent dataset of gene expression and genome-wide SNPs in 400 non-HapMap transformed B cell lines.³⁷ In this dataset, a marker correlated to rs13277113 (rs4840568, r²=0.77) was associated with both decreased expression of *BLK* (P = 8.9 x 10⁻²⁷, probe 206255_at) and increased expression of *CBorf13* (P = 4.6 x 10⁻³⁵, probe 226614_s_at).

Multiple conserved transcription factor binding sites, including motifs for IRF1, PPARG and an interferon-stimulated response element, are located in the 5' region of *BLK* and *CBorf13.* However, neither rs13277113 nor correlated variants (r²>0.5) altered known transcription factor binding sites or other known functional nucleic acid motifs. We conclude that rs13277113, or variation strongly associated with rs13277113, alters the level of mRNA expression of *BLK* and *C8orf13.*

### ITGAM/ITGAX

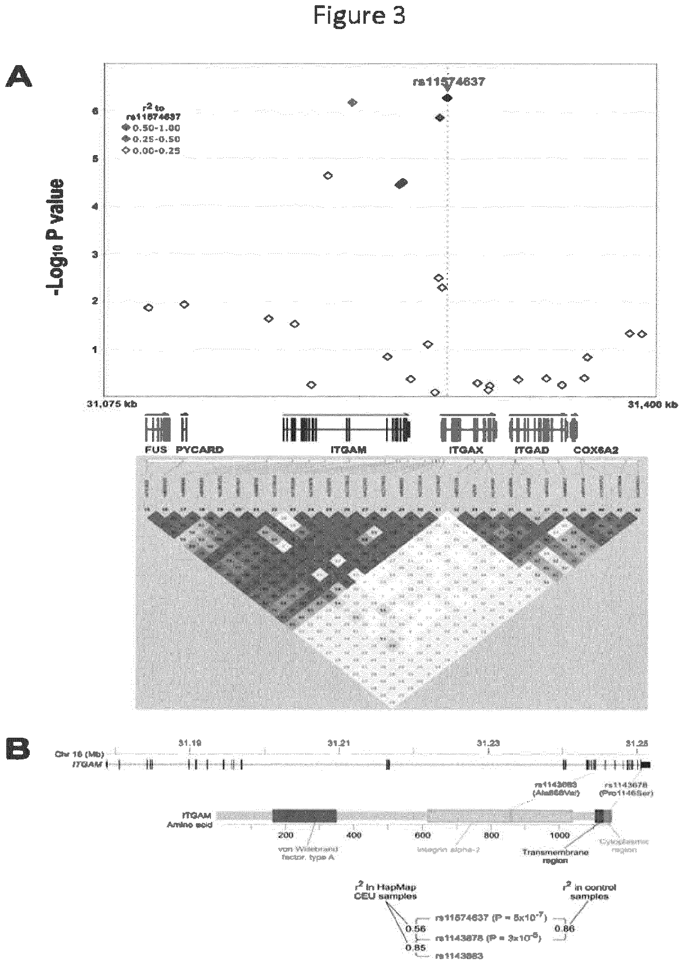

Variants within a cluster of integrin alpha chain genes on chromosome 16 were also significantly associated with SLE (Figure 3, Table 2). Reproducible association of the "C" allele of rs11574637 was observed *across* the 3 SLE series (P = 5 x 10⁻⁷, OR = 1.30, 95% C.I. = 1.17-1.45). Importantly, the "C" allele of rs11574637 showed similar strong enrichment in the Swedish replication series (P = 4 x 10⁻⁷, OR = 1.59, 95% C.I. = 1.33-1.91; Table 2), and meta-analysis showed a combined P = 3 x 10⁻¹¹. We conclude that variation linked to rs11574637 marks a confirmed SLE risk allele, and that the ITGAM/ITGAX locus contributes to SLE pathogenesis.

rs 11574637 is part of a large block of correlated SNPs that clovers ∼150 kb encoding several genes including *ITGAM* and the 5' portion of *ITGAX* (Figure 3A). Both *ITGAM* and *ITGAX* are expressed at detectable levels in EBV transformed B cells, however rs11574637 did not correlate significantly with mRNA expression levels of either gene (data not shown). Of potential interest, SNP rs11574637 is correlated with 2 nonsynonymous variants of *ITGAM.* In the control population, a Pro1146Ser variant (rs1143678, P = 2.5 x 10-⁵) was correlated with an r² of 0.85 to the disease-associated rs11574637 variant. The "C" allele of rs1143678 and the 1146Ser allele form a haplotype on 18.2% of control chromosomes; the "C" allele is also present on a separate 2% haplotype lacking the 1146Ser allele. A second nonsynonymous allele (rs1143683, Ala858Val) was not directly genotyped in the current study, but is highly correlated with Pro1146Ser (r² = 0.85 in HapMap CEU). Further studies will be required to determine if the *ITGAM* nonsynonymous variants or additional allele(s) underlie the association within the *ITGAM*/*ITGAX* region.

### Associations With SLE Clinical Features

Finally, the associations between the two top SNPs, rs11574637 (*BLK*) and rs13277113 (*ITGAM*)*,* and the presence of individual ACR criteria, using the combined case series 1-3 (Figure 9, and see Methods), was examined. The strongest association was an inverse relationship between the rs11574637 minor allele and the presence of arthritis, OR=0.73 (95% CI = 0.59-0.91, P = 0.0045). Both variants were modestly associated with hematologic criteria: rs11574637, OR=1.21 (95% CI = 1.00-1.47, P = 0.04) and rs13277113, OR = 1.23 (95% CI = 1.03-1.46, P = 0.02). No other significant associations were observed.

### Discussion

The current effort describes the results of a comprehensive genome-wide association study performed in SLE. By studying a large number of SLE cases -1311 - and an even larger group of controls - 3340, the major alleles contributing risk to SLE were detected. The strong signals observed in the HLA region, *IRF5* and *STAT4* served as positive controls for the experiment, and confirm that these loci are among the most important genetic factors in this disease.

The src family tyrosine kinase *BLK* is an interesting new candidate gene for SLE. Expression of *BLK* is highly restricted to the B lymphocyte lineage.⁴¹ *Blk* expression in the mouse is first observed in cycling late pro-B cells, continues throughout B cell development, and is subsequently downregulated in plasma B cells.⁴² A knockout mouse for *Blk* has no gross phenotype⁴³, and functional studies in human B cells have not been performed. Without being bound by theory, *BLK* is one of the tyrosine kinases that transduces signals downstream of the B cell receptor, and it perhaps has a redundant role in the mouse, given the lack of a phenotype in the knockouts. There is precedent for major species differences in the role of B cell receptor associated kinases. For example, Bruton's tyrosine kinase (*BTK*) deficiency in humans leads to X-linked agammaglobulinemia, and a complete lack of B cells.⁴⁴ However, deficiency of *Btk* in the mouse is associated with a much milder phenotype, with production of mature B cells that are functionally impaired.⁴⁵

Signaling through the B cell receptor is important for establishing the B cell repertoire through induction of anergy, deletion and receptor editing during B cell development.^{46,47} As shown here, the risk allele at *BLK* is associated with reduced expression *of BLK* mRNA in transformed B cell lines. Without being bound by theory, the altered protein levels of BLK might influence tolerance mechanisms in B cells, predisposing individuals to systemic autoimmunity. A similar mechanism has recently been shown for *Ly108,* one of the major genetic loci in the NZM2410 mouse model for lupus.⁴⁸ Accordingly, in one embodiment of the invention, one of skill in the art can use the information provided herein to assess the effect of the risk haplotype on expression of the ubiquitously expressed gene *C8orf13.*

A second locus identified in this scan is *ITGAM*/*ITGAX.* While ITGAX is not excluded from consideration based on the strong LD in the region that extends into the 5' portion of *ITGAX,* the data suggests that *ITGAM* may be the relevant gene in the region. ITGAM (also known as CD11b, Mac-1, and the complement receptor type 3) is a well characterized integrin alpha chain molecule that is expressed by a variety of myeloid cell types, including dendritic cells, macrophages, monocytes, and neutrophils.⁴⁹⁻⁵¹ ITGAM forms a heterodimer with ITGB2 (CD18), and mediates adhesion between cell types in the immune system, and the adhesion of myeloid cells to endothelium.⁵² Mice deficient for *ITGAM* show enhanced disease progression and inflammation in several models of autoimmunity,⁵³⁻⁵⁵ including lupus, and recent data suggest that *ITGAM* may function normally to suppress Th17 differentiation,⁵⁶ a pathway that has been linked with induction of autoimmunity. Of interest, the expression of GD11b has been reported to be elevated on the neutrophils of active SLE patients.⁵⁷ The risk allele for *ITGAM* with its two highly correlated nonsynonymous alleles may predispose to altered function and/or regulation of expression of the protein, thereby contributing to systemic autoimmunity.

In summary, the current data identify two new susceptibility loci for SLE: *BLK*/*C8orf13* on chromosome 8 and *ITGAM*/*ITGAX* on chromosome 16. The most likely candidate genes within these two loci are *BLK* and *ITGAM.* The identification of these genes provides important new insights into the genetic basis of SLE and also suggests potential new targets for therapy.

### Example 3 A Genome-Wide Association Scan in Systemic Lupus Erythematosus (SLE), and Identification of Novel Loci Correlated With SLE

In this Example, the initial data set consisted of the cases and the controls from the genome wide association study described above in Examples 1 and 2, with genotypes from Illumina HumanHap550v1 chips and Illumina HumanHap550v3 chips. The data set from Illumina HumanHap550v1 chips consisted of 555352 SNPs in each of 464 cases and 1962 controls. The data set from Illumina HumanHap550v3 chips consisted of 561466 SNPs in each of 971 cases and 1621 controls. For each data set, quality-control filters were applied similarly to the manner described above in Examples 1 and 2. The resulting data set from HumanHap550v1 chips consisted of 534523 SNPs in each of 422 cases and 1881 controls. The resulting data set from HumanHap550v3 chips consisted of 549273 SNPs in each of 929 cases and 1558 controls.

The above data set from Illumina HumanHap550v1 chips was merged with the above data set from Illumina HumanHap550v3 chips. The resulting data set consisted of 564307 SNPs in each of 1351 cases and 3439 controls. This data set was merged with genotypes from the CGEMS breast and prostate cancer studies: 553820 SNPs in each of 4527 samples, used as controls. The resulting data set consisted of 570099 SNPs in each of 1351 cases and 7966 controls. Quality-control filters were applied similarly to the manner described above in Examples 1 and 2. The resulting data set consisted of 446856 SNPs in each of 1351 cases and 7966 controls.

The above data set was used to impute genotype probabilities for each polymorphic CEU SNP in the Phase II HapMap, via the program IMPUTE (www.stats.ox.ac.uk/∼marchini/software/gwas/impute.html). The recommended effective population size (-Ne 11418) was used.

Association between SLE status and each imputed SNP was calculated with the program SNPTEST (www.stats.ox.ac.uk/∼marchini/software/gwas/snptest.html). Population outliers were excluded; they were determined with the program EIGENSTRAT, in a manner similar to that described above in Examples 1 and 2. Both additive and general frequentist models were tested.

### References

1. Hochberg MC. The epidemiology of systemic lupus erythematosus. In: Wallace DJ, Hahn BH, eds. Dubois' Lupus Erythematosus. 5th ed. Baltimore: Williams and Wilkins; 1997.
2. Wakeland EK, Liu K, Graham RR, Behrens TW. Delineating the genetic basis of systemic lupus erythematosus. Immunity 2001;15(3):397-408.
3. Nath SK, Kilpatrick J, Harley JB. Genetics of human systemic lupus erythematosus: the emerging picture. Curr Opin Immunol 2004;16(6):794-800.
4. Goldberg MA, Arnett FC, Bias WB, Shulman LE. Histocompatibility antigens in systemic lupus erythematosus. Arthritis Rheum 1976;19(2):129-32.
5. Graham RR, Ortmann WA, Langefeld CD, et al. Visualizing human leukocyte antigen class II risk haplotypes in human systemic lupus erythematosus. Am J Hum Genet 2002;71(3):543-53.
6. Graham RR, Ortmann W, Rodine P, et al. Specific combinations of HLA-DR2 and DR3 class II haplotypes contribute graded risk for disease susceptibility and autoantibodies in human SLE. Eur J Hum Genet 2007;15(8):823-30.
7. Sigurdsson S, Nordmark G, Goring HH, et al. Polymorphisms in the tyrosine kinase 2 and interferon regulatory factor 5 genes are associated with systemic lupus erythematosus. Am J Hum Genet 2005;76(3):528-37.
8. Graham RR, Kozyrev SV, Baechler EC, et al. A common haplotype of interferon regulatory factor 5 (IRF5) regulates splicing and expression and is associated with increased risk of systemic lupus erythematosus. Nat Genet 2006;38(5):550-55.
9. Graham RR, Kyogoku C, Sigurdsson S, et al. Three functional variants of IFN regulatory factor 5 (IRF5) define risk and protective haplotypes for human lupus. Proc Natl Acad Sci U S A 2007;104(16):6758-63.
10. Remmers EF, Plenge RM, Lee AT, et al. STAT4 and the risk of rheumatoid arthritis and systemic lupus erythematosus. N Engl J Med 2007;357(10):977-86.
11. Ronnblom L, Alm GV. A pivotal role for the natural interferon alpha-producing cells (plasmacytoid dendritic cells) in the pathogenesis of lupus. J Exp Med 2001;194(12):F59-63.
12. Baechler EC, Gregersen PK, Behrens TW. The emerging role of interferon in human systemic lupus erythematosus. Curr Opin Immunol 2004;16(6):801-07.
13. Banchereau J, Pascual V. Type I interferon in systemic lupus erythematosus and other autoimmune diseases. Immunity 2006;25(3):383-92.
14. Miyagi T, Gil MP, Wang X, Louten J, Chu WM, Biron CA. High basal STAT4 balanced by STAT1 induction to control type 1 interferon effects in natural killer cells. J Exp Med 2007;Epublication; Sept 10.
15. A haplotype map of the human genome. Nature 2005;437(7063):1299-320.
16. Dewan A, Liu M, Hartman S, et al. HTRA1 promoter polymorphism in wet age-related macular degeneration. Science 2006;314(5801):989-92.
17. Genome-wide association study of 14,000 cases of seven common diseases and 3,000 shared controls. Nature 2007;447(7145):661-78.
18. Matarin M, Brown WM, Scholz S, et al. A genome-wide genotyping study in patients with ischaemic stroke: initial analysis and data release. Lancet neurology 2007;6(5):414-20.
19. Moffatt MF, Kabesch M, Liang L, et al. Genetic variants regulating ORMDL3 expression contribute to the risk of childhood asthma. Nature 2007;448(7152):470-73.
20. Plenge RM, Seielstad M, Padyukov L, et al. TRAF1-C5 as a Risk Locus for Rheumatoid Arthritis -- A Genomewide Study. N Engl J Med 2007.
21. Saxena R, Voight BF, Lyssenko V, et al. Genome-wide association analysis identifies loci for type 2 diabetes and triglyceride levels. Science 2007;316(5829):1331-36.
22. Scott LJ, Mohlke KL, Bonnycastle LL, et al. A genome-wide association study of type 2 diabetes in Finns detects multiple susceptibility variants. Science 2007;316(5829):1341-45.
23. Scuteri A, Sanna S, Chen WM, et al. Genome-Wide Association Scan Shows Genetic Variants in the FTO Gene Are Associated with Obesity-Related Traits. PLoS Genet 2007;3(7):e115.
24. Pe'er I, de Bakker PI, Maller J, Yelensky R, Altshuler D, Daly MJ. Evaluating and improving power in whole-genome association studies using fixed marker sets. Nat Genet 2006;38(6):663-67.
25. Bauer JW, Baechler EC, Petri M, et al. Elevated serum levels of interferon-regulated chemokines are biomarkers for active human systemic lupus erythematosus. PLoS medicine 2006;3(12):e491.
26. Criswell LA, Pfeiffer KA, Lum RF, et al. Analysis of families in the multiple autoimmune disease genetics consortium (MADGC) collection: the PTPN22 620W allele associates with multiple autoimmune phenotypes. Am J Hum Genet 2005;76(4):561-71.
27. Seligman VA, Suarez C, Lum R, et al. The Fcgamma receptor IIIA-158F allele is a major risk factor for the development of lupus nephritis among Caucasians but not non-Caucasians. Arthritis Rheum 2001;44(3):618-25.
28. Demirci FY, Manzi S, Ramsey-Goldman R, et al. Association of a common interferon regulatory factor 5 (IRF5) variant with increased risk of systemic lupus erythematosus (SLE). Ann Hum Genet 2007;71(Pt 3):308-11.
29. Hochberg MC. Updating the American College of Rheumatology revised criteria for the classification of systemic lupus erythematosus. Arthritis Rheum 1997;40(9):1725.
30. Mitchell MK, Gregersen PK, Johnson S, Parsons R, Vlahov D. The New York Cancer Project: rationale, organization, design, and baseline characteristics. J Urban Health 2004;81(2):301-10.
31. Gunderson KL, Steemers FJ, Ren H, et al. Whole-genome genotyping. Methods Enzymol 2006;410:359-76.
32. Hsu TM, Chen X, Duan S, Miller RD, Kwok PY. Universal SNP genotyping assay with fluorescence polarization detection. Biotechniques 2001;31(3):560.
33. Price AL, Patterson NJ, Plenge RM, Weinblatt ME, Shadick NA, Reich D. Principal components analysis corrects for stratification in genome-wide association studies. Nat Genet 2006;38(8):904-09.
34. Purcell S, Neale B, Todd-Brown K, et al. PLINK: a tool set for whole-genome association and population-based linkage analyses. Am J Hum Genet 2007;81(3):559-75.
35. Devlin B, Roeder K, Wasserman L. Genomic control for association studies: a semiparametric test to detect excess-haplotype sharing. Biostatistics 2000;1(4):369-87.
36. Stranger BE, Nica AC, Forrest MS, et al. Population genomics of human gene expression. Nat Genet 2007;39(10):1217-24.
37. Dixon AL, Liang L, Moffatt MF, et al. A genome-wide association study of global gene expression. Nat Genet 2007;39(10):1202-7.
38. de Bakker PI, McVean G, Sabeti PC, et al. A high-resolution HLA and SNP haplotype map for disease association studies in the extended human MHC. Nat Genet 2006;38(10):1166-72.
39. Hirschhorn JN, Daly MJ. Genome-wide association studies for common diseases and complex traits. Nature reviews 2005;6(2):95-108.
40. Cheung VG, Spielman RS, Ewens KG, Weber TM, Morley M, Burdick JT. Mapping determinants of human gene expression by regional and genome-wide association. Nature 2005;437(7063):1365-69.
41. Dymecki SM, Zwollo P, Zeller K, Kuhajda FP, Desiderio SV. Structure and developmental regulation of the B-lymphoid tyrosine kinase gene blk. J Biol Chem 1992;267(7):4815-23.
42. Wasserman R, Li YS, Hardy RR. Differential expression of the blk and ret tyrosine kinases during B lineage development is dependent on Ig rearrangement. J Immunol 1995;155(2):644-51.
43. Texido G, Su IH, Mecklenbrauker I, et al. The B-cell-specific Src-family kinase Blk is dispensable for B-cell development and activation. Mol Cell Biol 2000;20(4):1227-33.
44. Tsukada S, Saffran DC, Rawlings DJ, et al. Deficient expression of a B cell cytoplasmic tyrosine kinase in human X-linked agammaglobulinemia. Cell 1993;72(2):279-90.
45. Khan WN, Alt FW, Gerstein RM, et al. Defective B cell development and function in Btk-deficient mice. Immunity 1995;3(3):283-99.
46. Cornall RJ, Goodnow CC. B cell antigen receptor signalling in the balance of tolerance and immunity. Novartis Foundation symposium 1998;215:21-30.
47. Nemazee D, Weigert M. Revising B cell receptors. J Exp Med 2000;191(11):1813-7.
48. Kumar KR, Li L, Yan M, et al. Regulation of B cell tolerance by the lupus susceptibility gene Ly108. Science 2006;312(5780):1665-9.
49. Abbas AR, Baldwin D, Ma Y, et al. Immune response in silico (IRIS): immune-specific genes identified from a compendium of microarray expression data. Genes Immun 2005;6(4):319-31.
50. Hynes RO. Integrins: versatility, modulation, and signaling in cell adhesion. Cell 1992;69(1):11-25.
51. Lu H, Smith CW, Perrard J, et al. LFA-1 is sufficient in mediating neutrophil emigration in Mac-1-deficient mice. J Clin Invest 1997;99(6):1340-50.
52. Dunne JL, Collins RG, Beaudet AL, Ballantyne CM, Ley K. Mac-1, but not LFA-1, uses intercellular adhesion molecule-1 to mediate slow leukocyte rolling in TNF-alpha-induced inflammation. J Immunol 2003;171(11):6105-10 and Tables 1-6.
53. Hammerberg C, Katiyar SK, Carroll MC, Cooper KD. Activated complement component 3 (C3) is required for ultraviolet induction of immunosuppression and antigenic tolerance. J Exp Med 1998;187(7):1133-38.
54. Sohn JH, Bora PS, Suk HJ, Molina H, Kaplan HJ, Bora NS. Tolerance is dependent on complement C3 fragment iC3b binding to antigen-presenting cells. Nat Med 2003;9(2):206-12.
55. Watts GM, Beurskens FJ, Martin-Padura I, et al. Manifestations of inflammatory arthritis are critically dependent on LFA-1. J Immunol 2005;174(6):3668-75.
56. Ehirchiou D, Xiong Y, Xu G, Chen W, Shi Y, Zhang L. CD11b facilitates the development of peripheral tolerance by suppressing Th17 differentiation. J Exp Med 2007;204(7):1519-24.
57. Buyon JP, Shadick N, Berkman R, et al. Surface expression of Gp 165/95, the complement receptor CR3, as a marker of disease activity in systemic Lupus erythematosus. Clin Immunol Immunopathol 1988;46(1):141-49.
58. Giglio S, Broman KW, Matsumoto N, et al. Olfactory receptor-gene clusters, genomic-inversion polymorphisms, and common chromosome rearrangements. Am J Hum Genet 2001;68(4):874-83.
59. Sugawara H, Harada N, Ida T, et al. Complex low-copy repeats associated with a common polymorphic inversion at human chromosome 8p23. Genomics 2003;82(2):238-44.

### Example 4 Genome-wide association scan in 1310 SLE cases and 7859 controls

### Methods: Sample information and genotyping SLE cases and controls

The selection and genotyping of the SLE case samples was described previously (*1*). Briefly, DNA samples from a) 338 subjects from the Autoimmune Biomarkers Collaborative Network (ABCoN), an NIH/NIAMS-funded repository (*2*), b) 141 subjects from the Multiple Autoimmune Disease Genetics Consortium (MADGC) (*3*), (ABCON+MADGC = case series 1), c) 613 subjects from the University of California San Francisco (UCSF) Lupus Genetics Project (*4*, *5*) (case series 2) and d) 335 subjects from the University of Pittsburgh Medical Center (UPMC) (*6*) plus 8 samples collected at The Feinstein Institute for Medical Research (case series 3) were genotyped using the Illumina 550K array. All SLE cases were North Americans of European descent, as determined by self-report. The diagnosis of SLE (fulfillment of four or more of the American College of Rheumatology (ACR) defined criteria (*7*)) was confirmed in all cases by medical record review (94%) or through written documentation of criteria by treating rheumatologists (6%). Clinical data for these case series were presented elsewhere (*4*, *3, 2, 6, 5*).

A total of 8147 control samples genotyped using the Illumina 550K array were examined in the association analyses. Three sources were used for controls (all North Americans of European descent): 1861 samples from the New York Health Project (NYHP) collection (*8*); 1722 samples from the publicly available iControlDB database (www.illumina.com/pages.ilmn?ID=231); and 4564 samples from the publicly available Cancer Genetics Markers of Susceptibility (CGEMS) project (http://cgems.cancer.gov/). Genotyping of the NYHP samples was described previously (*1*).

### Genotype data quality filters

Sample and SNP filtering was conducted using analytical modules within the software programs PLINK (*9*) and EIGENSTRAT (*10*), as described below.

### a) SLE cases, NYCP samples, and iControlDB samples

The Illumina 550K SNP array, version 1 (HH550v1) was used to genotype 464 cases and 1962 controls, and the Illumina 550K SNP array, version 3 (HH550v3) was used to genotype 971 cases and 1621 controls as described (*1*). Samples where the reported sex did not match the observed sex (HH550v1: 10, HH550v3: 11) and samples with > 5% missing genotypes (HH550v1: 25, HH550v3: 21) were excluded from the analysis. Cryptic relatedness between the SLE cases and controls was determined by the estimation of the identity-by-state (IBS) across the genome for all possible pair-wise sample combinations. A sample from each pair estimated to be duplicates or 1st-3rd degree relatives were excluded (Pi_hat ≥ 0.10 and Z1 ≥ 0.15; HH550v1: 88, HH550v3: 73). SNPs with HWE P ≤ 1 x 10⁻⁶ in controls (HH550v1 : 3176, HH550v3: 2240) and SNPs with > 5% missing data (HH550v1 : 12605, HH550v3: 7137) were removed. The SNPs were tested for a significant difference in the frequency of missing data between cases and controls, and SNPs with P ≤ 1 x 10⁻⁵ in the differential missingness test implemented in PLINK were removed (HH550v1: 5027, HH550v3: 2804). The SNPs were also tested for a significant allele frequency difference between genders; all SNPs had P ≥ 1 x 10⁻⁹ in controls. The data was examined for the presence of batch effects (for example, between ABCoN samples and all other cases), and SNPs with an allele frequency difference with a P < 1 x 10⁻⁹ were excluded (HH550v1: 18, HH550v3: 10). Variants with heterozygous haploid genotypes were set to missing (HH550v1: 2305, HH550v3: 875). In addition, variants with a minor allele frequency < 0.0001 were removed (HH550v1: 97, HH550v3: 57).

### b) CGEMS samples

For the 2277 prostate cancer samples and, separately, 2287 breast cancer samples, heterozygous haploid genotypes were set to missing (prostate: 2717, breast: 0). Samples where the reported gender did not match the observed gender (prostate: 0, breast: 2) and samples with > 5% missing data (prostate: 15, breast: 1) were excluded. Samples were tested for cryptic relatedness, as described above, and one sample was removed from each pair estimated to be duplicates or 1st-3rd degree relatives (Pi_hat ≥ 0.10 and Z1 ≥ 0.15; prostate: 12, breast: 7). SNPs with a MAF < 0.0001 (prostate: 3254, breast: 2166) were removed.

### c) All samples

Additional data quality filters were applied to the merged dataset consisting of all SLE cases and controls. SNPs with > 5% missing data (N=65,421) and samples with > 5% missing data (N=0) were removed. A test for duplicate samples was conducted using 957 independent SNPs with MAF ≥ 0.45, and no duplicate samples were found. SNPs with HWE P ≤ 1 x 10⁻⁶ in controls (N=2174) and SNPs with > 2% missing data (N=5522) were removed. We tested the SNPs for a significant difference in the proportion of missing data between cases and controls and removed SNPs with excess missing data differential (P ≤ 1 x 10⁻⁵, N=16080). SNPs were tested for a significant difference between genders and all SNPs had P ≥ 1 x 10⁻⁹ in controls. SNPs were also examined for the presence of batch effects; in particular, between CGEMS breast cancer samples and all other controls, and between CGEMS prostate cancer samples and all other controls and removed SNPs with P < 1 x 10⁻⁹ (N=73). After application of the above quality filters, 480,831 SNPs remained.

The cases and controls were tested for the presence of population outliers using EIGENSTRAT. SNPs with MAF < 2% in cases (N=16068), HWE P ≤ 1 x 10⁻⁴ in controls (N=977), or > 1% missing data (N=17029); SNPs in regions of abnormal LD patterns due to structural variation on chromosomes 6 (from 24-36 Mb), 8 (8-12 Mb), 11 (42-58 Mb), and 17 (40-43 Mb); and SNPs in the pseudoautosomal region of chromosome X (N=12) were excluded for the purpose of determining the principal components (EIGENSTRAT) of variation to detect population outliers. Samples with greater than 6 standard deviations from the mean along any of the top 10 principal components were removed (N=148).

The final data set had 1310 cases, 7859 controls, and 480,831 SNPs, and the genomic control inflation factor (λ_{gc}) (*11*) was 1.06 after the application of the above data quality filters.

### Imputing unobserved genotypes

The extensive linkage disequilibrium present in the human genome allows the inference of untyped variants in certain situations with a high degree of confidence. IMPUTE, a program for imputing unobserved genotypes in genome-wide case-control studies based on a set of known haplotypes (HapMap Phase II haplotypes, www.hapmap.org), was used in the analysis (www.stats.ox.ac.uk/∼marchini/software/gwas/impute.html).

### Imputing the GNE cases and NYCP, iDB, and CGEMS controls

After quality control filters, there were 1310 GNE cases, 3344 NYCP and iDB controls, 4515 CGEMS controls, and 446,856 SNPs. The program IMPUTE (v0.3.1) was run with the included CEU haplotype, legend, and map files aligned to NCBI Build 35. The effective population size was set to the recommended value of 11418. No strand file was used; strand alignment checking in IMPUTE was turned on. Cases, NYCP and iDB controls, and CGEMS controls were imputed separately, and each chromosome was imputed separately in its entirety. 2,562,708 SNPs were imputed.

SNPTEST (v1.1.3) was used to do association tests on both the actual and imputed genotypes. For SNPs that were already genotyped, the actual genotypes were used. The association test was the Cochran-Armitage test for an additive genetic effect, with the "-proper" option to completely take into account the uncertainty of the genotypes. Only SNPs with an information score above 0.50 (i.e., frequentist_add_proper_info > 0.50) were kept (2,481,907 SNPs [97%]).

Results. A non-redundant list of SLE loci associated with SLE (P < 1x10⁻⁵) in the analysis of 1310 cases and 7859 controls is presented in Table 1. The rank ordered list was generated by displaying the single variant with the lowest P value in a +/- 100kb interval from generated by displaying the single variant with the lowest P value in a +/- 100kb interval from the analysis of 2.3 million SNPs as described above.

**Table 1: Loci associated with SLE (P ≤ 1x10⁻⁵) in the analysis of 1310 cases and 7859 controls. The rank ordered list was generated by displaying the single variant with the lowest P value in a +/- 100kb interval from the analysis of 2.3 million SNPs as described. The SNP (dbSNP id), Chromosome , position (base pair position in build 35 of the human genome), minor allele frequency in the SLE cases and controls, P value from SNPTEST (under an additive model, correcting for imputation accuracy), the Imputation Information Score (an estimate of the imputation accuracy) and Odds Ratio (with 95% confidence intervals) are shown.**

| Allele frequency | | | | | | | |
|---|---|---|---|---|---|---|---|
| SNP | Chromosome | Position (Build 35) | Cases (N=1310) | Controls (N=7859) | P | Imputation Information Score | Odds Ratio (95% c.i.) |
| rs2187668 | 6 | 32713862 | 0.190 | 0.117 | 2.49E-24 | 1.00 | 1.76 (1.58-1.97) |
| rs13236009 | 7 | 128257124 | 0.175 | 0.111 | 2.37E-20 | 0.96 | 0.59 (0.52-0.66) |
| rs11889341 | 2 | 191769248 | 0.308 | 0.230 | 1.55E-19 | 0.97 | 1.49 (1.36-1.64) |
| rs6565228 | 16 | 31236781 | 0.041 | 0.029 | 1.40E-11 | 0.69 | 0.71 (0.56-0.91) |
| rs2736345 | 8 | 11389894 | 0.335 | 0.278 | 3.80E-09 | 0.96 | 1.31 (1.2-1.44) |
| rs6889239 | 5 | 150437964 | 0.300 | 0.251 | 1.21E-07 | 1.00 | 0.78 (0.72-0.86) |
| rs2391592 | 7 | 27983196 | 0.531 | 0.472 | 2.34E-07 | 0.92 | 0.79 (0.72-0.86) |
| rs2177770 | 2 | 141630291 | 0.086 | 0.064 | 2.54E-07 | 0.72 | 1.37 (1.15-1.63) |
| rs12039904 | 1 | 169943930 | 0.283 | 0.238 | 8.66E-07 | 0.95 | 1.26 (1.15-1.39) |
| rs4591368 | 2 | 71637413 | 0.008 | 0.004 | 1.03E-06 | 0.57 | 0.5 (0.3-0.84) |
| rs12882608 | 14 | 82621870 | 0.205 | 0.170 | 1.10E-06 | 0.90 | 0.79 (0.71-0.89) |
| rs3024493 | 1 | 203332363 | 0.187 | 0.148 | 1.96E-06 | 0.96 | 0.76 (0.68-0.85) |
| rs11678272 | 2 | 42061217 | 0.313 | 0.270 | 2.38E-06 | 0.98 | 0.81 (0.74-0.89) |
| rs874952 | 2 | 65520176 | 0.121 | 0.157 | 2.77E-06 | 1.00 | 1.35 (1.19-1.53) |
| rs2053482 | 8 | 98289410 | 0.076 | 0.054 | 3.01E-06 | 0.93 | 0.7 (0.6-0.83) |
| rs2431697 | 5 | 159812556 | 0.389 | 0.438 | 3.50E-06 | 1.00 | 1.22 (1.12-1.33) |
| rs6679677 | 1 | 114015850 | 0.107 | 0.079 | 3.55E-06 | 0.91 | 0.72 (0.62-0.83) |
| rs12445476 | 16 | 84548770 | 0.158 | 0.196 | 4.11E-06 | 0.99 | 1.3 (1.16-1.46) |
| rs2208384 | 1 | 232216137 | 0.212 | 0.252 | 4.43E-06 | 0.99 | 1.26 (1.14-1.39) |
| rs6879995 | 5 | 158447777 | 0.353 | 0.304 | 4.47E-06 | 0.92 | 0.8 (0.73-0.88) |
| rs10502821 | 18 | 39720893 | 0.003 | 0.001 | 4.54E-06 | 0.89 | 0.18 (0.07-0.45) |
| rs3790565 | 1 | 67523377 | 0.225 | 0.187 | 4.84E-06 | 1.00 | 0.79 (0.71-0.87) |
| rs2024831 | 6 | 14822843 | 0.218 | 0.185 | 5.17E-06 | 0.92 | 0.81 (0.73-0.9) |
| rs2066943 | 4 | 85247083 | 0.013 | 0.026 | 5.68E-06 | 0.61 | 0.5 (0.34-0.74) |
| rs12986652 | 2 | 180300843 | 0.112 | 0.087 | 5.69E-06 | 0.96 | 0.75 (0.66-0.86) |
| rs1196592 | 18 | 34246756 | 0.356 | 0.312 | 5.77E-06 | 1.00 | 0.82 (0.75-0.89) |
| rs7759216 | 6 | 106695307 | 0.425 | 0.377 | 5.84E-06 | 1.00 | 1.22 (1.12-1.33) |
| rs17484292 | 1 | 180031707 | 0.056 | 0.040 | 5.97E-06 | 0.75 | 0.7 (0.57-0.85) |
| rs1579289 | 5 | 107837653 | 0.134 | 0.168 | 6.44E-06 | 0.94 | 1.31 (1.15-1.48) |
| rs11970105 | 6 | 49429182 | 0.149 | 0.117 | 6.65E-06 | 0.89 | 0.76 (0.67-0.86) |
| rs10082917 | 12 | 40285103 | 0.390 | 0.346 | 6.93E-06 | 0.94 | 1.21 (1.1-1.32) |
| rs7006016 | 8 | 29655999 | 0.110 | 0.087 | 8.02E-06 | 0.80 | 1.29 (1.11-1.5) |
| rs11757479 | 6 | 114612099 | 0.059 | 0.040 | 8.63E-06 | 0.84 | 0.67 (0.55-0.81) |
| rs4968210 | 17 | 7398076 | 0.414 | 0.368 | 9.64E-06 | 0.94 | 0.83 (0.76-0.9) |

### Example 5 Meta-analysis of reported SLE risk loci in the GNE association scan

### Methods

### Examination of SLE literature and criteria for confirmed SLE loci

A total of 16 alleles met one of the criteria described below for confirmed SLE risk loci (Table 2).

### 1) SLE risk loci with at least 2 independent reports of P ≤ 1 x 10⁻⁵.

The literature was examined for loci with 2 independent reports in nonoverlapping SLE cohorts with a P ≤ 1 x 10⁻⁵. The literature search represents publications prior to April 2008. The identical variant (or proxy with r² > 0.3) showing association to SLE with the same direction of effect was required. A total of 7 alleles fulfilled the requirements, including HLA-DRB1*0301 (HLA-DR3,(*18*, *19*)), HLA-DRB1***1501 (HLA-DR2,(*18*, *19*)), Protein Tyrosine Phospatase Non-receptor type 22 (PTPN22, (*20, 21*)), Interferon Regulatory Factor 5 (IRF5, (*22*, *23*)), Signal Transducer and Activator of Transcription 4 (STAT4, (*5*, *21*)), B Lymphoid tyrosine Kinase (BLK, (*21*, *1*)) and Integrin Alpha M (ITGAM, (*1*, *24*))*.* The identical allele or best proxy (r² > 0.85) in the 1310 SLE case and 7859 control genome-wide association scan described here was advanced into the analysis (Table 2).

### 2) SLE risk loci with a single report of P ≤ 1 x 10⁻⁵.

A literature search for SLE risk loci with a reported P ≤ 1 x 10⁻⁵ in a single publication as of April 2008 was performed and a total of 18 loci were identified.

In 13 of the loci, the identical variant or near-perfect proxy (r²>0.9) was genotyped in the 1310 SLE case and 7859 control genome scan described above (Table 4). A meta-analysis using the methodology described below was performed for the 13 loci, and 8 of the loci achieved a P ≤ 5 x 10⁻⁸. The loci (labeled by a single gene within the locus) achieving genome-wide significance include; Pituitary Tumor-Transforming Protein 1 (*PTTG1*), APG5 autophagy 5-like (*ATG5*)*,* CTD-binding SR-like protein rA9 (*KIAA1542*), Ubiquitin-conjugating Enzyme E2L3 (*UBE2L3*), PX domain containing serine/threonine kinase (*PXK*)*,* Fc fragment of IgG, low affinity IIa, Receptor (*FCGR2A*), Tumor Necrosis Factor (ligand) Superfamily 4 (*TNFSF4*), and B-cell scaffold protein with Ankyrin repeats 1 (*BANK1*)*.* The variant reaching genome wide significance in the meta-analysis was advanced into the analysis (Table 5, Table 2). In the remaining 5 loci, the reported variant or near-perfect proxy (r²>0.9) was not genotyped in the 1310 SLE case and 7859 control SLE genome-wide association scan (Table 2). However, a variant in interleukin-1 receptor-associated kinase 1 (IRAK1) had an observed P ≤ 1 x 10⁻⁴ and was advanced into the analysis (Table 1).

### Meta-analysis

The corrected association statistic for each series was combined by the summing of the Z-scores weighted for cohort size.

**Table 2. Association statistics for 16 confirmed SLE risk alleles in our GWAS of 1310 SLE cases and 7859 controls. The alleles are by P value.**

| Allele frequency | | | | | | | | |
|---|---|---|---|---|---|---|---|---|
| Locus | Chromosome | SNP | Position* (Mb) | Minor allele | Case | Control | P value | Odds ratio (95% CI) |
| *HLA-DR3* | 6p21.32 | rs2187668 | 32.714 | T | 0.190 | 0.117 | 9.5x10⁻²⁵ | 1.76 (1.58-1.97) |
| | | rs1048863 | 128.18 | | | | | |
| *IRF5* | 7q32.1 | 1 | 8 191.79 | C | 0.170 | 0.109 | 1.4 x 10⁻¹⁹ | 1.68 (1.50-1.89) |
| *STAT4* | 2q32.2 | rs7574865 | 0 | T | 0.312 | 0.235 | 2.5 x 10⁻¹⁴ | 1.48 (1.34-1.64) |
| *ITGAM* | 16p11.2 | rs9888739 | 31.221 | T | 0.175 | 0.127 | 2.3 x 10⁻¹¹ | 1.46 (1.31-1.63) |
| | | rs1327711 | | | | | | |
| *BLK* | 8p23.1 | 3 | 11.387 159.81 | A | 0.294 | 0.242 | 1.7 x 10⁻⁸ | 1.30 (1.19-1.43) |
| *PTTG1* | 5q33.3 | rs2431697 | 3 106.69 | C | 0.389 | 0.438 | 3.3 x 10⁻⁶ | 0.82 (0.75-0.89) |
| *ATG5* | 6q21 | rs6568431 rs1048926 | 5 169.96 | A | 0.423 | 0.376 | 5.5 x 10⁻⁶ | 1.22 (1.12-1.32) |
| *TNFSF4* | 1q25.1 | 5 | 8 114.09 | C | 0.278 | 0.238 | 8.7 x 10⁻⁶ | 1.24 (1.09-1.30) |
| *PTPN22* | 1p13.2 | rs2476601 | 0 152.71 | A | 0.116 | 0.089 | 8.9 x 10⁻⁶ | 1.35 (1.18-1.54) |
| | Xq28 | rs2269368 | 1 158.29 | T | 0.175 | 0.141 | 1.1 x 10⁻⁵ | 1.29 (1.15-1.45) |
| *FCGR2A* | 1q23.3 | rs1801274 | 3 | A | 0.463 | 0.500 | 4.1 x 10⁻⁴ | 0.86 (0.79-0.94) |
| | 11p15.5 | rs4963128 | 0.580 | T | 0.303 | 0.333 | 3.1 x 10⁻³ | 0.87 (0.80-0.96) |
| *UBE2L3* | 22q11.21 | rs5754217 | 20.264 | T | 0.215 | 0.192 | 6.4 x 10⁻³ | 1.15 (1.04-1.27) |
| *PXK* | 3p14.3 | rs6445975 | 58.345 | G | 0.305 | 0.281 | 0.010 | 1.13 (1.03-1.23) |
| *HLA-DR2* | 6p21.32 | rs3129860 | 32.509 | A | 0.160 | 0.147 | 0.092 | 1.10 (0.98-1.24) |
| | | rs1051648 | 103.10 | | | | | |
| *BANK1* | 4q24 | 7 | 8 | A | 0.288 | 0.304 | 0.096 | 0.93 (0.85-1.01) |

| | | | | | | | | |
|---|---|---|---|---|---|---|---|---|
| *Positions are from NCBI Build 35. | | | | | | | | |

**Table 3. SLE risk loci with at least 2 independent reports with the same SNP (or proxy with r² > 0.3) with P ≤ 1 x 10⁻⁵.**

| | | Report 1 | | | Report 2 | | | | | GNE GWAS | |
|---|---|---|---|---|---|---|---|---|---|---|---|
| Locus | Chromosome | Allele | P value | Reference | Allele | r² to allele in Report 1 | P value | Reference | Additional references | SNP | r² to allele in Report 1 |
| *PTPN22* | 1p13.2 | rs2476601 | 1.0 x 10⁻⁵ | (*20*) | rs2476601 | 1.00 | 5.2 x 10⁻⁶ | (*21*) | (*25*) | rs2476601 | 1.00 |
| | | | | | | | | | | | |
| *STAT4* | 2q32.2 | rs7574865 | 1.9 x 10⁻⁹ | (*5*) | rs7574865 | 1.00 | 2.8 x 10⁻⁹ | (*21*) | (*1*) | rs7574865 | 1.00 |
| *HLA-DR2* | 6p21.32 | DRB1*1501 | 1.0 x 10⁻⁵ | (*18*) | DRB1*1501 | 1.00 | 1.0 x 10⁻⁷ | (*19*) | (*26*) | rs3129860 | 0.97 |
| | | | | | | | | | | | |
| *HLA-DR3* | 6p21.32 | DRB1*0301 | 1.0 x 10⁻⁶ | (*18*) | DRB1*0301 | 1.00 | 1.0 x 10⁻⁵ | (*19*) | (*21*), (*1*), (*26*), (*27*) | rs2187668 | 0.87 |
| | | | | | | | | | | | |
| *IRF5* | 7q32.1 | rs2004640 | 5.2 x 10⁻⁸ | (*22*) | rs2004640 | 1.00 | 4.4 x 10⁻¹⁶ | (*23*) | (*21*), (*1*), (*28*) | rs10488631 | -- |
| *BLK* | 8p23.1 | rs13277113 | 1.0 x 10⁻¹⁰ | (*1*) | rs6985109 | 0.33 | 2.5 x 10⁻¹¹ | (*21*) | | rs13277113 | 1.00 |
| *ITGAM* | 16p11.2 | rs1143679 | 6.9 x 10⁻²² | (*24*) | rs11574637 | -- | 3.0 x 10⁻¹¹ | (*1*) | (*21*) | rs9888739 | 0.86 |

**Table 4. SLE risk loci reported only once with P ≤ 1 x 10⁻⁵ and in which meta-analysis was possible. 8 loci had a meta P ≤ 5 x 10⁻⁸.**

| | | Report | | | GNE GWAS* | | | |
|---|---|---|---|---|---|---|---|---|
| Locus | Chromosome | Allele | P value | Reference | SNP | r² to allele in Report | P value | Meta P |
| *PTTG1* | 5q33.3 | rs2431697 | 1.0 x 10⁻¹⁰ | (*21*) | rs2431697 | 1.00 | 3.3 x 10⁻⁶ | 5.3 x 10⁻¹⁴ |
| *ATG5* | 6q21 | rs6568431 | 1.7 x 10⁻⁸ | (*21*) | rs6568431 | 1.00 | 5.5 x 10⁻⁶ | 2.7 x 10⁻¹² |
| | 11p15.5 | rs4963128 | 3.0 x 10⁻¹⁰ | (*21*) | rs4963128 | 1.00 | 3.1 x 10⁻³ | 1.0 x 10⁻⁹ |
| *UBE2L3* | 22q11.21 | rs5754217 | 7.5 x 10⁻⁸ | (*21*) | rs5754217 | 1.00 | 6.4 x 10⁻³ | 7.3 x 10⁻⁹ |
| *PXK* | 3p14.3 | rs6445975 | 7.1 x 10⁻⁹ | (*21*) | rs6445975 | 1.00 | 0.010 | 1.0 x 10⁻⁸ |
| *FCGR2A* | 1q23.3 | rs1801274 | 6.8 x 10⁻⁷ | (*21*) | rs1801274 | 1.00 | 4.1 x 10⁻⁴ | 3.9 x 10⁻⁸ |
| *TNFSF4* | 1q25.1 | rs12039904 | 1.0 x 10⁻⁵ | (*29*) | rs10489265 | 0.91 | 8.7 x 10⁻⁶ | -- |
| *BANK1* | 4q24 | rs10516487 | 3.7 x 10⁻¹⁰ | (*30*) | rs10516487 | 1.00 | 0.096 | -- |
| *NMNAT2* | 1q25.3 | rs2022013 | 1.1 x 10⁻⁷ | (*21*) | rs2022013 | 1.00 | 0.15 | 5.1 x 10⁻⁶ |
| *ICA1* | 7p21.3 | rs10156091 | 1.9 x 10⁻⁷ | (*21*) | rs10156091 | 1.00 | 0.095 | 2.0 x 10⁻⁵ |
| *LYN* | 8q12.1 | rs7829816 | 5.4 x 10⁻⁹ | (*21*) | rs7829816 | 1.00 | 0.48 | 3.6 x 10⁻³ |
| *SCUBE1* | 22q13.2 | rs2071725 | 1.2 x 10⁻⁷ | (*21*) | rs2071725 | 1.00 | 0.63 | 8.3 x 10⁻³ |
| *ITPR3* | 6p21.31 | rs3748079 | 2.9 x 10⁻⁸ | (*31*) | rs3748079 | 1.00 | 0.95 | -- |

| | | | | | | | | |
|---|---|---|---|---|---|---|---|---|
| *GNE GWAS: our GWAS of 1310 SLE cases and 7859 controls. | | | | | | | | |

**Table 5. SLE risk loci reported only once with P ≤ 1 x 10⁻⁵ and in which meta-analysis was not possible. Only IRAK1 had a SNP with P ≤ 1 x 10⁻⁵ in our GNE GWAS.**

| | | | Report | | | GNE GWAS* | | |
|---|---|---|---|---|---|---|---|---|
| Locus | Chromosome | Allele | P value | Reference | Best SNP^{†} | P value | SNP | P value |
| *IRAK1* | Xq28 | rs10127175 | 9.6 x 10⁻⁶ | (*32*) | rs2269368 | 1.1 x 10⁻⁵ | rs2269368 | 1.1 x 10⁻⁵ |
| *CRP* | 1q23.2 | rs3093061 | 6.4 x 10⁻⁷ | (*33*) | rs3820099 | 3.0 x 10⁻³ | -- | -- |
| *SELP* | 1q24.2 | rs3917815 | 5.7 x 10⁻⁶ | (*32*) | rs9332628 | 8.8 x 10⁻³ | -- | -- |
| *PDCD1* | 2q37.3 | rs11568821 | 1.0 x 10⁻⁵ | (*34*) | rs3892357 | 0.84 | -- | -- |
| *TYK2* | 19p13.2 | rs2304256 | 2.2 x 10⁻⁸ | (*22*) | rs12720356 | 2.7 x 10⁻³ | -- | -- |

| | | | | | | | | |
|---|---|---|---|---|---|---|---|---|
| *GNE GWAS: our GWAS of 1310 SLE cases and 7859 controls. †Best SNP: the SNP with the lowest P at that locus. | | | | | | | | |

### Example 6 Summary

### Summary of SLE risk loci

SLE risk loci were identified using two primary methods- a) analysis of 1310 SLE cases and 7859 controls, and b) a meta-analysis with previously reported SLE risk loci.

A non-redundant list of the variants with strong association to SLE risk (P < 1 x 10⁻⁶) is provided in Table 6.

### Algorithm for assessing SLE risk and response to therapy

Variants associated with a phenotype are known to interact in an additive, allelic dose dependent manner (*38*, *39*). In one exemplary embodiment, the following algorithm can be used to assess risk to lupus, disease severity, and response to therapy. Lupus cases can be stratified into groups based on the number of risk alleles carried. In this exemplary embodiment, the risk allele is defined as the allele enriched in lupus cases relative to controls from the loci. For example in Table 6, there are a total of 19 alleles from 18 loci, making the maximum possible number of risk alleles equal to 38. The lupus cases stratified by the number of risk alleles and tertiles of the resulting distribution can be determined. The tertiles of lupus cases can then be examined for differences in disease severity, risk and response to therapy.

**Table 6: Lupus risk loci**

| Locus | Chromosome | SNP | Position* (Mb) | P value | Source |
|---|---|---|---|---|---|
| *HLA-DR3* | 6p21.32 | rs2187668 | 32.714 | 9.5 x 10⁻²⁵ | Table 2 |
| *IRF5* | 7q32.1 | rs10488631 | 128.188 | 1.4 x 10⁻¹⁹ | Table 2 |
| *STAT4* | 2q32.2 | rs7574865 | 191.790 | 2.5 x 10⁻¹⁴ | Table 2 |
| *ITGAM* | 16p11.2 | rs9888739 | 31.221 | 2.3 x 10⁻¹¹ | Table 2 |
| *BLK* | 8p23.1 | rs13277113 | 11.387 | 1.7 x 10⁻⁸ | Table 2 |
| *PTTG1* | 5q33.3 | rs2431697 | 159.813 | 3.3 x 10⁻⁶ | Table 2 |
| *ATG5* | 6q21 | rs6568431 | 106.695 | 5.5 x 10⁻⁶ | Table 2 |
| *TNFSF4* | 1q25.1 | rs10489265 | 169.968 | 8.7 x 10⁻⁶ | Table 2 |
| *PTPN22* | 1p13.2 | rs2476601 | 114.090 | 8.9 x 10-⁶ | Table 2 |
| | Xq28 | rs2269368 | 152.711 | 1.1 x 10⁻⁵ | Table 2 |
| *FCGR2A* | 1q23.3 | rs1801274 | 158.293 | 4.1 x 10⁻⁴ | Table 2 |
| | 11p15.5 | rs4963128 | 0.580 | 3.1 x 10⁻³ | Table 2 |
| *UBE2L3* | 22q11.21 | rs5754217 | 20.264 | 6.4 x 10⁻³ | Table 2 |
| *PXK* | 3p14.3 | rs6445975 | 58.345 | 0.01 | Table 2 |
| *HLA-DR2* | 6p21.32 | rs3129860 | 32.509 | 0.092 | Table 2 |
| *BANK1* | 4q24 | rs10516487 | 103.108 | 0.096 | Table 2 |
| *TNIP1* | 5 | rs6889239 | 150.438 | 2.2 x 10⁻⁸ | Table 1 |
| *JAZF1* | 7 | rs2391592 | 27.983 | 2.3 x 10⁻⁷ | Table 1 |
| *LRP1B* | 2 | rs2177770 | 141.630 | 2.5 x 10⁻⁷ | Table 1 |

| | | | | | |
|---|---|---|---|---|---|
| * Chromosomal position of variant in basepairs in NCBI Build 35 (Hg17, May 2004) of the Human genome (http://www.ncbi.nhn.nih.eov/genome/guide/human/release_notes.html#b35) | | | | | |

### References

- 1.: G. Hom et al., N Engl J Med 358, 900 (2008).
- 2.: J. W. Bauer et al., PLoS Med 3, e491 (2006).
- 3.: L. A. Criswell et al., Am J Hum Genet 76, 561 (2005).
- 4.: V. A. Seligman et al., Arthritis Rheum 44, 618 (2001).
- 5.: E. F. Remmers et al., N Engl J Med 357, 977 (2007).
- 6.: F. Y. Demirci et al., Ann Hum Genet 71, 308 (2007).
- 7.: M. C. Hochberg, Arthritis Rheum 40, 1725 (1997).
- 8.: M. K. Mitchell, P. K. Gregersen, S. Johnson, R. Parsons, D. Vlahov, J Urban Health 81, 301 (2004).
- 9.: S. Purcell et al., Am J Hum Genet 81, 559 (2007).
- 10.: A. L. Price et al., Nat Genet 38, 904 (2006).
- 11.: B. Devlin, K. Roeder, L. Wasserman, Biostatistics 1, 369 (2000).
- 12.: R. R. Graham et al., Arthritis research 3, 299 (2001).
- 13.: M. C. Hochberg, Arthritis and Rheumatism 40, 1725 (1997).
- 14.: C. Wellcome Trust Case Control, Nature 447, 661 (2007).
- 15.: S. Purcell.
- 16.: S. Purcell et al., American Journal of Human Genetics 81, 559 (2007).
- 17.: A. L. Price et al., Nat Genet 38, 904 (2006).
- 18.: K. Hartung, Baur, M.P., Coldewey, R., Fricke, M., Kalden, J.R., Lakomek, H.J., Peter, H.H., Schendel, D., Schneider, P.M., Seuchter, S.A., Stangel, W., Deicher, H.R.G., J Clin. Invest. 90, 1346 (1992).
- 19.: Z. Yao et al., Eur J Immunogenet 20, 259 (1993).
- 20.: Y. H. Lee et al., Rheumatology (Oxford) 46, 49 (2007).
- 21.: J. B. Harley et al., Nat Genet 40, 204 (2008).
- 22.: S. Sigurdsson et al., Am JHum Genet 76, 528 (2005).
- 23.: R. R. Graham et al., Nat Genet 38, 550 (2006).
- 24.: S. K. Nath et al., Nat Genet 40, 152 (2008).
- 25.: C. Kyogoku et al., Am J Hum Genet 75, 504 (2004).
- 26.: J. B. Harley, K. L. Moser, P. M. Gaffney, T. W. Behrens, Curr Opin Immunol 10, 690 (1998).
- 27.: M. M. Fernando et al., PLoS Genet 3, e192 (2007).
- 28.: R. R. Graham et al., Proc Natl Acad Sci U S A 104, 6758 (2007).
- 29.: D. S. Cunninghame Graham et al., Nature Genetics 40, 83 (2008).
- 30.: S. V. Kozyrev et al., Nat Genet 40, 211 (2008).
- 31.: T. Oishi et al., J Hum Genet 53, 151 (2008).
- 32.: C. O. Jacob et al., Arthritis Rheum 56, 4164 (2007).
- 33.: J. C. Edberg et al., Hum Mol Genet 17, 1147 (2008).
- 34.: L. Prokunina et al., Nat Genet 32, 666 (2002).
- 35.: S. Paabo, Nature 421, 409 (2003).
- 36.: K. A. Frazer et al., Nature 449, 851 (2007).
- 37.: P. I. de Bakker et al., Nat Genet 37, 1217 (2005).
- 38.: J. Maller et al., Nat Genet 38, 1055 (2006).
- 39.: G. Lettre et al., Nat Genet 40, 584 (2008).

### Example 7 Sequencing Summary

### Methods

Genomic DNA from 192 SLE patients and 96 healthy controls was whole genome amplified prior to resequencing. Genomic DNA was resequenced of all the exons and selected noncoding regions (2.5 kb of the promoter region-upstream of exon 1) in B-lymphoid Kinase (BLK), Intergrin Alpha M (ITGAM), and Intergrin Alpha X (ITGAX).

Initial allele calling was performed by software provided by "Polymorphic". All the coding polymorphisms as well as common noncoding alleles were manually verified to confirm the allele calls, and create the genotyping files, used for the association and haplotype analysis.

Variants of ITGAM/ITGAX are provided in Tables 7 and 9 and Tables 8 and 10. The variants of Tables 7 and 9 are not present in the database dbSNP build129. The variants of Tables 8 and 10 were discovered by sequencing of ITGAM/ITGAX and BLK.

**Table 7: Variants of ITGAM and ITGAX exons and promoter region.**

| | | | | **Allele Frequency** | | |
|---|---|---|---|---|---|---|
| **ID** | **Minor Allele** | **Chromosome** | **Position** | **Allele** | **Cases** | **Controls** |
| exon8_Gln246Arg | A | 16 | 31192218 | A | 0.0026 | 0.005 |
| exon8_Glu247Lys | G | 16 | 31192220 | G | 0.0026 | 0.005 |
| 237T>C_ex17 | C | 16 | 31243375 | C | 0.175 | 0.174 |
| 186G>A_ex20 | A | 16 | 31244226 | G | 0.652 | 0.62 |
| exon26_Gly1003Glu | G | 16 | 31248726 | G | 0.0026 | 0.005 |
| 3'UTR; 10 bp insertion | Insertion [GAGTGTGTGC] | 16 | 31250691 | G | 0.434 | NaN |
| noncoding1a_329T> C | C | 16 | 31250736 | C | 0.201 | NaN |

**Table 8: Variants of ITGAM and ITGAX exons and promoter region.**

| | | | | **Allele Frequency** | | |
|---|---|---|---|---|---|---|
| **ID** | **Minor Allele** | **Chromosome** | **Position** | **Allele** | **Cases** | **Controls** |
| rs3764327 | T | 16 | 31180630 | C | 0.736 | 0.677 |
| rs1143679 | A | 16 | 31184312 | A | 0.144 | 0.109 |
| rs35314490 | A | 16 | 31190665 | A | 0.155 | 0.078 |
| rs9939679 | C | 16 | 31195622 | C | 0.16 | 0.109 |
| rs11861251 | C | 16 | 31196897 | C | 0.17 | 0.115 |
| rs1143683 | T | 16 | 31244389 | C | 0.827 | 0.823 |
| rs41321249 | A | 16 | 31248925 | A | 0 | 0.021 |
| rs7188189 | T | 16 | 31250109 | C | 0.914 | 0.844 |
| rs1143678 | T | 16 | 31250506 | C | 0.825 | 0.823 |
| rs4594268 | T | 16 | 31250744 | T | 0.246 | NaN |
| rs9933520 | G | 16 | 31250887 | G | 0.178 | 0.172 |
| rs3087796 | G | 16 | 31251154 | A | 0.738 | 0.635 |
| rs41523147 | C | 16 | 31251171 | C | 0.026 | 0.005 |
| rs4597342 | T | 16 | 31251270 | C | 0.729 | 0.661 |
| rs11574633 | C | 16 | 31274819 | C | 0.16 | 0.115 |
| rs2230429 | G | 16 | 31282036 | G | 0.348 | 0.271 |
| rs12448775 | T | 16 | 31292149 | T | 0.042 | 0.031 |
| rs41419150 Insertion [CTTTA] | | 16 | 31251462-31251462 | | 0.176 | 0.102 |

**Table 9: Variants of BLK exons and promoter region.**

| | | | | **Allele Frequency** | | |
|---|---|---|---|---|---|---|
| **ID** | **Minor Allele** | **Chromosome** | **Position** | **Allele** | **Cases** | **Controls** |
| 1120_C>T | T | 8 | 11387925 | T | 0.021 | 0.005 |
| 434_C>T | T | 8 | 11404452 | T | 0.144 | 0.12 |
| ex5_112T>C | T | 8 | 11443842 | T | 0.516 | 0.417 |
| ex9_121T>C | T | 8 | 11451476 | C | 0.829 | 0.798 |
| ex11_6G>A | G | 8 | 11456175 | A | 0.882 | 0.818 |
| 975_G>A | A | 8 | 11456175 | G | 0.89 | 0.818 |
| exon6_Trp131Arg | T | 8 | 11445099 | T | 0.0034 | 0 |
| exon8_Pro237Pro | T | 8 | 11450340 | T | 0.0026 | 0.005 |
| exon10_Thr325Lys | C | 8 | 11452900 | C | 0.0034 | 0 |
| exon13_Arg474Arg | T | 8 | 11458929 | T | 0.0034 | 0 |

**Table 10: Variants of BLK exons and promoter region.**

| | | | | **Allele Frequency** | | |
|---|---|---|---|---|---|---|
| **ID** | **Minor Allele** | **Chromosome** | **Position** | **Allele** | **Cases** | **Controls** |
| rs10097015 | T | 8 | 11458793 | T | 0.419 | 0.358 |
| rs1042689 | T | 8 | 11459203 | T | 0.377 | 0.323 |
| rs1042701 | A | 8 | 11459455 | G | 0.558 | 0.51 |
| rs11784016 | T | 8 | 11404079 | C | 0.717 | 0.667 |
| rs1382567 | C | 8 | 11388309 | T | 0.521 | 0.484 |
| rs1382568 | C | 8 | 11388630 | C | 0.317 | 0.198 |
| rs1382568 | G | 8 | 11388631 | A | 0.524 | 0.49 |
| rs2250788 | A | 8 | 11389466 | G | 0.838 | 0.771 |
| rs2251056 | C | 8 | 11386986 | A | 0.84 | 0.781 |
| rs2409782 | C | 8 | 11404502 | C | 0.238 | 0.234 |
| rs2736344 | T | 8 | 11388088 | C | 0.861 | 0.776 |
| rs2898289 | A | 8 | 11455795 | A | 0.38 | 0.307 |
| rs4629826 | C | 8 | 11404447 | G | 0.937 | 0.917 |
| rs4840568 | A | 8 | 11388429 | A | 0.327 | 0.214 |
| rs4841557 | A | 8 | 11452981 | A | 0.416 | 0.328 |
| rs4841558 | C | 8 | 11453006 | C | 0.413 | 0.328 |
| rs4841561 | T | 8 | 11456182 | T | 0.384 | 0.307 |
| rs4841561 | T | 8 | 11456183 | T | 0.369 | 0.307 |
| rs55758736 | A | 8 | 11442984 | A | 0.016 | 0.021 |
| rs56185487 | A | 8 | 11443008 | A | 0.005 | 0 |
| rs7843987 | C | 8 | 11459540 | T | 0.555 | 0.521 |
| rs922483 | T | 8 | 11389322 | T | 0.359 | 0.224 |
| rs9694294 | C | 8 | 11388131 | G | 0.846 | 0.776 |

### Example 8

### Subjects and study design

A genome-wide association study for SLE was performed. 1079 SLE cases and 1411 controls were genotyped with the Illumina HumanHap550 Genotyping BeadChip (555,352 SNPs). The SLE cases were from three distinct cohorts. Control samples were chosen based on available HLA typing, ethnicity, gender, and age. Most controls (all but 277) were chosen such that the frequency of HLA DR2 and DR3 haplotypes would match that found in SLE.

There have been three versions of the Illumina HumanHap550. The number of SNPs shared between version 1 and version 3 is 545,080; only these SNPs were analyzed. Version 1 was used for all cohort 1 and cohort 2 samples and 1001 control samples. Version 3 was used for all cohort 3 samples and 410 control samples.

Chips with average call rates < 80% were redone. After all redos were complete, samples with < 90% call rates were removed.

Samples were initially divided into two groups for analysis. The first group (Group 1) consisted of all cohort 1 and cohort 2 samples (466 cases) and 724 control samples. The second group (Group 2) consisted of all cohort 3 samples (613 cases) and the remaining 687 control samples.

### Filtering in Group 1

Samples were checked for agreement between genotype-determined gender and clinical records; a discrepancy was found in 10 samples (3 cases, 7 controls), which were removed from further analysis.

Samples were then tested for intercontinental admixture using the program STRUCTURE (the online link can be accessed by typing "pritch.bsd.uchicago.edu/structure" with ".html" as the suffix)(essentially as described in Pritchard et al., Genetics (2000), 155:945-959; Falush et al., Genetics (2003), 164:1567-1587; Falush et al., Molecular Ecology Notes (2007), doi:10.1111/j.1471-8286.2007.01758.x). The HumanHap550 includes a "DNA Test Panel" of 276 SNPs which are ideal for determining percent-ancestry to the CEU, YRI, and CHB+JPT populations of the HapMap project. (CHB and JPT could not be discriminated using these SNPs.) 274 of the 276 SNPs in the DNA Test Panel were genotyped in all HapMap populations; STRUCTURE was run with genotypes for these 274 SNPs in the set consisting of the remaining Group-1 samples (463 cases, 717 controls) plus one sample from each pedigree in the HapMap project (*i.e.,* 20 CEPH samples from Utah (CEU), 30 Yoruba samples (YRI), 45 Han Chinese samples (CHB), and 44 Japanese samples (JPT)). The HapMap samples were included as positive controls and to aid the clustering algorithm. STRUCTURE was run independently three times with the same parameters: using the admixture ancestry model and the correlated allele-frequency model with no prior population information, assuming three populations, with 30,000 burn-in steps followed by 100,000 Markov-Chain Monte Carlo steps. The three runs had very similar coefficients of ancestry for each sample, and each HapMap sample had > 93.0% ancestry to its geographic origin; each CEU sample had > 97.0% CEU ancestry. Samples which had < 90.0% CEU ancestry in any of the three runs (28 cases, 24 controls) were removed from further analysis.

For the remaining samples (435 cases, 693 controls), SNPs with call rates < 95% (23,275 SNPs (4%)) were removed from further analysis. Then, SNPs with Hardy-Weinberg probability ≤ 0.001 in controls (15,622 SNPs (3%)) were removed from further analysis.

### Filtering in Group 2

Samples were not explicitly checked for agreement between genotype-determined gender and clinical records.

SNPs with call rates < 95% (34,998 SNPs (6%)) were removed from further analysis.

Samples were then tested for intercontinental admixture using STRUCTURE, as described above. Samples which had < 90.0% CEU ancestry in any of the three runs (21 cases, 24 controls) were removed from further analysis.

For the remaining samples (592 cases, 663 controls), SNPs with Hardy-Weinberg probability ≤ 0.001 in controls (22,202 SNPs (4%)) were removed from further analysis.

### Combining Groups 1 and 2

Groups 1 and 2 were combined for the final analysis.

The remaining samples (435 cases, 693 controls) in Group 1 were combined with the remaining samples (592 cases, 663 controls) in Group 2 to yield the Final Group (1027 cases, 1356 controls). Only the SNPs remaining in both Group 1 and Group 2 (496,458 SNPs) were analyzed further.

All samples without gender discrepancies (1076 cases, 1404 controls) were checked to see if they could be duplicates or related. Initially all pairs of samples were compared across 800 SNPs spread across the genome. Duplicate and related candidates were then checked across 540,000+ SNPs. Three groups of outliers were detected. The first group (20 pairs) had > 95% identity between each pair and was deemed duplicates. The second group (17 pairs) had 67-77% identity between each pair and was deemed related. The third group (5 pairs) had 58-63% identity between each pair and was deemed related. (Average identity between samples was 51-55%.) Overall, 39 samples (29 cases, 10 controls) were removed from the Final Group.

SNPs in mitochondrial DNA (19 SNPs) were removed from further analysis.

The resulting Main Group (998 cases, 1346 controls, 496,439 SNPs) was used in the analysis below.

The same analysis was also performed on specific subsets of the Main Group:Subset 1:
Females only (907 cases, 967 controls) and Subset 2: Cases with lupus nephritis, and all controls (286 cases, 1346 controls)

### Analysis and Results

All SNPs in the Main Group were analyzed using EIGENSTRAT, which is a program that is essentially described in Price et al., Nature Genetics (2006), 38:904 - 909 (the online link can be accessed by typing "genepath.med.harvard.edu/∼reich/EIGENSTRAT" with ".htm" as the suffix), which also corrects for population stratification. The top 10 principal components were used to remove outliers for 5 rounds and then correct for stratification. The EIGENSTRAT chi-square statistic was then calculated, and the one-tailed probability of the chi-squared distribution was calculated with Microsoft Excel's CHIDIST function with one degree of freedom.

To determine the top candidate regions, we first reduced the number of candidate SNPs by using a P-value threshold: for Subset 1 (females) and Subset 2 (nephritis), SNPs with a P > 2.0 x 10⁻⁵ were removed from further analysis, and for the Main Group (998 cases, 1346 controls), SNPs with a P > 7.0 x 10⁻⁵ were removed from further analysis. In the females subset, 19 SNPs remained. In the nephritis subset, 35 SNPs remained. In the Main Group, 47 SNPs remained. Then, the linkage-disequilibrium (LD) region containing each SNP was determined by examining LD plots utilizing the HelixTree program (the online link can be accessed by typing "www.goldenhelix.com/pharmhelixtreefeatures" with ".html" as the suffix) (Golden Helix, Montana, USA). The EM algorithm was used to calculate D' and r² using only the genotypes of the cases and controls. Regions were delineated by eye, using D' ≥ ∼0.9 as bounds.

Once each region was delineated, the genes in each region were looked at with an art-established genome browser (e.g., the UCSC Genome Browser, essentially described in Kuhn et al., Nucleic Acids Res. (2007), 35(database issue):D668-73; the online link can be accessed by typing "genome.ucsc" with ".edu" as the suffix, March 2006 assembly). Immune-specific gene expression, as determined in the IRIS study (Abbas et al., Genes and Immunity (2005), 6:319-331, including its online supplementary material) was examined. Top candidate regions were identified, for example, by the presence of immune-specific genes in a region. In the nephritis subset, 11 regions containing 20 candidate SNPs were chosen as likely to contain at least one risk allele for SLE (Figure 12). In the females subset, 6 additional regions containing 9 candidate SNPs were chosen (Figure 13). In the Main Group, 6 additional regions containing 8 can didate SNPs were chosen (Figure 14). A total of 23 regions containing 37 candidate SNPs were chosen. It should be noted that SNPs were listed under the study group that had the strongest result for the SNPs, thus duplicate hits among the study groups are not shown. In addition, hits in the MHC region were not included. LD regions delineated based on the data in Figures 12-14 were determined, and are summarized in Figure 15-17, respectively.

The following numbered paragraphs (paras.) contain further statements of various aspects of the present disclosure -
1. A method of assessing whether a subject is at risk of developing lupus, the method comprising, detecting in a biological sample obtained from said subject, the presence of a genetic signature indicative of risk of developing lupus, wherein said genetic signature comprises a set of one or more SNPs selected from any of the SNPs set forth in Figures 1-17 and Tables 1-10.
2. The method of para 1, wherein said set of SNPs comprises about 1-10, 10-20, 20-30, 30-40, or 40-50 SNPs selected from any of the SNPs set forth in Figures 1-17 and Tables 1-10.
3. The method of para 1, wherein said set of SNPs comprises 2 or more SNPs, 3 or more SNPs, 4 or more SNPs, 5 or more SNPs, 6 or more SNPs, 7 or more SNPs, 8 or more SNPs, 9 or more SNPs, 10 or more SNPs, 11 or more SNPs, 12 or more SNPs, 13 or more SNPs, 14 or more SNPs, 15 or more SNPs, 16 or more SNPs, 17 or more SNPs, 18 or more SNPs, 19 or more SNPs, or 20 or more SNPs selected from any of the SNPs set forth in Figures 1-17 and Tables 1-10.
4. The method of para 1, wherein said set of SNPs comprises 1-19 SNPs selected from Table 6.
5. The method of para 1, wherein said set of SNPs comprises a BLK SNP selected from any of the BLK SNPs set forth in Tables 7-10.
6. The method of para 1, wherein said set of SNPs comprises an ITGAM SNP selected from any of the ITGAM SNPs set forth in Tables 7-10.
7. The method of para 6, wherein said set of SNPs further comprises a BLK SNP selected from any of the BLK SNPs set forth in Tables 7-10.
8. The method of para 1, wherein said set of SNPs comprises one or more SNPs selected from the following group of SNPs: rs2187668, rs10488631, rs7574865, rs9888739, rs13277113, rs2431697, rs6568431, rs10489265, rs2476601, rs2269368, rs1801274, rs4963128, rs5754217, rs6445975, rs3129860, rs10516487, rs6889239, rs2391592, and rs2177770.
9. A method of diagnosing lupus in a subject, the method comprising, detecting in a biological sample obtained from said subject, the presence of a genetic signature indicative of lupus, wherein said genetic signature comprises a set of one or more SNPs selected from any of the SNPs set forth in Figures 1-17 and Tables 1-10.
10. An isolated polynucleotide comprising (a) a PRO-associated polynucleotide or fragment thereof that is at least about 10 nucleotides in length, wherein the PRO-associated polynucleotide or fragment thereof comprises a genetic variation at a nucleotide position corresponding to the position of a single nucleotide polymorphism (SNP) listed in Figures 1-17 and Tables 1-10, or (b) the complement of (a).
11. The isolated polynucleotide of para 10, wherein the genetic variation is in genomic DNA that encodes a gene (or its regulatory region) comprising a single nucleotide polymorphism (SNP) listed in Figures 1-17 and Tables 1-10.
12. The isolated polynucleotide of para 11, wherein the SNP is in a non-coding region of the gene.
13. The isolated polynucleotide of para 11, wherein the SNP is in a coding region of the gene.
14. The isolated polynucleotide of para 10, wherein the isolated polynucleotide is a primer.
15. The isolated polynucleotide of para 10, wherein the isolated polynucleotide is an oligonucleotide.
16. An oligonucleotide that is (a) an allele-specific oligonucleotide that hybridizes to a region of a PRO-associated polynucleotide comprising a genetic variation at a nucleotide position corresponding to the position of a single nucleotide polymorphism (SNP) listed in Figures 1-17 and Tables 1-10, or (b) the complement of (a).
17. The oligonucleotide of para 16, wherein the SNP is in genomic DNA that encodes a gene (or its regulatory region) comprising a single nucleotide polymorphism (SNP) listed in Figures 1-17 and Tables 1-10.
18. The oligonucleotide of para 17, wherein the SNP is in a non-coding region of the gene.
19. The oligonucleotide of para 17, wherein the SNP is in a coding region of the gene.
20. The oligonucleotide of para 16, wherein the allele-specific oligonucleotide is an allele-specific primer.
21. A kit comprising the oligonucleotide of para 16 and, optionally, at least one enzyme.
22. The kit of para 21, wherein the at least one enzyme is a polymerase.
23. The kit of para 21, wherein the at least one enzyme is a ligase.
24. A microarray comprising the oligonucleotide of para 16.
25. A method of detecting the absence or presence of a variation in a PRO-associated polynucleotide at a nucleotide position corresponding to the position of a single nucleotide polymorphism (SNP) as set forth in Figures 1-17 and Tables 1-10, the method comprising (a) contacting nucleic acid suspected of comprising the variation with an allele-specific oligonucleotide that is specific for the variation under conditions suitable for hybridization of the allele-specific oligonucleotide to the nucleic acid; and (b) detecting the absence or presence of allele-specific hybridization.
26. The method of para 25, wherein the variation comprises a SNP as set forth in Figures 1-17 and Tables 1-10.
27. A method of amplifying a nucleic acid comprising a variation in a PRO-associated polynucleotide at a nucleotide position corresponding to the position of a single nucleotide polymorphism (SNP) as set forth in Figures 1-17 and Tables 1-10, the method comprising (a) contacting the nucleic acid with a primer that hybridizes to the nucleic acid at a sequence 3' of the variation, and (b) extending the primer to generate an amplification product comprising the variation.
28. The method of para 27, wherein the variation comprises a SNP as set forth in Figures 1-17 and Tables 1-10.
29. A method of determining the genotype of a biological sample from a mammal, the method comprising detecting, in nucleic acid material derived from the biological sample, the absence or presence of a variation in a PRO-associated polynucleotide at a nucleotide position corresponding to the position of a single nucleotide polymorphism (SNP) as set forth in Figures 1-17 and Tables 1-10.
30. The method of para 29, wherein the variation comprises a SNP as set forth in Figures 1-17 and Tables 1-10.
31. The method of para 29, wherein the biological sample is known to comprise, or suspected of comprising, a PRO or PRO-associated polynucleotide comprising the variation.
32. The method of para 29, wherein the biological sample is a disease tissue.
33. The method of para 29, wherein the detecting comprises carrying out a process selected from a primer extension assay; an allele-specific primer extension assay; an allele-specific nucleotide incorporation assay; an allele-specific oligonucleotide hybridization assay; a 5' nuclease assay; an assay employing molecular beacons; and an oligonucleotide ligation assay.
34. A method of sub-classifying lupus in a mammal, the method comprising detecting the presence of a variation in a PRO-associated polynucleotide at a nucleotide position corresponding to the position of a single nucleotide polymorphism (SNP) as set forth in Figures 1-17 and Tables 1-10 in a biological sample derived from the mammal, wherein the biological sample is known to comprise, or suspected of comprising, a PRO or PRO-associated polynucleotide comprising the variation.
35. The method of para 34, wherein the variation is a genetic variation.
36. The method of para 35, wherein the variation comprises a SNP as set forth in Figures 1-17 and Tables 1-10.
37. The method of para 34, wherein the detecting comprises carrying out a process selected from a primer extension assay; an allele-specific primer extension assay; an allele-specific nucleotide incorporation assay; an allele-specific oligonucleotide hybridization assay; a 5'nuclease assay; an assay employing molecular beacons; and an oligonucleotide ligation assay.
38. A method for predicting whether a subject with lupus will respond to a lupus therapeutic agent, the method comprising determining whether the subject comprises a variation in a PRO-associated polynucleotide at a nucleotide position corresponding to the position of a single nucleotide polymorphism (SNP) as set forth in Figures 1-17 and Tables 1-10, wherein the presence of a variation indicates that the subject will respond to the therapeutic agent.
39. The method of para 38, wherein the variation is a genetic variation.
40. The method of para 39, wherein the variation comprises a SNP as set forth in Figures 1-17 and Tables 1-10.
41. A method of diagnosing or prognosing lupus in a subject, the method comprising detecting the presence of a variation in a PRO or PRO-associated polynucleotide derived from a biological sample obtained from the subject, wherein:
   (a) the biological sample is known to comprise, or suspected of comprising, a PRO or PRO-associated polynucleotide comprising the variation;
   (b) the variation comprises, or is located at a nucleotide position corresponding to, a SNP set forth in Figures 1-17 and Tables 1-10; and
   (c) the presence of the variation is a diagnosis or prognosis of lupus in the subject.
42. A method of aiding in the diagnosis or prognosis of lupus in a subject, the method comprising detecting the presence of a variation in a PRO or PRO-associated polynucleotide derived from a biological sample obtained from the subject, wherein:
   (a) the biological sample is known to comprise, or suspected of comprising, a PRO or PRO-associated polynucleotide comprising the variation;
   (b) the variation comprises, or is located at a nucleotide position corresponding to, a SNP set forth in Figures 1-17 and Tables 1-10; and
   (c) the presence of the variation is a diagnosis or prognosis of a condition or symptom of lupus in the subject.
43. The method of para 41 or 42, wherein the PRO-associated polynucleotide encodes a PRO that is encoded by a sequence within a linkage disequilibrium region.
44. The method of para 43, wherein the linkage disequilibrium region is one of those set forth in Figures 1-17 and Tables 1-10.
45. The method of para 41 or 42, wherein the variation is in a genomic DNA that encodes a gene, or its regulatory region, and wherein the respective gene, or its regulatory region, comprises a SNP set forth in Figures 1-17 and Tables 1-10.
46. The method of para 45, wherein the SNP is in a non-coding region of the gene.
47. The method of para 45, wherein the SNP is in a coding region of the gene.
48. A method of identifying a therapeutic agent effective to treat lupus in a patient subpopulation, the method comprising correlating efficacy of the agent with the presence of a genetic variation at a nucleotide position corresponding to a single nucleotide polymorphism (SNP) in the patient subpopulation, wherein the SNP is one of those listed in Figures 1-17 and Tables 1-10, thereby identifying the agent as effective to treat lupus in said patient subpopulation.
49. A method of treating a lupus condition in a subject in whom a genetic variation is known to be present at a nucleotide position corresponding to a single nucleotide polymorphism (SNP) listed in Figures 1-17 and Tables 1-10, the method comprising administering to the subject a therapeutic agent effective to treat the condition.
50. A method of treating a subject having a lupus condition, the method comprising administering to the subject a therapeutic agent effective to treat the condition in a subject who has a genetic variation at a nucleotide position corresponding to a single nucleotide polymorphism (SNP) listed in Figures 1-17 and Tables 1-10.
51. A method of treating a subject having a lupus condition, the method comprising administering to the subject a therapeutic agent shown to be effective to treat said condition in at least one clinical study wherein the agent was administered to at least five human subjects who each had a genetic variation at a nucleotide position corresponding to a single nucleotide polymorphism (SNP) listed in Figures 1-17 and Tables 1-10.
52. The method of para 51, wherein the at least five subjects had two or more different SNPs in total for the group of at least five subjects.
53. The method of para 51, wherein the at least five subjects had the same SNP for the entire group of at least five subjects.
54. A method of treating a lupus subject of a specific lupus patient subpopulation, wherein the subpopulation is characterized at least in part by association with genetic variation at a nucleotide position corresponding to a SNP listed in Figures 1-17 and Tables 1-10, and wherein the method comprises administering to the subject an effective amount of a therapeutic agent that is approved as a therapeutic agent for said subpopulation.
55. The method of para 54, wherein the subpopulation has lupus nephritis.
56. The method of para 54, wherein the subpopulation is female.
57. The method of para 54, wherein the subpopulation is of European ancestry.
58. A method comprising manufacturing a lupus therapeutic agent, and packaging the agent with instruction to administer the agent to a subject who has or is believed to have lupus and who has a genetic variation at a position corresponding to a single nucleotide polymorphism (SNP) listed in Figures 1-17 and Tables 1-10.
59. A method of specifying a therapeutic agent for use in a lupus patient subpopulation, the method comprising providing instruction to administer the therapeutic agent to a patient subpopulation characterized by a genetic variation at a position corresponding to a single nucleotide polymorphism (SNP) listed in Figures 1-17 and Tables 1-10.
60. A method for marketing a therapeutic agent for use in a lupus patient subpopulation, the method comprising informing a target audience about the use of the therapeutic agent for treating the patient subpopulation as characterized by the presence, in patients of such subpopulation, of a genetic variation at a position corresponding to a single nucleotide polymorphism (SNP) listed in Figures 1-17 and Tables 1-10.
61. A method for modulating signaling through the B cell receptor in a subject in whom a genetic variation is known to be present at a nucleotide position corresponding to a single nucleotide polymorphism (SNP) listed in Figures 1-17 and Tables 1-10, the method comprising administering to the subject a therapeutic agent effective to modulate signaling through the B cell receptor.
62. A method for modulating the differentiation of Th17 cells in a subject in whom a genetic variation is known to be present at a nucleotide position corresponding to a single nucleotide polymorphism (SNP) listed in Figures 1-17 and Tables 1-10, the method comprising administering to the subject a therapeutic agent effective to modulate the differentiation of Th17 cells.
63. A set of SNPs comprising a genetic signature indicative of the risk of developing lupus, wherein said set of SNPs comprises one or more SNPs selected from any of the SNPs set forth in Figures 1-17 and Tables 1-10.
64. The set of SNPs of para 63, wherein said set of SNPs comprises about 1-10, 10-20, 20-30, 30-40, or 40-50 SNPs selected from any of the SNPs set forth in Figures 1-17 and Tables 1-10.
65. The set of SNPs of para 63, wherein said set of SNPs comprises one or more SNPs selected from the group consisting of rs9888739, rs13277113, rs7574865, rs2269368, rs6889239, rs2391592 and rs21177770.
66. The set of SNPs of para 63, wherein said set of SNPs comprises 2 or more SNPs, 3 or more SNPs, 4 or more SNPs, 5 or more SNPs, 6 or more SNPs, 7 or more SNPs, 8 or more SNPs, 9 or more SNPs, 10 or more SNPs, 11 or more SNPs, 12 or more SNPs, 13 or more SNPs, 14 or more SNPs, 15 or more SNPs, 16 or more SNPs, 17 or more SNPs, 18 or more SNPs, 19 or more SNPs, or 20 or more SNPs selected from any of the SNPs set forth in Figures 1-17 and Tables 1-10.
67. The set of SNPs of para 63, wherein said set of SNPs comprises 1-19 SNPs selected from Table 6.
68. The set of SNPs of para 63, wherein said set of SNPs comprises a BLK SNP selected from any of the BLK SNPs set forth in Tables 7-10.
69. The set of SNPs of para 63, wherein said set of SNPs comprises an ITGAM SNP selected from any of the ITGAM SNPs set forth in Tables 7-10.
70. The set of SNPs of para 69, wherein said set of SNPs further comprises a BLK SNP selected from any of the BLK SNPs set forth in Tables 7-10.
71. The set of SNPs of para 63, wherein said set of SNPs comprises one or more SNPs selected from the following group of SNPs: rs2187668, rs10488631, rs7574865, rs9888739, rs13277113, rs2431697, rs6568431, rs10489265, rs2476601, rs2269368, rs1801274, rs4963128, rs5754217, rs6445975, rs3129860, rs10516487, rs6889239, rs2391592, and rs2177770.
72. A set of SNPs comprising a genetic signature indicative of lupus, wherein said set of SNPs comprises one or more SNPs selected from any of the SNPs set forth in Figures 1-17 and Tables 1-10.

## Claims

1. A method of diagnosing lupus or assessing risk of developing lupus in a subject of a specific lupus patient subpopulation, the method comprising:
a) detecting in a biological sample obtained from the subject the presence of a genetic signature indicative of lupus or risk of developing lupus, wherein said genetic signature comprises the absence of a minor allele of single nucleotide polymorphism (SNP) rs4963128 and the presence of a minor allele of SNP rs2269368; and
b) determining that the subject has lupus or is at risk of developing lupus when the genetic signature is present.

2. The method of claim 1 wherein the genetic signature further comprises the presence of a minor allele of one or more of rs5754217, rs7574865, rs3129860, rs10489265, and rs6445975; and/or the absence of a minor allele of rs2431697.

3. The method of claim 1, wherein the genetic signature further comprises the presence of a minor allele of one or more of rs5754217, and rs7574865.

4. The method of claim 3, wherein the genetic signature further comprises the presence of a minor allele of one or more of rs10488631 and rs1143679; and/or the absence of a minor allele of rs1801274.

5. The method of claim 1, wherein the genetic signature further comprises the presence of a minor allele of one or more of rs3129860 and rs10489265.

6. The method of claim 5, wherein the genetic signature further comprises the presence of a minor allele of one or more of rs6568431, rs2187668, rs13277113, and rs2476601; and/or the absence of a minor allele of rs10516487.

7. The method of claim 1, wherein the genetic signature further comprises the presence of a minor allele of rs6445975; and/or the absence of a minor allele of rs2431697.

8. A therapeutic agent effective to treat lupus for use in a method of treating lupus in a subject of a specific lupus patient subpopulation, wherein the presence of the genetic signature in the subject is determined according to the method of claim 1, thereby identifying the subject as part of a specific lupus patient subpopulation for treatment.

9. The therapeutic agent for use of claim 8, wherein the genetic signature further comprises the presence of a minor allele of one or more of rs5754217, rs7574865, rs3129860, rs10489265, and rs6445975; and/or the absence of a minor allele of rs2431697.

10. The therapeutic agent for use of claim 8 , wherein the genetic signature further comprises the presence of a minor allele of_one or more of rs5754217, and rs7574865.

11. The therapeutic agent for use of claim 10, wherein the genetic signature further comprises the presence of a minor allele of one or more of rs10488631 and rs1143679; and/or the absence of a minor allele of rs1801274.

12. The therapeutic agent for use of claim 8, wherein the genetic signature further comprises the presence of a minor allele of_one or more of rs3129860 and rs10489265.

13. The therapeutic agent for use of claim 12, wherein the genetic signature further comprises the presence of a minor allele of one or more of rs6568431, rs2187668, rs13277113, and rs2476601; and/or the absence of a minor allele of_rs10516487.

14. The therapeutic agent for use of claim 8, wherein the genetic signature further comprises the presence of a minor allele of rs6445975; and/or the absence of a minor allele of rs2431697.

15. A composition comprising a plurality of nucleic acid molecules each capable of allele-specific hybridization to a set of target regions of a polynucleotide, said regions comprising minor alleles of the SNPs as defined in any one of claims 1 to 7.

## Patentansprüche

1. Verfahren zum Diagnostizieren von Lupus oder Bestimmen des Risikos der Entwicklung von Lupus in einem Individuum einer spezifischen Subpopulation von Lupuspatienten, wobei das Verfahren Folgendes umfasst:
a) Detektieren des Vorliegens einer genetischen Signatur, die ein Indikator für Lupus oder das Risiko der Entwicklung von Lupus ist, in einer von dem Individuum erhaltenen biologischen Probe, wobei die genetische Signatur das Nichtvorliegen eines Minor-Allels von Einzelnucleotid-Polymorphismus (SNP) rs4963128 und das Vorliegen eines Minor-Allels von SNP rs2269368 umfasst; und
b) Bestimmen, dass das Individuum Lupus aufweist oder bei ihm das Risiko der Entwicklung von Lupus besteht, wenn die genetische Signatur vorliegt.

2. Verfahren nach Anspruch 1, worin die genetische Signatur weiters das Vorliegen eines Minor-Allels von einem oder mehreren von rs5754217, rs7574865, rs3129860, rs10489265 und rs6445975; und/oder das Nichtvorliegen eines Minor-Allels von rs2431697 umfasst.

3. Verfahren nach Anspruch 1, worin die genetische Signatur weiters das Vorliegen eines Minor-Allels von einem oder mehreren von rs5754217 und rs7574865 umfasst.

4. Verfahren nach Anspruch 3, worin die genetische Signatur weiters das Vorliegen eines Minor-Allels von einem oder mehreren von rs10488631 und rs1143679; und/oder das Nichtvorliegen eines Minor-Allels von rs1801274 umfasst.

5. Verfahren nach Anspruch 1, worin die genetische Signatur weiters das Vorliegen eines Minor-Allels von einem oder mehreren von rs3129860 und rs10489265 umfasst.

6. Verfahren nach Anspruch 5, worin die genetische Signatur weiters das Vorliegen eines Minor-Allels von einem oder mehreren von rs6568431, rs2187668, rs13277113 und rs2476601; und/oder das Nichtvorliegen eines Minor-Allels von rs10516487 umfasst.

7. Verfahren nach Anspruch 1, worin die genetische Signatur weiters das Vorliegen eines Minor-Allels von rs6445975; und/oder das Nichtvorliegen eines Minor-Allels von rs2431697 umfasst.

8. Therapeutikum, das wirksam für die Behandlung von Lupus ist, zur Verwendung in einem Verfahren zur Behandlung von Lupus in einem Individuum einer spezifischen Subpopulation von Lupuspatienten, worin das Vorliegen der genetischen Signatur in dem Individuum gemäß dem Verfahren nach Anspruch 1 bestimmt wird, wodurch das Individuum als Teil einer spezifischen Subpopulation von Lupuspatienten zur Behandlung identifiziert wird.

9. Therapeutikum zur Verwendung nach Anspruch 8, worin die genetische Signatur weiters das Vorliegen eines Minor-Allels von einem oder mehreren von rs5754217, rs7574865, rs3129860, rs10489265 und rs6445975; und/oder das Nichtvorliegen eines Minor-Allels von rs2431697 umfasst.

10. Therapeutikum zur Verwendung nach Anspruch 8, worin die genetische Signatur weiters das Vorliegen eines Minor-Allels von einem oder mehreren von rs5754217 und rs7574865 umfasst.

11. Therapeutikum zur Verwendung nach Anspruch 10, worin die genetische Signatur weiters das Vorliegen eines Minor-Allels von einem oder mehreren von rs10488631 und rs1143679; und/oder das Nichtvorliegen eines Minor-Allels von rs1801274 umfasst.

12. Therapeutikum zur Verwendung nach Anspruch 8, worin die genetische Signatur weiters das Vorliegen eines Minor-Allels von einem oder mehreren von rs3129860 und 10489265 umfasst.

13. Therapeutikum zur Verwendung nach Anspruch 12, worin die genetische Signatur weiters das Vorliegen eines Minor-Allels von einem oder mehreren von rs6568431, rs2187668, rs13277113 und rs2476601; und/oder das Nichtvorliegen eines Minor-Allels von rs10516487 umfasst.

14. Therapeutikum zur Verwendung nach Anspruch 8, worin die genetische Signatur weiters das Vorliegen eines Minor-Allels von rs6445975; und/oder das Nichtvorliegen eines Minor-Allels von rs2431697 umfasst.

15. Zusammensetzung, umfassend eine Vielzahl von Nucleinsäuremolekülen, von denen jedes zu Allel-spezifischer Hybridisierung an einen Satz von Zielregionen eines Polynucleotids in der Lage ist, wobei die Regionen Minor-Allele der SNPs umfassen, wie in einem der Ansprüche 1 bis 7 definiert.

## Revendications

1. Procédé pour diagnostiquer le lupus ou évaluer le risque de développer le lupus chez un sujet d'une sous-population de patients atteints de lupus spécifique, le procédé comprenant le fait :
a) de détecter dans un échantillon biologique obtenu à partir du sujet la présence d'une signature génétique indiquant le lupus ou le risque de développer le lupus, où ladite signature génétique comprend l'absence d'un allèle mineur de polymorphisme mononucléotidique (SNP) rs4963128 et la présence d'un allèle mineur de SNP rs2269368 ; et
b) de déterminer que le sujet souffre d'un lupus ou présente un risque de développer le lupus lorsque la signature génétique est présente.

2. Procédé de la revendication 1, dans lequel la signature génétique comprend en outre la présence d'un allèle mineur d'un ou de plusieurs parmi rs5754217, rs7574865, rs3129860, rs10489265 et rs6445975 ; et/ou l'absence d'un allèle mineur de rs2431697.

3. Procédé de la revendication 1, dans lequel la signature génétique comprend en outre la présence d'un allèle mineur de rs5754217, et de rs7574865.

4. Procédé de la revendication 3, dans lequel la signature génétique comprend en outre la présence d'un allèle mineur d'un ou de plusieurs parmi rs10488631 rs1143679 ; et/ou l'absence d'un allèle mineur de rs1801274.

5. Procédé de la revendication 1, dans lequel la signature génétique comprend en outre la présence d'un allèle mineur d'un ou de plusieurs parmi rs3129860 et rs10489265.

6. Procédé de la revendication 5, dans lequel la signature génétique comprend en outre la présence d'un allèle mineur d'un ou de plusieurs parmi rs6568431, rs2187668, rs13277113, et rs2476601 ; et/ou l'absence d'un allèle mineur de rs10516487.

7. Procédé de la revendication 1, dans lequel la signature génétique comprend en outre la présence d'un allèle mineur de rs6445975 ; et/ou l'absence d'un allèle mineur de rs2431697.

8. Agent thérapeutique efficace pour traiter le lupus destiné à être utilisé dans un procédé de traitement du lupus chez un sujet d'une sous-population de patients atteints de lupus spécifique, où la présence de la signature génétique chez le sujet est déterminée selon le procédé de la revendication 1, identifiant ainsi le sujet comme faisant partie d'une sous-population de patients atteints de lupus spécifique pour le traitement.

9. Agent thérapeutique pour l'utilisation de la revendication 8, dans lequel la signature génétique comprend en outre la présence d'un allèle mineur d'un ou de plusieurs parmi rs5754217, rs7574865, rs3129860, rs10489265 et rs6445975 ; et/ou l'absence d'un allèle mineur de rs2431697.

10. Agent thérapeutique pour l'utilisation de la revendication 8, dans lequel la signature génétique comprend en outre la présence d'un allèle mineur d'un ou de plusieurs parmi rs5754217 et rs7574865.

11. Agent thérapeutique pour l'utilisation de la revendication 10, dans lequel la signature génétique comprend en outre la présence d'un allèle mineur d'un ou de plusieurs parmi rs10488631 et rs1143679 ; et/ou l'absence d'un allèle mineur de rs1801274.

12. Agent thérapeutique pour l'utilisation de la revendication 8, dans lequel la signature génétique comprend en outre la présence d'un allèle mineur d'un ou de plusieurs parmi rs3129860 et rs10489265.

13. Agent thérapeutique pour l'utilisation de la revendication 12, dans lequel la signature génétique comprend en outre la présence d'un allèle mineur d'un ou de plusieurs parmi rs6568431, rs2187668, rs13277113 et rs2476601 ; et/ou l'absence d'un allèle mineur de rs10516487.

14. Agent thérapeutique pour l'utilisation de la revendication 8, dans lequel la signature génétique comprend en outre la présence d'un allèle mineur de rs6445975 ; et/ou l'absence d'un allèle mineur de rs2431697.

15. Composition comprenant une pluralité de molécules d'acide nucléique capables chacune d'une hybridation spécifique d'allèle à un ensemble de régions cibles d'un polynucléotide, lesdites régions comprenant des allèles mineurs des SNP tels que définis dans l'une quelconque des revendications 1 à 7.
